(19) Europäisches Patentamt
European Patent Office
Office européen des brevets

(11) Numéro de publication : **0 521 768 B1**

(12) # FASCICULE DE BREVET EUROPEEN

(45) Date de publication du fascicule du brevet : **02.02.94 Bulletin 94/05**

(21) Numéro de dépôt : **92401850.0**

(22) Date de dépôt : **30.06.92**

(51) Int. Cl.$^5$ : **C07D 487/04,** A61K 31/505, // (C07D487/04, 249:00, 239:00)

(54) **Nouveaux dérivés de triazolopyrimidine antagonistes des récepteurs à l'angiotensine II; leurs procédés de préparation, compositions pharmaceutiques les contenant.**

Le dossier contient des informations techniques présentées postérieurement au dépôt de la demande et ne figurant pas dans le présent fascicule.

(30) Priorité : **05.07.91 FR 9108486**

(43) Date de publication de la demande : **07.01.93 Bulletin 93/01**

(45) Mention de la délivrance du brevet : **02.02.94 Bulletin 94/05**

(84) Etats contractants désignés : **AT BE CH DE DK ES FR GB GR IT LI LU MC NL PT SE**

(56) Documents cités :
**EP-A- 0 152 841**
**EP-A- 0 435 827**
**WO-A-91/15209**
**GB-A- 897 870**
**US-A- 4 269 980**

(73) Titulaire : **LABORATOIRES UPSA**
**1 bis, rue du Docteur Camille Bru**
**F-47000 Agen (FR)**

(72) Inventeur : **Bru-Magniez, Nicole**
**24,26, Avenue Raphael**
**F-75016 Paris (FR)**
Inventeur : **Nicolai, Eric**
**52, rue St. Ouen, Résidence Le Hameau Bt. C**
**F-14000 Caen (FR)**
Inventeur : **Teulon, Jean-Marie**
**13, Avenue Guibert**
**F-78170 La Celle Saint Cloud (FR)**

(74) Mandataire : **Hubert, Philippe**
**Cabinet Beau de Loménie 158, rue de l'Université**
**F-75340 Paris Cédex 07 (FR)**

EP 0 521 768 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne en tant que produits nouveaux, les dérivés de triazolopyrimidine de formule générale (I) ci-dessous ainsi que leurs formes tautomères et éventuellement leurs sels d'addition en particulier les sels d'addition pharmaceutiquement acceptables.

Les composés en question présentent un profil pharmacologique très intéressant dans la mesure où ils sont doués de propriétés antagonistes des récepteurs à l'angiotensine II. Ils sont donc particulièrement indiqués pour le traitement des maladies cardiovasculaires, en particulier pour le traitement de l'hypertension, pour le traitement de l'insuffisance cardiaque et pour le traitement des maladies de la paroi artérielle.

La présente invention concerne également le procédé de préparation des dits produits et leurs utilisations en thérapeutique.

On connaît, notamment par le document EP-A-0.435.827 des composés présentant une activité antagoniste des récepteurs à l'angiotensine II.

Cependant, ces composés connus sont dérivés de pyrimidinone.

De même, le document WO-A-9115209, qui a été publié le 17.10.1991 et donc opposable sur le plan de la nouveauté, décrit des dérivés de pyrimidine et également de pyrimidinone, de pyridopyrimidine structurellement différents des composés de l'invention.

Le document EP-A-0.152.841 décrit des composés dérivés d'un système tricyclique présentés comme vasodilatateurs rénaux et diurétiques.

Le document GB-A-897.870 décrit des dérivés de triazolopyrimidine qui, à la différence des composés revendiqués, présentent en position 8 du cycle triazolopyrimidine, un radical choisi parmi l'atome d'hydrogène, un alkyle, un alcényle ou un atome d'halogène qui ne permet pas d'obtenir une bonne affinité avec les récepteurs à l'angiotensine II.

Enfin, le document US-A-4.269.980 décrit généralement des dérivés de triazolopyrimidine substitués en positions 5, 7 ou 8 par un groupe phényle éventuellement substitué.

Ces composés sont très différents des composés de l'invention, tant sur le plan de leur structure chimique que sur le plan de leurs propriétés pharmacologiques, puisqu'ils sont présentés comme anxiolytiques.

Les dérivés de triazolopyrimidine conformes à l'invention et leurs formes tautomères sont caractérisés en ce qu'ils répondent à la formule générale (I):

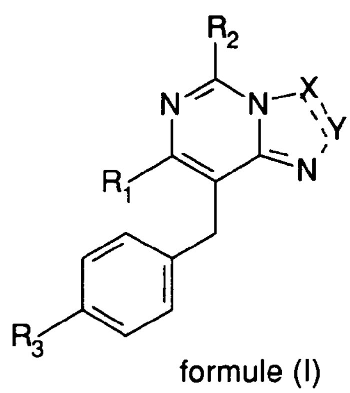

formule (I)

Dans la formule (I),

$R_1$ représente un radical alkyle inférieur de 1 à 6 atomes de carbone ou un radical cycloalkyle en $C_3$-$C_7$,

$R_2$ représente l'atome d'hydrogène, un radical alkyle inférieur de 1 à 6 atomes de carbone, un radical halogéno alkyle inférieur de 1 à 6 atomes de carbone, un groupement $NR_4R'_4$, un groupement $NH$-$NH_2$, un groupement $(CH_2)_m$-$OR_4$, -$(CH_2)_m$-$SR_4$, $R_4$ et $R'_4$ identiques ou différents représentent un atome d'hydrogène, un radical alkyle inférieur de 1 à 6 atomes de carbone et m représente un nombre entier de 0 à 5,

L'ensemble —X---Y— ou —Y---X— représente l'un des radicaux bivalents suivants :

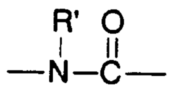 , 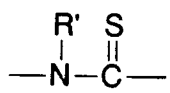 , 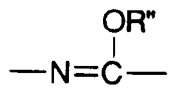 , 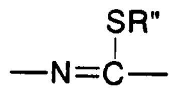 , 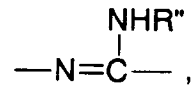 ,

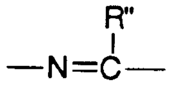 , 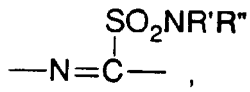 ,

dans lesquels R' et R" identiques ou différents représentent :

- un atome d'hydrogène, un radical alkyle inférieur de 1 à 6 atomes de carbone, un radical halogéno alkyle inférieur de 1 à 6 atomes de carbone,
- un groupement $(CH_2)_n$ $COOR_5$; $(CH_2)_{n'}$-O-$R_5$, $(CH_2)_{n'}$-O-CO-$R_5$, $(CH_2)_{n'}$-S-$R_5$, n étant un nombre entier de 0 à 5,n' étant un nombre entier de 1 à 5 et $R_5$ étant un atome d'hydrogène ou radical alkyle inférieur de 1 à 6 atomes de carbone ;
- un radical phényle, pyridyle, thiényle ou furyle

$R_3$ représente un groupement $NO_2$, $NH_2$, ou représente l'un des radicaux suivants :

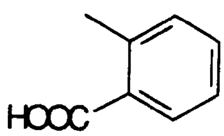 , 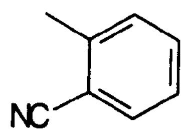 , 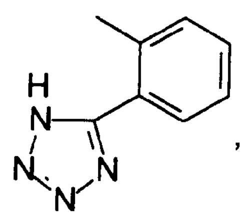 , 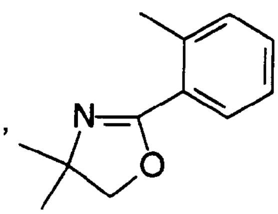 ,

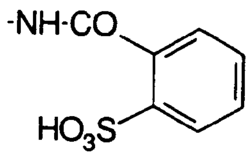 , 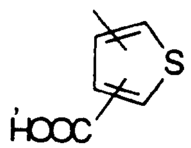 , 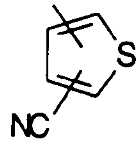 , 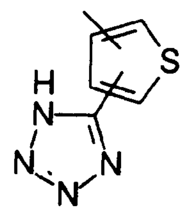

Les dérivés précités doivent être considérés également sous leurs formes tautomères et peuvent se présenter sous la forme de sels d'addition en particulier des sels d'addition pharmaceutiquement acceptables.

Dans la description et les revendications, on entend par alkyle inférieur une chaîne hydrocarbonée ayant de 1 à 6 atomes de carbone, linéaire ou ramifiée. Un radical alkyle inférieur est par exemple un radical méthyle, éthyle, propyle, isopropyle, butyle, isobutyle, tertiobutyle, pentyle, isopentyle, hexyle, isohexyle.

Par radical halogéno alkyle inférieur, on entend un radical alkyle de 1 à 6 atomes de carbone dont 1 à 7 atomes d'hydrogène ont été substitués par 1 à 7 atomes d'halogène. Un radical halogéno alkyle inférieur est par exemple un radical trifluorométhyle, un radical trifluoro-2,2,2 éthyle, un radical pentafluoro éthyle, un radical difluoro-2,2 trifluoro-3,3,3 propyle, un radical heptafluoro propyle, un radical chloro méthyle ou bromo méthyle.

Par radical cycloalkyle en $C_3$-$C_7$, on entend un radical hydrocarboné cyclique saturé, il s'agit de préférence d'un radical cyclopropyle, cyclobutyle, cyclopentyle, cyclohexyle ou cycloheptyle.

On entend par halogène un atome de chlore, de brome, d'iode ou de fluor.

L'invention concerne en particulier des dérivés de formule générale (I) dans laquelle :

$R_1$ représente un radical alkyle inférieur de 1 à 6 atomes de carbone ou un radical cycloalkyle en $C_3$-$C_7$ ;

$R_2$ représente l'atome d'hydrogène, un radical alkyle inférieur de 1 à 6 atomes de carbone, un groupement NH-$NH_2$, un groupement $(CH_2)_mOR_4$, $(CH_2)_m$ $SR_4$; $R_4$ représentant un atome d'hydrogène, un radical alkyle inférieur de 1 à 6 atomes de carbone et m représentant un nombre entier de 0 à 2 ;

L'ensemble —X----Y— représente un radical choisi parmi les radicaux bivalents suivants :

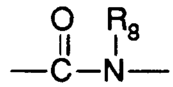 ; 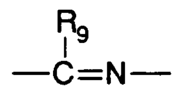 ; 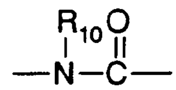 ; 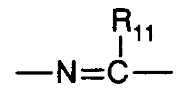

dans lesquels :

$R_8$ représente un radical choisi dans le groupe constitué par l'atome d'hydrogène, $-(CH_2)_nOH$, $-(CH_2)_nCOOH$, $-R_{12}$, $-(CH_2)_nCOOR_{12}$ ; $R_{12}$ représentant un radical alkyle inférieur de 1 à 6 atomes de carbone et n un nombre entier égal à 1 ou 2 ;

$R_9$ représente l'atome d'hydrogène ou un radical -SH ;

$R_{10}$ représente l'atome d'hydrogène ou un radical alkyle inférieur de 1 à 6 atomes de carbone;

$R_{11}$ représente un radical choisi dans le groupe constitué par l'atome d'hydrogène, un radical alkyle inférieur de 1 à 6 atomes de carbone, un radical halogénoalkyle inférieur de 1 à 6 atomes de carbone, phényle, pyridinyle, $-O\ (CH_2)_nOH$, $-OR_{12}$, $-O\ (CH_2)_n\ OCOR_{12}$, SH, $-SR_{12}$, $-S(CH_2)_nCOOR_{12}$, $-S(CH_2)_n\ OCOR_{12}$, $-NH\ (CH_2)_n\ COOR_{12}$, $-NR_{13}R_{14}$, $SO_2NR_{13}R_{14}$, $(CH_2)_nOH$, $(CH_2)_n\ OR_{12}$, COOH, $COOR_{12}$, $(CH_2)_nCOOH$, $(CH_2)_nCOOR_{12}$ ; n et $R_{12}$ étant définis comme indiqué précédemment, $R_{13}$ et $R_{14}$ identiques ou différents représentant l'atome d'hydrogène ou un radical alkyle inférieur ayant de 1 à 6 atomes de carbone ;

$R_3$ représente l'un des radicaux suivants :

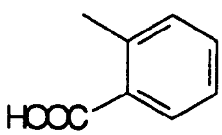 , 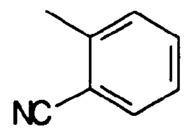 , 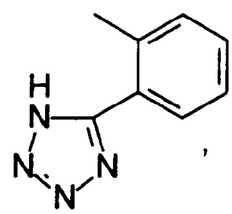 , 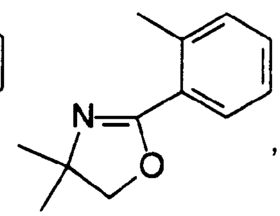 ,

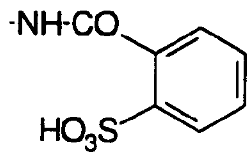 , 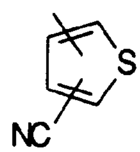 , 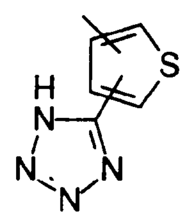

Selon une variante de réalisation, $R_1$ est un groupement n-propyl ;

selon une autre variante de réalisation, $R_1$ est un groupement n-butyl ;

selon une autre variante de réalisation, $R_1$ est un groupement éthyl ;

selon une variante de réalisation, $R_2$ est un groupement méthyl ;

selon une autre variante de réalisation, $R_2$ est un groupement éthyl ;

selon une autre variante de réalisation, $R_2$ est un groupement méthoxy méthyl ;

selon une variante de réalisation l'ensemble —X---Y— représente l'un des radicaux bivalents suivants :

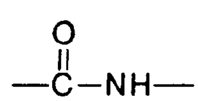 ;

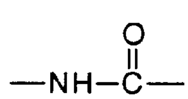

ou leur forme tautomère ;

selon une autre variante de réalisation l'ensemble —X---Y— représente le radical

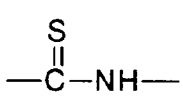

ou sa forme tautomère,

selon une autre variante de réalisation l'ensemble —X---Y— représente le radical

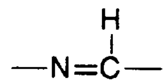

ou

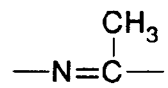 ,

selon une autre variante de réalisation l'ensemble —X---Y— représente le radical

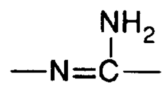

ou

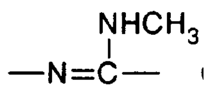 ,

ou leur forme tautomère

selon une autre variante de réalisation l'ensemble —X---Y— représente le radical

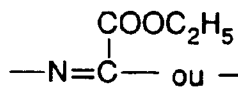 ou 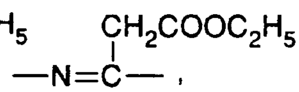 ,

selon une variante de réalisation, $R_3$ est un groupement (tétrazolyl-5)-2 phényl.

Les composés de l'invention particulièrement préférés sont ceux qui sont choisis parmi les produits de formule :

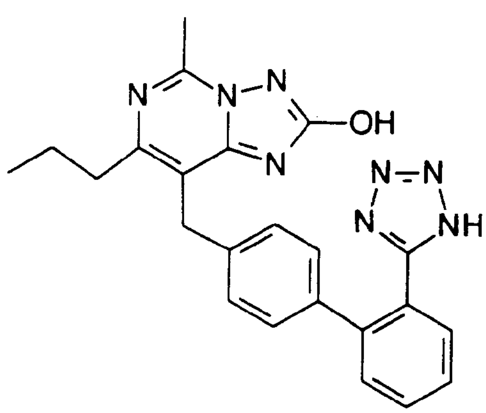

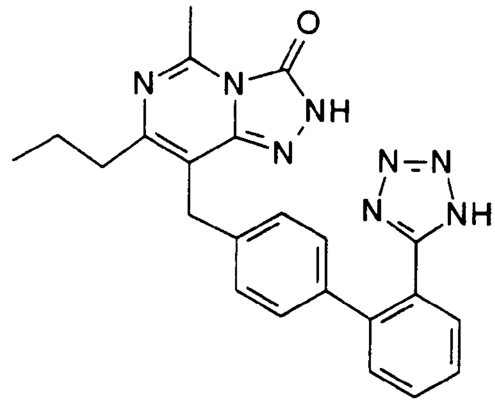

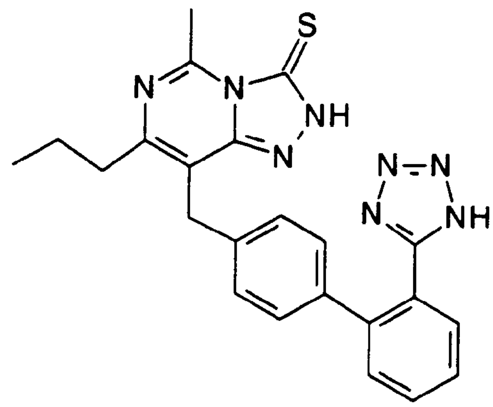

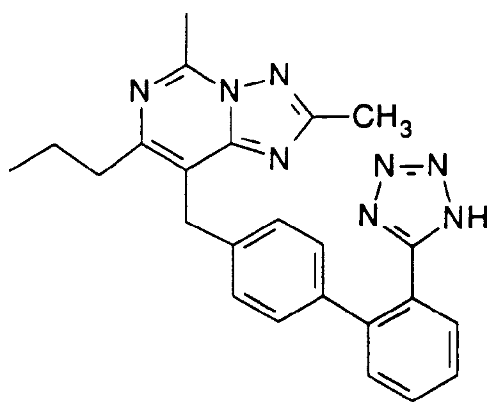

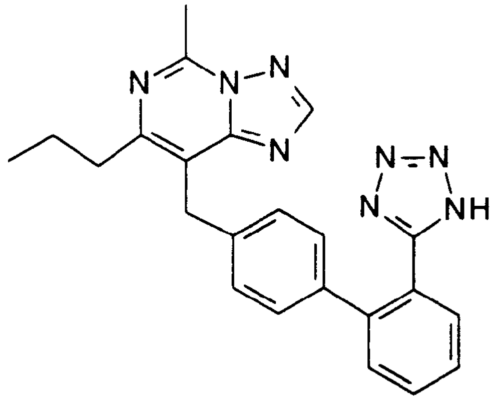

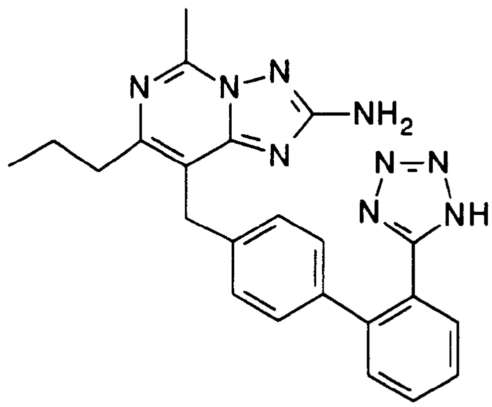

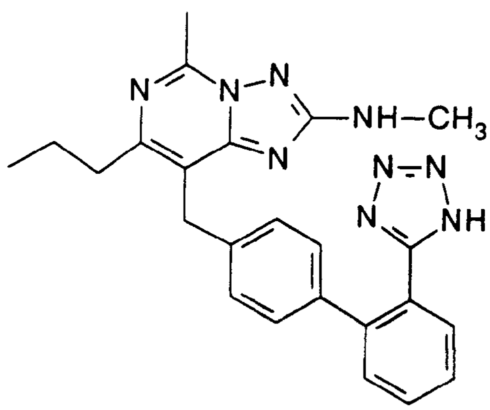

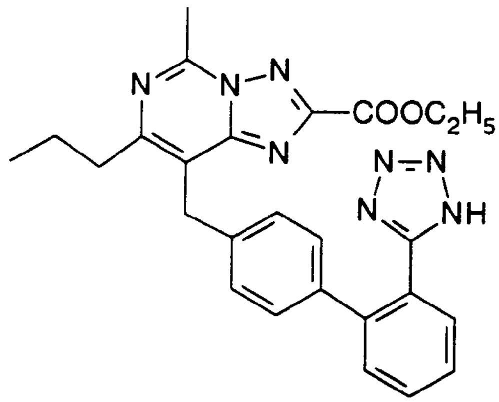

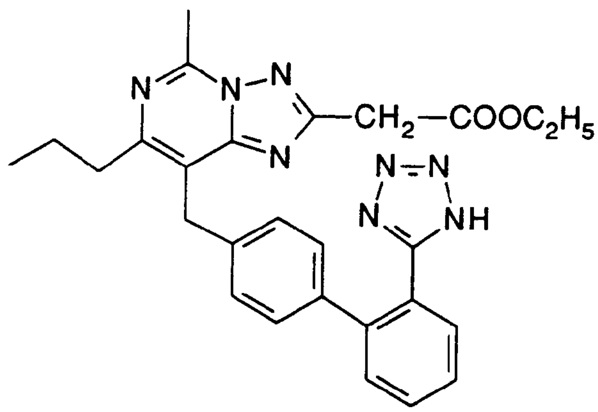

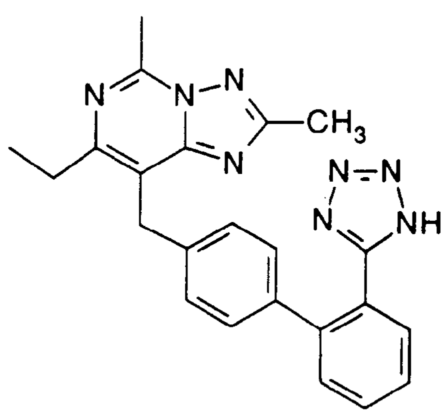

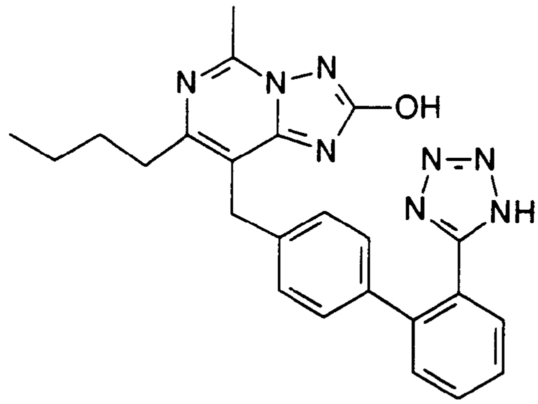

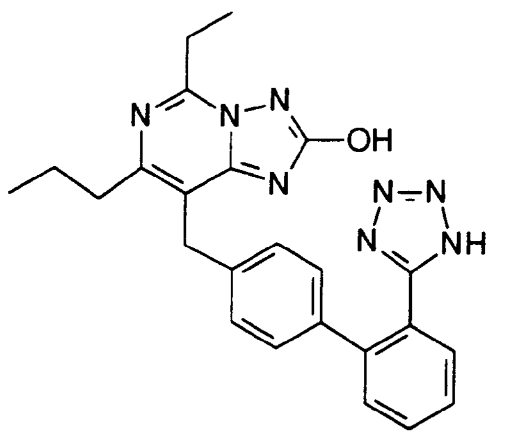

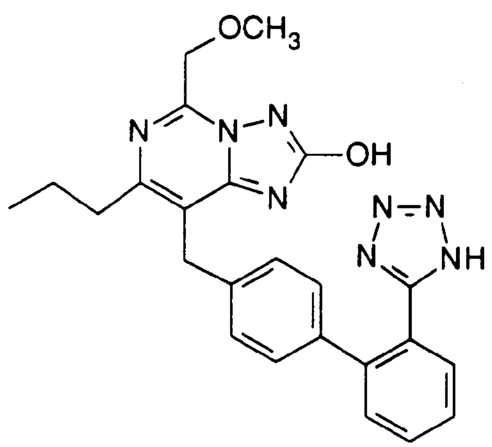

Selon l'invention, les composés de formule (I) pourront être synthétisés selon la suite de réactions suivantes :

On préparera :

- les oxo-3 alkanoates d'alkyle de formule (II) :

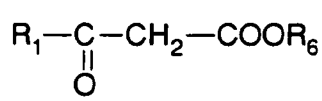

Formule (II)

dans laquelle $R_1$ est défini comme ci-dessus et $R_6$ représente un radical alkyle inférieur, de préférence méthyle ou éthyle, par des méthodes connues en soi, telles que par exemple la réaction de Claisen ou la méthode à l'acide de Meldrum, ces méthodes pouvant être trouvées dans la littérature aux références :

- OIKAWA.Y; SUGANO.K; YONEMITSU.O; J.Org.Chem., 1978, 43(10), 2087-88.
- WIERENGA.W; SKULNICK.H.I.; J.Org.Chem., 1979, 44 (2), 310-1 .
- HOUGHTON.R.P.; LAPHAM.D.J.; SYNTHESIS, 1982, 6, 451-2 .
- BRAM.G ; VILKAS.M ; Bull.Soc.Chim.France, 1964(5), 945-51.

Par benzylation des composés de formule (II), par des composés de formule (III)

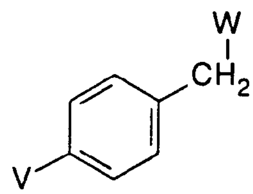

Formule (III)

en présence d'une base telle qu'un carbonate de sodium ou de potassium dans l'acétone, un alcoolate de sodium ou de potassium dans un alcool, un hydrure de sodium ou de lithium dans des solvants comme le tétrahydrofurane, le dioxane ou le diméthyl formamide par exemple, à une température comprise entre 50 et 100°C ou encore, en présence d'un équivalent de chlorure ou de bromure de lithium et de deux équivalents de diisopropyl éthyl amine au reflux du tétrahydrofurane selon la référence : SUNG-EUN YOO; KYU YANG YI; Bull. Korean. Chem. Soc. 1989, 10 (1), 112
On obtiendra les composés de formule :

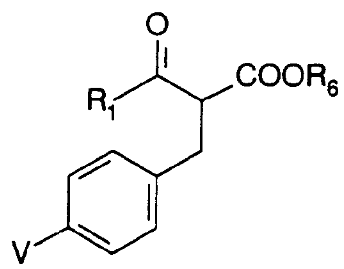

Formule (IV)

Dans la formule (IV), $R_1$, $R_6$ sont définis comme précédemment et V a la même définition que dans la formule (III).

Ces composés de formule (IV) peuvent être également obtenus par condensation d'un aldéhyde de formule

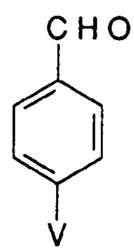

Formule (V)

sur les composés de formule (II) suivie d'une hydrogénation en présence d'un catalyseur comme le Nickel de Raney, le palladium sur charbon ou l'oxyde de platine dans un solvant comme un alcool ou le tétrahydrofurane sous pression ou à pression ordinaire lorsque les substitutions présentes le permettent.

D'une manière plus générale, on trouvera des méthodes de préparation des composés de formule (IV) dans les références suivantes :

- DURGESHWARI.P; CHAUDHURY.N.D; J.Ind.Chem.Soc, 1962,39, 735-6
- ZAUGG.H.E; DUNNIGAN. D.A ; MICHAELS. R.J ; SWETT. L.R ; J.Org.Chem., 1961, 26, 644-51 .
- BORRIES KUBEL; Liebigs.Ann.Chem, 1980, 1392-1401,.
- IOFFE.T; POPOV.E.M; VATSURO.K.V.; TULIKOVA.E.K.; KABACHNIK.M.I. ; Tetrahedron, 1962, 18, 923-940.
- SHEPHERD.T.M; Chem. Ind. (London), 1970, 17, 567.

Dans la formule (III), W représente un atome d'halogène préférentiellement le chlore ou le brome.

Dans la même formule :

- V peut être un groupement nitro, le dérivé de formule (III) est alors commercial.
- V peut être un groupement

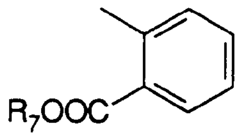

$R_7$ étant un radical alkyle inférieur ou benzyle ; les composés de formule (III) sont alors préparés par réaction d'un magnésien du p-bromo toluène sur un composé de formule :

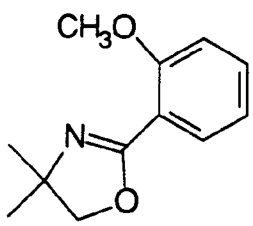

pour obtenir un composé de formule :

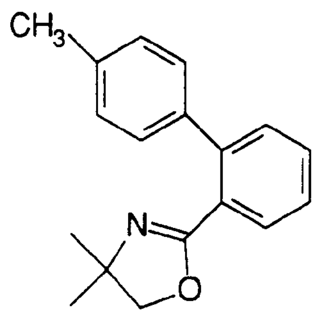

qui est ensuite hydrolysé pour conduire au composé de formule :

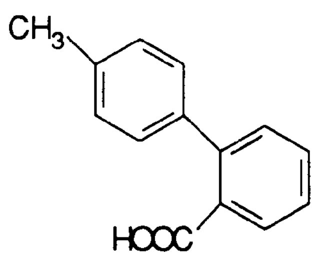

On trouvera des modes opératoires pour les trois étapes décrites ci-dessus dans la référence :

- MEYERS.A.I. ; MIHELICH E.D., J.Am.Chem.Soc., 1975, 97, 7383.

L'acide est ensuite estérifié par un alcool de formule $R_7OH$, $R_7$ étant défini comme ci-dessus.
Ces dérivés sont alors bromés ou chlorés par exemple par le N-bromo succinimide, le N-chloro succinimide ou le brome dans un solvant comme le tétrachlorure de carbone ou le dibromoéthane ou le dichloroéthane pour conduire aux composés de formule (III) où V est le groupement

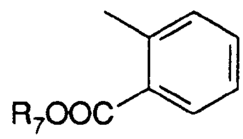

- V peut être le groupement

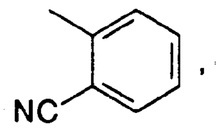

dans ce cas le composé :

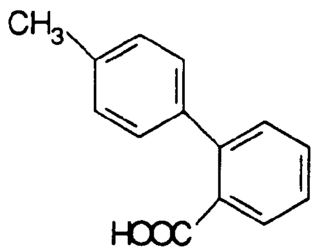

précédemment préparé sera transformé en amide primaire par action du chlorure d'acide, obtenu avec le chlorure de thionyle ou l'oxychlorure de phosphore, sur l'ammoniaque et cet amide sera transformé en nitrile par action de l'oxychlorure de phosphore dans le diméthylformamide ou du chlorure de thionyle. Le nitrile obtenu :

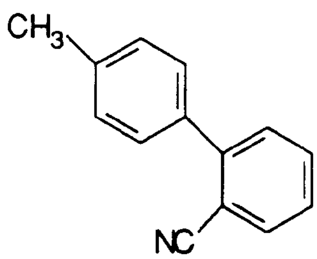

sera ensuite bromé ou chloré dans les mêmes conditions que l'ester précédent pour conduire aux composés de formule (III) où V est le groupement

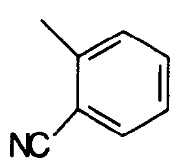

Une autre variante de préparation consiste à traiter le dérivé

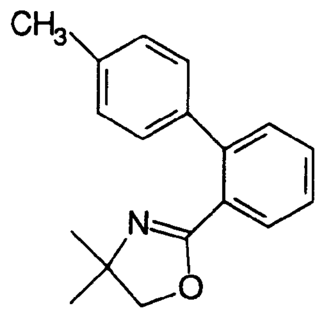

précédemment préparé, par l'oxychlorure de phosphore en présence de pyridine pour conduire au composé

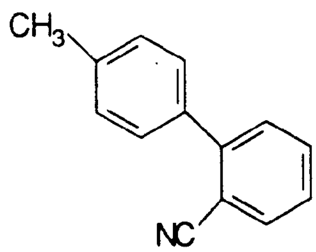

Une autre variante de réalisation consiste à préparer le magnésien du p-méthoxyméthyl bromo benzène :

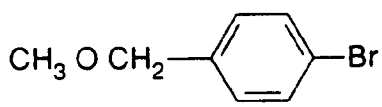

qui réagira ensuite avec le dérivé de formule

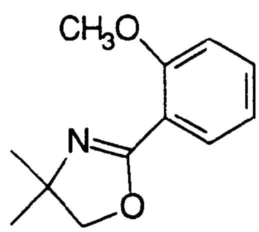

pour conduire au dérivé de formule

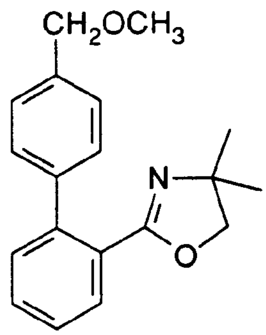

qui par déméthylation avec par exemple un traitement à $BBr_3$ conduira au composé de formule

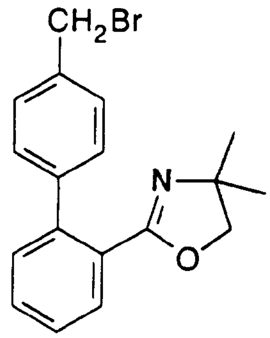

qui par condensation selon les conditions précédemment décrites avec les dérivés de formule (II) conduira aux composés de formule (IV) où V représente un groupement

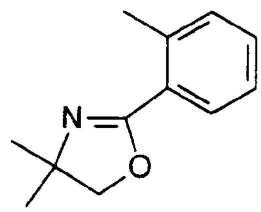

Ce composé pourra être éventuellement traité par l'oxychlorure de phosphore en présence de pyridine pour conduire aux composés de formule (IV) où V représente

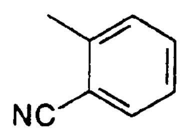

- V peut être un groupement

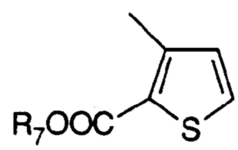,

$R_7$ étant un radical alkyle inférieur ou benzyle, les composés de formule (III) correspondants sont obtenus de la façon suivante :

A partir de l'acide (p-méthyl phényl)-3 thiophène-2 carboxylique

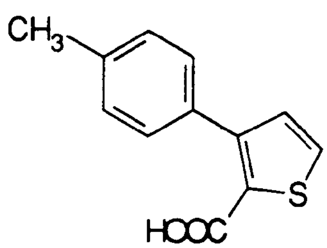

dont on peut trouver la préparation dans la référence :

-FISSELMANN. H ; HABITCH. H ; Ger. Offen. : 1,092,929 (1960) ; CA: 57 : 5894 g on obtiendra les composés de formule :

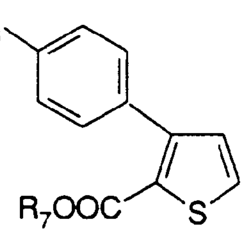

par estérification à l'aide d'un alcool de formule $R_7OH$, $R_7$ étant défini comme ci-dessus, par des méthodes classiques connues de l'homme de l'art.

Ces composés sont ensuite traités par le N-chloro succinimide ou le N-bromo succinimide dans un solvant comme le tétrachlorure de carbone ou le dibromoéthane par exemple, pour donner les composés de formule (III) dans lesquels V représente le groupement

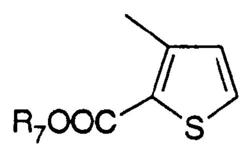 ,

$R_7$ étant défini comme ci-dessus

- V peut être le groupement

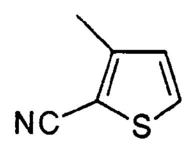

dans ce cas les composés de formule (III) correspondants seront préparés de la façon suivante :

à partir du composé acide (p-méthyl phényl)-3 thiophène-2 carboxylique dont la préparation est donnée ci-dessus, par traitement avec le chlorure de thionyle puis l'ammoniac on obtient le composé amide qui est ensuite déshydraté par le chlorure de thionyle ou l'oxychlorure de phosphore sans solvant ou dans le diméthyl formamide pour conduire au composé nitrile :

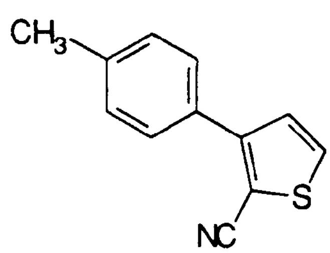

Ce composé nitrile est ensuite halogéné par le N-chloro succinimide ou le N-bromo succinimide dans un solvant comme le tétrachlorure de carbone ou le dibromoéthane pour donner les composés de formule (III) dans lesquels V représente le groupement

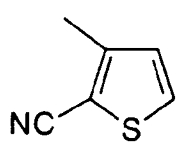

- V peut être un groupement

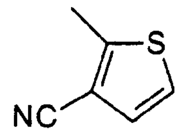

dans ce cas les composés de formule (III) correspondants sont synthétisés de la façon suivante :

à partir de la chloro-4 méthyl-4' butyrophénone de formule :

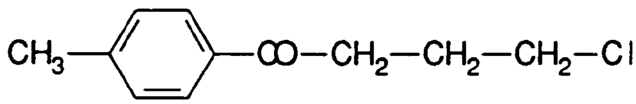

dont la préparation peut être trouvée dans le brevet BE : 577,977 du 15 mai 1959, CA : 54, 4629 c,

par traitement par l'oxychlorure de phosphore et le diméthyl formamide selon les conditions décrites dans la référence :

- VOLODINA. M.A ; TENENT'EV. A.P. ; KUDRYASHOVA. V.A. ; KABOSHINA. L.N. ; Khim. Geterosikl. Soedim ; 1967, 5-8 ;

on obtiendra le composé de formule :

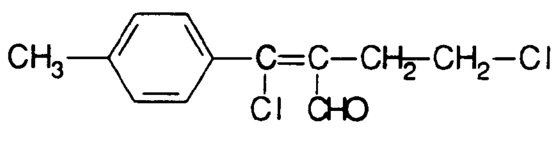

Ce composé est ensuite traité par le sulfure de sodium dans un solvant comme le tétrahydrofurane au reflux pour donner le dérivé

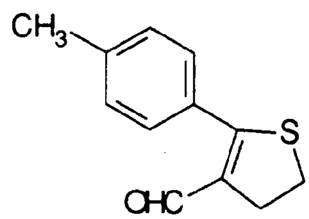

qui est ensuite transformé en deux étapes en dérivé nitrile par déshydratation de l'oxime formée à partir de l'aldéhyde et de l'hydroxylamine. Cette déshydratation pourra être effectuée par exemple à l'aide de l'anhydride acétique pour donner le composé nitrile :

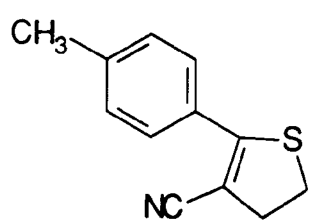

qui pourra être ensuite aromatisé par traitement avec du brome dans le tétrachlorure de carbone puis avec le tertiobutylate de potassium dans le tétrahydrofurane pour donner le composé :

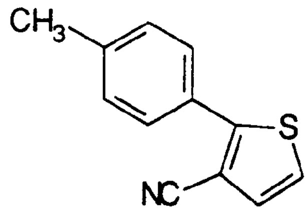

Ce composé peut être ensuite chloré ou bromé par des agents d'halogénation tels que le N-chloro succinimide ou le N-bromo succinimide dans un solvant comme le tétrachlorure de carbone ou le dibromoéthane pour donner les composés de formule (III) dans lesquels V représente le groupement

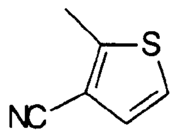

- V peut être un groupement

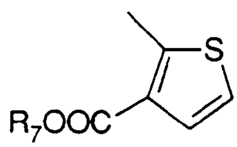

,

$R_7$ étant défini comme ci-dessus ci-dessus, dans ce cas les composés de formule (III) correspondants pourront être préparés à partir du nitrile précédemment préparé de formule :

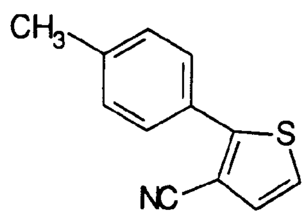

par hydrolyse classique de la fonction nitrile puis estérication de l'acide obtenu ou passage directement de la fonction nitrile à la fonction ester selon les méthodes connues de l'homme de l'art, suivie d'une chloration ou d'une bromation de l'ester par le N-chloro succinimide ou le N-bromo succinimide dans le tétrachlorure de carbone ou le dibromo éthane par exemple.

Dans la formule (V), V a la même définition que dans la formule (III) mais la méthode de condensation mettant en jeu les aldéhydes de formule (V) et les céto esters de formule (II) ne sera utilisée que lorsque V possède une fonction que respecte l'hydrogénation.

Ces composés de formule (V) pourront être préparés à partir des dérivés de formule (III) par des méthodes connues de l'homme de l'art telles que la réaction de Sommelet dont on peut trouver un exemple à la référence :
Organic Synthesis Collec. Vol. IV p. 918
ou la méthode au nitro propane décrite dans la référence :
Organic Synthesis Collec. Vol. IV p. 932

Par action, d'un composé de formule (VI)

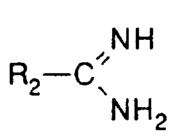

Formule (VI)

dans laquelle $R_2$ est défini comme ci-dessus,
sur les composés de formule (IV), on obtiendra, par condensation dans un alcool en présence d'un alcoolate de sodium ou de potassium à une température pouvant aller de l'ambiante à l'ébullition du solvant, les composés de formule (VII) ou leur forme tautomère

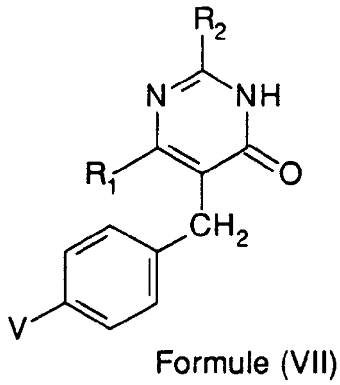

Formule (VII)

dans laquelle $R_1$, $R_2$, et V sont définis comme ci-dessus.

Les composés de formule (VI) sont commerciaux ou seront préparés selon les méthodes connues de l'homme de l'art par action de l'ammoniac en milieu alcoolique sur les imino éthers ou leur chlorhydrate de formule $R_2C(OR)=NH$ eux-mêmes préparés à partir du nitrile correspondant, de formule $R_2CN$, par traitement au gaz chlorhydrique dans l'alcool correspondant,

Par chauffage dans du $POCl_3$, par exemple, des dérivés de formule (VII), on obtiendra les dérivés de formule (VIII) :

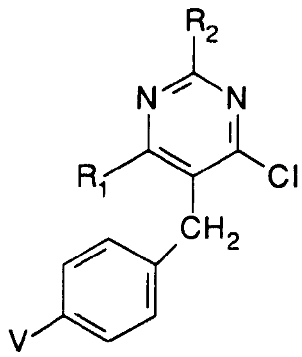

Formule (VIII)

dans laquelle $R_1$, $R_2$ et V sont définis comme ci-dessus.

Le chauffage des dérivés de formule (VIII) en présence d'hydrazine ou d'hydrate d'hydrazine dans un alcool au reflux permettra d'obtenir les dérivés de formule (IX) :

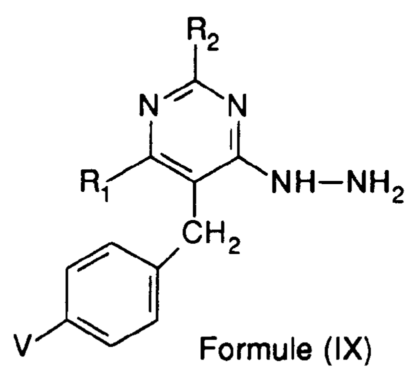

Formule (IX)

dans laquelle $R_1$, $R_2$ et V sont définis comme ci-dessus.

Ces dérivés de formule (IX) seront cyclisés par action du carbonyl di imidazole au reflux du tétrahydrofurane ou par action de l'urée par chauffage sans solvant ou dans un solvant comme la N-méthyl pyrrolidone ou par action du chloroformiate de méthyle ou d'éthyle ou par action du phosgène ou d'un précurseur de phosgène dans un solvant comme le toluène, ou par action du xanthogénate de potassium au reflux d'un alcool comme le méthoxy éthanol par exemple ou par action du sulfure de carbone dans un alcool, l'éthanol par exemple en présence ou non d'une amine comme la triéthylamine, pour conduire aux composés de formule (X) :

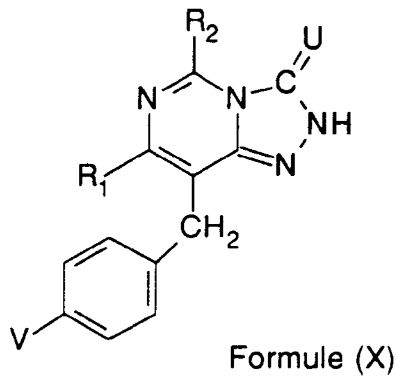

Formule (X)

dans laquelle $R_1$, $R_2$ et V sont définis comme ci-dessus et U représente l'atome d'oxygène ou de soufre.

Les dérivés de formule (X) où U est l'atome d'oxygène peuvent être également directement obtenus par chauffage des dérivés de formule (VIII) avec l'éthyl carbazate ou le méthyl carbazate.

Ces dérivés triazolo [4,3-c] pyrimidine de formule (X) pourront subir une isomérisation en milieu basique dans l'eau ou dans un mélange eau-alcool à une température comprise entre 20 et 100°, optimalement voisine de 60°, cette isomérisation pouvant également être réalisée en milieu acide par chauffage dans le dichlorobenzène en présence d'acide formique ou dans l'acide acétique en présence ou non d'acétate de sodium ou de potassium, pour conduire aux composés triazolo [1,5-c] pyrimidine de formule (XI) :

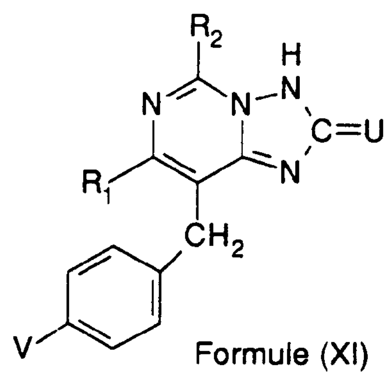

Formule (XI)

dans laquelle $R_1$, $R_2$, V et U sont définis comme ci-dessus.

Les composés de formule (XI), dans lesquels U est l'atome de soufre, peuvent également être obtenus directement par chauffage des dérivés de formule (IX) et du sulfure de carbone dans la pyridine ou le butanol au reflux.

Les dérivés de formule (X) ou de formule (XI) pourront être métallés. Selon les conditions de métallation et la nature du composé, on orientera la substitution sur l'atome d'azote ou sur l'hétéroatome U. En particulier lorsque U représente un atome de soufre, c'est principalement sur le S que se fera la substitution, lorsque U représente l'atome d'oxygène, les composés de formule (X) conduiront principalement aux dérivés N-substitués et les composés de formule (XI) conduiront principalement aux composés O-substitués. Pour privilégier les N-substitutions, on utilisera comme agent de métallation de préférence l'hydrure de sodium, l'hydrure de lithium, un alcoolate de sodium ou de potassium dans un solvant comme le diméthyl formamide, le tétrahydrofurane ou un alcool ; pour favoriser les O-substitutions on préférera comme agent de métallation la soude, la potasse, un carbonate de sodium ou de potassium dans un solvant comme l'acétone ou la méthyl éthyl cétone.

Par réaction de ces dérivés métallés avec des dérivés halogénés de formule :

$A-(CH_2)_n-CH_3$
$A-(CH_2)_n-COOR_5$
$A-(CH_2)_{n'}-O-R_5$
$A-(CH_2)_{n'}-OCOR_5$
$A-(CH_2)_{n'}-S-R_5$

dans lesquelles A est un atome d'halogène plus particulièrement le chlore ou le brome, et n, n' et $R_5$ ont la même signification que précédemment, on obtiendra les dérivés substitués sur le cycle triazole (soit sur un azote, soit par l'intermédiaire de l'atome d'oxygène ou de soufre) par les groupements :

$-(CH_2)_n-CH_3$
$-(CH_2)_n-COOR_5$
$-(CH_2)_{n'}-OR_5$
$-(CH_2)_{n'}-OCOR_5$
$-(CH_2)_{n'}-S-R_5$

(toutefois, pour réaliser ces réactions, on pourra dans certains cas, choisir de préférence les dérivés où $R_5$ est un alkyle inférieur de 1 à 6 atomes de carbone ; les dérivés où $R_5$ représente l'atome d'hydrogène seront obtenus par hydrolyse).

Les dérivés de formule (X') ou leur forme tautomère :

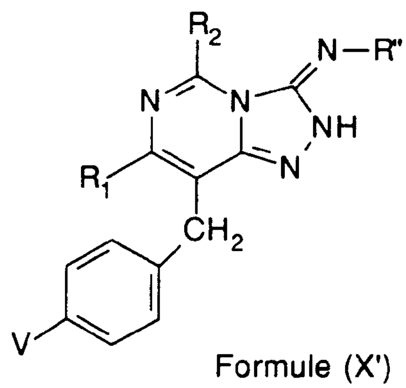

Formule (X')

dans laquelle $R_1$, $R_2$, R" et V sont définis comme ci-dessus pourront être préparés de la façon suivante :

dans le cas où R" est l'atome d'hydrogène par action du bromure de cyanogène sur les dérivés de formule (IX)

dans le cas où R" est différent de l'atome d'hydrogène en plusieurs étapes :

soit par l'action, sur les dérivés de formule (IX), d'un isocyanate de formule O=C=N-R" où R" est défini comme ci-dessus mais différent de l'atome d'hydrogène suivi d'une cyclisation de l'urée obtenue par chauffage avec du $POCl_3$ par exemple,

soit par l'action, sur le dérivés de formule (IX), d'un isothiocyanate de formule S=C=N-R" où R" est défini comme ci-dessus mais différent de l'atome d'hydrogène suivi de la méthylation en $S-CH_3$ par l'iodure de méthyle de la thiourée obtenue puis cyclisation thermique de ce dernier composé par chauffage dans un solvant approprié qui peut être par exemple un alcool.

Les dérivés de formule (XI') ou leur forme tautomère

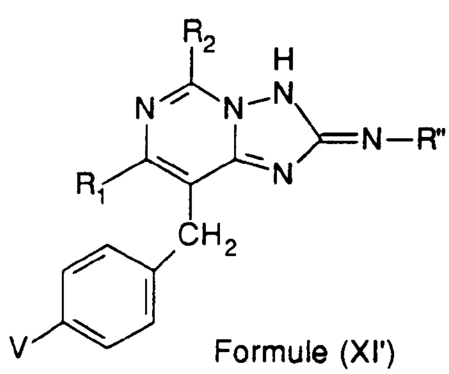

Formule (XI')

dans laquelle $R_1$, $R_2$, R" et V sont définis comme ci-dessus pourront être obtenus par isomérisation des composés de formule (X') selon les conditions d'isomérisation précédemment décrites.

Les dérivés de formule (X") :

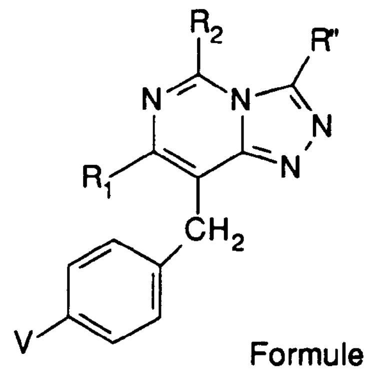

Formule (X")

dans laquelle $R_1$, $R_2$, R" et V sont définis comme ci-dessus, pourront être préparés de deux façons différentes :

soit par cyclisation par chauffage avec un ortho ester de formule R"-C $(OMe)_3$ ou R"-C $(OEt)_3$, R" étant défini comme ci-dessus, de l'hydrazino pyrimidine de formule (IX),

soit par cyclisation à l'aide de $POCl_3$ de l'hydrazide obtenu par action d'un chlorure d'acide de formule R"COCl ou de l'ester méthylique ou éthylique correspondant, R" étant défini comme ci-dessus, sur l'hydrazino pyrimidine de formule (IX).

L'isomérisation des dérivés de formule (X") conduite par chauffage dans l'acide formique ou l'acide acétique permettra d'obtenir les composés triazolo [1,5-c] pyrimidine de formule (XI") :

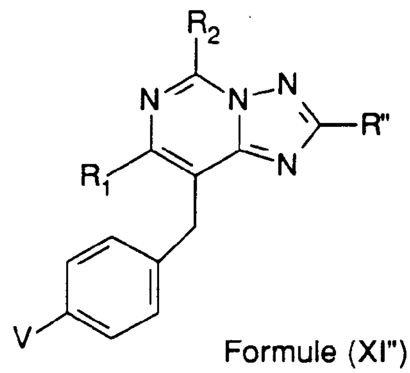

Formule (XI")

dans laquelle $R_1$, $R_2$, R" et V sont définis comme ci-dessus.

Plus généralement, on pourra utiliser certaines méthodes de préparation de triazolo pyrimidines décrites aux références suivantes :

- MILLER G.W. ; ROSE F.L. ; J. Chem. Soc., 1963, 5642-5659
- MILLER G.W. ; ROSE F.L. ; J. Chem. Soc., 1965, 3357-3368
- MILLER G.W. ; ROSE F.L. ; J. Chem. Soc., 1965, 3369-3372
- BROWN D.J. ; NAGAMATSU T. ; Aust. J. Chem., 1978(31), 2505-2515
- BROWN D.J. ; GRIGG G.W. ; IWAI Y. ; MAC ANDREW K.N. ; NAGAMATSU T. ; VAN HEESWYCK R. ; Aust. J. Chem., 1979(32), 2713-2726
- MILLER G.W. ; ROSE F.L. ; Brevet G.B. 951,652 du 11 mars 1964
- MILLER G.W. ; ROSE F.L. ; Brevet G.B. 859,287 du 18 janvier 1961.

Le traitement, par du chlorate de sodium des dérivés de formule (XI) dans lesquels U représente l'atome de soufre, dans l'acide chlorhydrique concentré à froid, permettra d'obtenir les dérivés de formule :

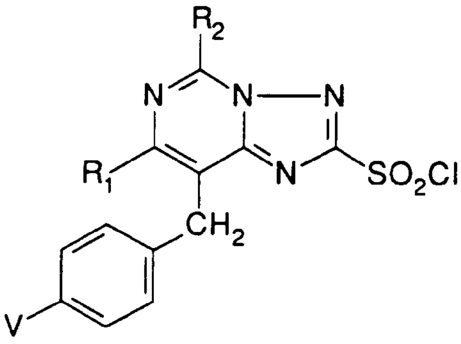

dans laquelle $R_1$, $R_2$ et V sont définis comme ci-dessus, qui seront mis en réaction avec des amines de formule HNR'R", dans laquelle R' et R" sont définis comme ci-dessus pour conduire aux dérivés de formule (XI'") :

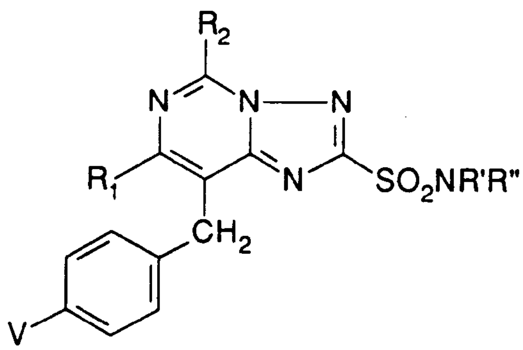

Formule (XI'")

dans laquelle $R_1$, $R_2$, R', R" et V sont définis comme ci-dessus.

L'ensemble des dérivés de formule (X), (XI), (X'), (XI'), (X"), (XI") et (XI'") peuvent être regroupés avec les dérivés substitués sur un azote du triazole ou substitués sur le triazole par l'intermédiaire d'un atome d'oxygène ou de soufre dans la formule (XII) :

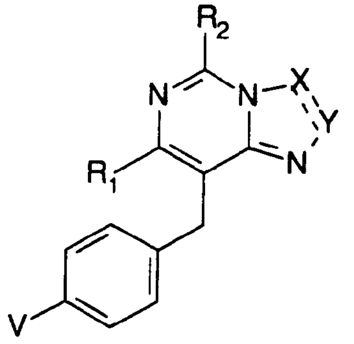

Formule (XII)

dans laquelle $R_1$, $R_2$, X, Y et V sont définis comme ci-dessus.

Une autre variante de réalisation, dans le cas où $R_2$ représente $NH-NH_2$ ou $NR_4R'_4$, consistera à traiter par l'hydrate d'hydrazine ou une amine $HNR_4R'_4$ le composé de formule (XII) où $R_2$ représente le groupement $SCH_3$, ce composé étant lui-même obtenu comme précédemment indiqué en utilisant comme composé de formule (VI) la S-méthyl thiourée ou la thiourée, le dérivé où $R_2$ = SH obtenu étant alors méthylé par l'iodure de méthyle selon les conditions connues de l'homme de l'art.

Les composés de formule (XII) dans lesquels :

- V est un groupement nitro pourront subir une hydrogénation catalytique, par exemple en présence de Nickel de Raney, dans un alcool à pression atmosphérique ou sous pression pour conduire aux composés de formule (XII) où V est un groupement amino.

Par action de l'anhydride benzosulfonique sur ces composés aminés on obtiendra les composés de formule générale (I) où $R_3$ représente le groupement

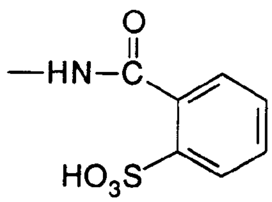

Les composés de formule (XII) dans lesquels :
-V est le groupement

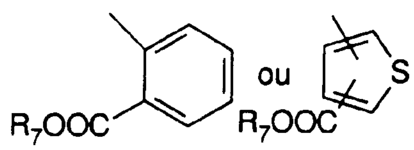

seront hydrolysés ou hydrogénés en présence d'un catalyseur tel que le palladium dans le cas où $R_7$ est un benzyle, pour conduire aux composés de formule (I) où $R_3$ est un groupement

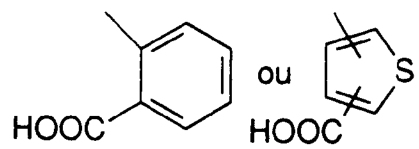

Les composés de formule (XII) dans lesquels :
- V est le groupement

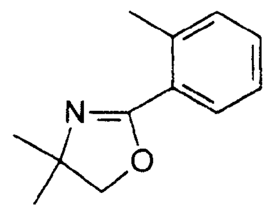

pourront être transformés en composé de formule (XII) dans lesquels V est le groupement

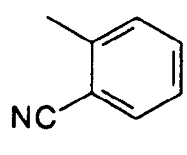

par action du $POCl_3$ en présence de pyridine par exemple

Les composés de formule (XII) dans lesquels :
- V est un groupement

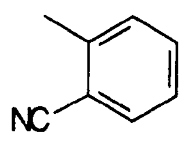

pourront réagir avec un équivalent d'azoture de sodium dans un solvant tel que le diméthyl formamide en présence d'un sel d'ammonium comme le chlorure d'ammonium ou par chauffage dans le toluène avec l'azoture de triméthyl étain suivi d'un traitement par l'acide chlorhydrique gazeux dans le tétrahydrofurane pour conduire aux composés de formule générale (I) où $R_3$ représente un groupement

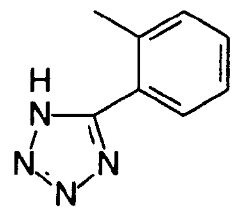

Pour mettre en oeuvre cette réaction, dans le cas où $R_2$, X ou Y possèdent une fonction alcool aliphatique il peut être souhaitable de la protéger selon les méthodes connues de l'homme de l'art par un acétate, un tétrahydropyrane puis de la libérer si nécessaire après formation du tétrazole.

Les composés de formule (XII) dans lesquels :

- V représente le groupement

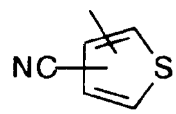

pourront être traités par un azoture de trialkyl étain dans le toluène au reflux puis par l'acide chlorhydrique gazeux dans le tétrahydrofurane pour conduire aux dérivés de formule (I) dans lesquels $R_3$ représente le groupement

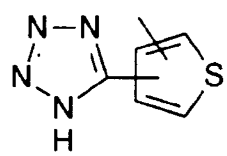

Pour mettre en oeuvre cette réaction, dans le cas où $R_2$, X ou Y possèdent une fonction alcool aliphatique, il peut être souhaitable de la protéger selon les méthodes connues de l'homme de l'art par un acétate, un tétrahydropyrane, puis de la libérer si nécessaire après formation du tétrazole.

D'autres alternatives de préparation des composés de formule (I) peuvent également être utilisées.

On pourra en particulier, préparer par action des céto esters de formule (II) sur les dérivés de formule (VI) selon les méthodes précédemment décrites, les hydroxy pyrimidine de formule (XIII)

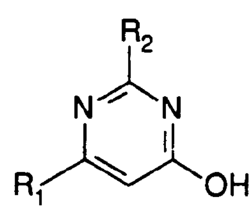

Formule (XIII)

dans laquelle $R_1$ et $R_2$ sont définis comme ci-dessus.

Ces pyrimidines seront bromées par action du brome dans l'acide acétique pour conduire aux composés de formule (XIV)

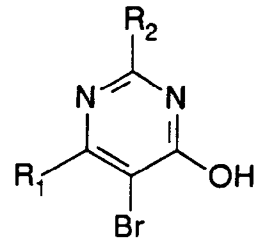

Formule (XIV)

dans laquelle $R_1$ et $R_2$ sont définis comme ci-dessus.

Les composés de formule (XIV) seront ensuite transformés comme indiqués précédemment en composés chlorés de formule (XV) par action de $POCl_3$,

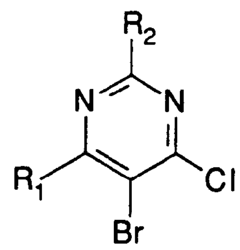

Formule (XV)

dans laquelle $R_1$ et $R_2$ sont définis comme précédemment.

Les dérivés de formule (XV) subiront les mêmes transformations que les dérivés de formule (VIII) pour conduire aux dérivés de formule (XVI)

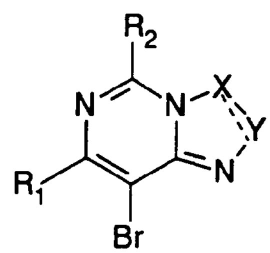

Formule (XVI)

dans laquelle $R_1$, $R_2$, X et Y sont définis comme ci-dessus.

Une autre voie de synthèse des dérivés de formule (XVI) consistera à bromer par action du brome dans l'acide acétique les dérivés de formule (XVII)

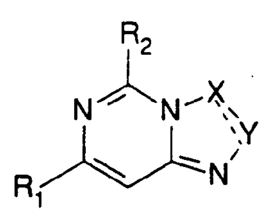

Formule (XVII)

dans laquelle $R_1$, $R_2$, X et Y sont définis comme ci-dessus, les dérivés de formule (XVII) étant préparés selon le même schéma de synthèse mais à partir des dérivés de formule (XIII).

L'action par exemple du dérivé :

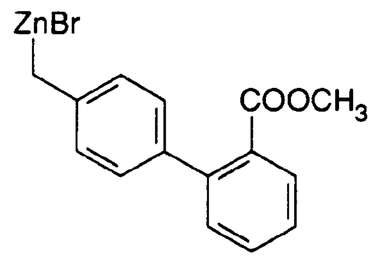

préparé à partir du dérivé de formule (III) où V est le groupement

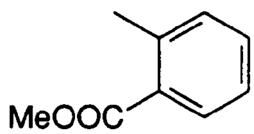

et W l'atome de brome, sur de la poudre de zinc activée dans du tétrahydrofurane sur le dérivé de formule (XVI) en présence de Pd $(PPh_3)_4$ au reflux du tétrahydrofurane permettra l'obtention des dérivés de formule (I) où $R_3$ représente le groupement

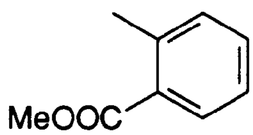

.

La fonction ester COOMe pourra être transformée en acide, amide, nitrile et tétrazole selon les suites de réaction précédemment décrites. D'une façon plus générale, l'utilisation du dérivé

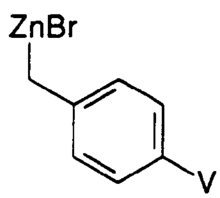

V ayant la même signification que ci-dessus et préparé de la même façon, permettra l'obtention des dérivés de formule (I).

Une autre voie d'accès aux dérivés de formule (I) consistera à traiter le dérivé de formule (XIII), dans laquelle, par exemple, $R_2 = CH_3$ selon la suite des réactions précédemment décrites :

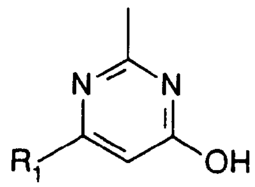

Formule (XIII)

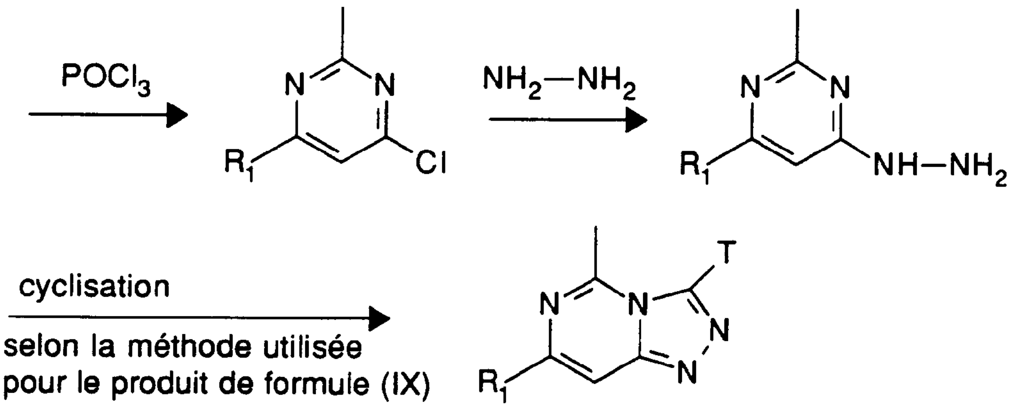


l'hydrolyse en milieu acide de la 1,2,4 triazolo [4,3-c] pyrimidine, dans laquelle $R_1$ a la même signification que ci-dessus et où T est R" précédemment défini mais représente également un groupement OH ou SH, conduira aux composés de formule (XVIII)

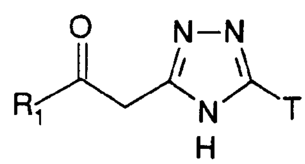

Formule (XVIII)

dans laquelle $R_1$ et T ont la même signification que ci-dessus.

Ces dérivés de formule (XVIII) pourront être condensés avec un aldéhyde de formule (V) pour conduire aux composés de formule (XIX)

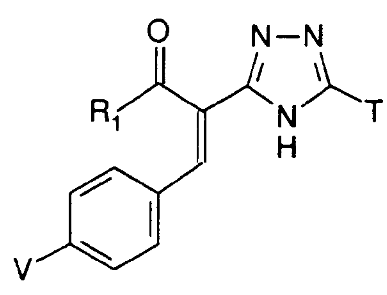

Formule (XIX)

dans laquelle $R_1$, T et V sont définis comme ci-dessus.

Ces dérivés de formule (XIX) seront cyclisés par action d'un aldéhyde de formule $R_2CHO$, $R_2$ étant défini comme ci-dessus en présence d'ammoniac pour conduire aux composés de formule (I) ou subiront une réduction à l'aide du cyanoborohydrure de sodium par exemple pour conduire aux composés de formule (XX)

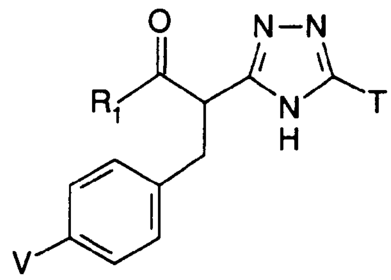

Formule (XX)

dans la formule (XX), $R_1$ V et T sont définis comme ci-dessus.

Ces dérivés de formule (XX) seront ensuite cyclisés par action d'un dérivé de formule (VI) ou par action d'un imino éther de formule $R_2C(OR)=NH$ pour conduire aux composés de formule (I).

Une autre voie d'accès aux dérivés de formule (I) consistera à préparer selon le schéma précédemment établi les composés de formule (XII) déjà citée mais où V représente un aldéhyde ou un précurseur d'aldéhyde. Dans ce cas le céto ester de formule (II) sera substitué selon les méthodes précédemment indiquées par un dérivé de formule

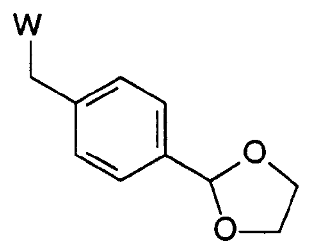

dans laquelle W est un atome d'halogène préférentiellement brome ou chlore.

Cette méthode permettra d'obtenir les dérivés de formule (XXI) après hydrolyse du kétal en milieu acide

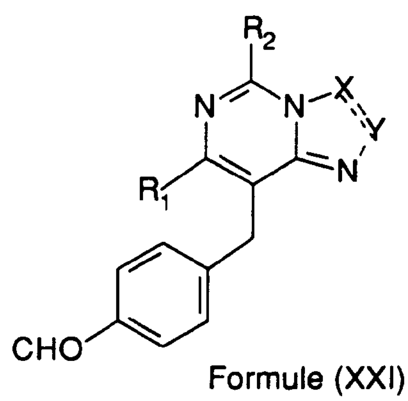

Formule (XXI)

dans laquelle $R_1$, $R_2$, X et Y sont définis comme ci-dessus ; ces aldéhydes seront condensés avec le malonitrile pour conduire aux composés de formule (XXII)

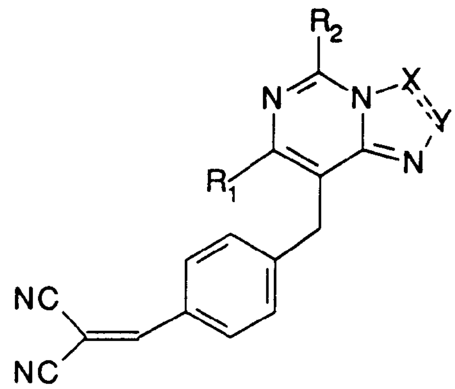

Formule (XXII)

dans laquelle $R_1$, $R_2$, X et Y sont définis comme ci-dessus, qui seront eux-mêmes condensés dans le toluène par exemple sur la butadiène-1,3-yl-4 morpholine, obtenue par condensation du crotonaldéhyde sur la morpholine, selon la référence :

-Bir SAIN ; Jagir S.SANDHU ; J. Org. Chem. 1990, 55, 2545, pour conduire aux composés de formule (XII) où V représente le groupement

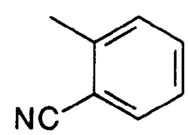

.

Le passage du nitrile à la fonction acide pourra être réalisé par hydrolyse acide ou basique, le passage de la fonction nitrile à la fonction tétrazole se fera par les méthodes précédemment décrites.

Des sels d'addition de certains composés de formule (I) peuvent s'obtenir, en particulier des sels d'addition pharmaceutiquement acceptables. On peut citer, en particulier lorsque $R_2$, $R_3$ ou R' ou R" présentent une fonction acide, les sels de sodium, potassium, calcium, d'amine comme la dicyclohexylamine ou d'amino acide comme la lysine ; et lorsque $R_2$, $R_3$, R' ou R" présentent une fonction amine, les sels d'acide minéral ou organique comme chlorhydrate, méthane sulfonate, acétate, maléate, succinate, fumarate, sulfate, lactate, citrate.

Les nouveaux composés selon l'invention possèdent des propriétés pharmacologiques remarquables comme antagonistes des récepteurs à l'angiotensine II et peuvent être utilisés en thérapeutique pour le traitement de maladies cardiovasculaires en particulier pour traiter l'hypertension, l'insuffisance cardiaque et les maladies de la paroi artérielle.

Ainsi, l'invention s'étend aux compositions pharmaceutiques renfermant comme principe actif, les médicaments constitués par une quantité pharmaceutiquement efficace d'au moins un composé de formule (I), tel que précédemment défini, ainsi qu'éventuellement ses sels d'addition pharmaceutiquement acceptables.

Ces compositions peuvent être administrées par voie buccale, rectale, par voie parentérale, par voie transdermique ou par voie oculaire.

Ces compositions peuvent être solides ou liquides et se présenter sous les formes pharmaceutiques couramment utilisées en médecine humaine comme, par exemple, les comprimés simples ou dragéifiés, les gélules, les granulés, les suppositoires, les préparations injectables, les systèmes transdermiques et les collyres. Elles sont préparées selon les méthodes usuelles. Le principe actif, constitué par une quantité pharmaceutiquement efficace d'au moins un composé de formule (I) défini comme ci-dessus ou un de ses sels d'addition pharmaceutiquement acceptables, peut y être incorporé à des excipients habituellement employés dans ces compositions pharmaceutiques, tels que le talc, la gomme arabique, le lactose, l'amidon, le stéarate de magnésium, la polyvidone, les dérivés de la cellulose, le beurre de cacao, les glycérides semi-synthétiques, les véhicules aqueux ou non, les corps gras d'origine animale ou végérale, les glycols, les divers agents mouillants, dispersants ou émulsiants, les gels de silicone, certains polymères ou copolymères, les conservateurs, arômes et colorants.

L'invention couvre encore une composition pharmaceutique à activité antagoniste des récepteurs à l'angiotensine II permettant notamment de traiter favorablement les maladies cardiovasculaires en particulier l'hypertension, l'insuffisance cardiaque et les maladies de la paroi artérielle caractérisée en ce qu'elle comprend une quantité pharmaceutiquement efficace d'au moins un composé de formule (I) précité ou un de ses sels

d'addition pharmaceutiquement acceptables, pouvant être incorporé dans un excipient, véhicule ou support pharmaceutiquement acceptable.

La posologie varie notamment en fonction de la voie d'administration, de l'affection traitée et du sujet en cause.

Par exemple, chez l'adulte de poids moyen 60 à 70 kg, elle peut varier entre 1 et 400 mg de principe actif en une ou plusieurs doses journalières par voie orale, ou de 0,01 à 50 mg en une ou plusieurs doses journalières par voie parentérale.

L'invention couvre encore un procédé de préparation d'une composition pharmaceutique, caractérisé en ce qu'on incorpore une quantité pharmaceutiquement efficace d'au moins un composé de formule (I) tel que défini précédemment, ou un de ses sels d'addition pharmaceutiquement acceptables, dans un excipient, véhicule ou support pharmaceutiquement acceptable, cette composition pharmaceutique pouvant être avantageusement préparée sous forme de gélules, de comprimés dosés de 1 à 400 mg ou sous forme de préparations injectables dosées de 0,01 à 50 mg.

L'invention couvre encore un procédé de traitement thérapeutique des mammifères, caractérisé en ce que l'on administre à ce mammifère une quantité thérapeutiquement efficace d'au moins un composé de formule (I) tel que précédemment défini, ou un de ses sels d'addition pharmaceutiquement acceptables.

En thérapeutique animale, la dose journalière utilisable devrait habituellement se situer de 1 à 100 mg par kg.

Exemple 1 : **oxo-3 hexanoate d'éthyle**

**Formule (II)** : $R_1$ = n-Propyl $R_6$ = Ethyl

176 g de diméthyl-2,2 dioxo-4,6 dioxane-1,3 (acide de Meldrum) sont dissous dans 550 ml de dichlorométhane et 188 ml de pyridine. Le mélange est refroidi à 0°C par un bain d'eau et de glace et 133 ml de chlorure de butyryle sont ajoutés goutte à goutte. A la fin de l'addition le mélange est agité trois heures à température ambiante. La solution est lavée avec une solution d'acide chlorhydrique dilué, séchée sur sulfate de magnésium et évaporée sous vide pour donner une huile. Cette huile est dissoute dans 700 ml d'éthanol et le mélange est chauffé au reflux pendant six heures. L'éthanol est évaporé sous vide et le résidu obtenu est distillé pour donner 145,4 g d'oxo-3 hexanoate d'éthyle sous forme d'un liquide de point d'ébullition $Eb_{20}$ = 98-100°C.

Exemple 2 : **Oxo-3 heptanoate d'éthyle**

**Formule (II)** : $R_1$ = n-Butyl, $R_6$ = Ethyl

Préparé selon le mode opératoire de l'exemple 1.

Liquide de point d'ébullition $Eb_{20}$ = 115-120°C.

Exemple 3 : **(nitro-4 benzyl)-2 oxo-3 hexanoate d'éthyle**

**Formule (IV)** : $R_1$ = n-Propyl V = $NO_2$ $R_6$ = Ethyl

127,7 g d'oxo-3 hexanoate d'éthyle sont dissous dans 700 ml de tétrahydrofurane. 174,5 g de bromure de nitro-4 benzyle et 35 g de chlorure de lithium sont ajoutés et le mélange est agité à température ambiante. On introduit alors goutte à goutte, 286 ml de diisopropyl éthyl amine, ce qui provoque un léger effet exothermique. Le mélange est ensuite agité trois heures à température ambiante, puis dix heures au reflux. Les solvants sont évaporés sous vide et le résidu est repris à l'eau puis extrait au chloroforme. La phase organique est décantée puis lavée avec une solution diluée d'acide chlorhydrique, séchée sur sulfate de magnésium et évaporée sous vide. Le résidu huileux obtenu est repris à l'éther isopropylique et les cristaux formés sont filtrés. Les eaux mères sont concentrées sous vide et le résidu est chauffé sous 20 mm de mercure à 130°C afin d'éliminer les produits de départ résiduels. On obtient ainsi 174 g de (nitro-4 benzyl)-2 oxo-3 hexanoate d'éthyle sous forme d'une huile utilisée telle quelle pour la suite.

Exemple 4 : **[(cyano-2' biphényl-yl-4) méthyl]-2 oxo-3 hexanoate d'éthyle**

**Formule (IV)** : $R_1$ = n-Propyl, $R_6$ = Ethyl

V = 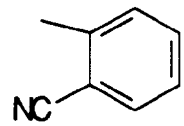

Préparé selon le mode opératoire de l'exemple 3 à partir du bromométhyl-4' cyano-2 biphényl.
Huile utilisée telle quelle pour la suite.
Préparation du bromométhyl-4' cyano-2 biphényl :

A) Méthyl-4' cyano-2 biphényl:

18,5g d'acide (méthyl-4' biphényl-yl-2) carboxylique préparé selon MEYERS A.I. ; MIHELICH E.D. ; J. Am. Chem. Soc., 1975, 97 (25), 7383, sont chauffés au reflux dans 60 ml de chlorure de thionyle pendant deux heures. Le chlorure de thionyle est concentré sous vide et le résidu est versé sur une solution à 28% d'hydroxyde d'ammonium, le mélange est agité pendant 30 minutes et les cristaux obtenus sont essorés et lavés à l'éther puis séchés pour donner 14,5g de (méthyl-4' biphényl-yl-2) carboxamide sous forme de cristaux de point de fusion 128°C. Ces cristaux sont repris dans 50 ml de chlorure de thionyle et le mélange est chauffé 3 heures au reflux puis concentré sous vide pour donner 9 g de méthyl-4' cyano-2 biphényl sous forme de cristaux de point de fusion 45-46°C.

B) Bromométhyl-4' cyano-2 biphényl :

7,9g de méthyl-4' cyano-2 biphényl préparé en A) sont dissous dans 100 ml de tétrachlorure de carbone en présence de 7,3g de N-bromo succinimide et de 0,3g de peroxyde de benzoyle. Le mélange est chauffé au reflux pendant 6 heures et les cristaux sont filtrés, la solution restante est concentrée sous vide et le résidu est cristallisé dans l'éther pour donner 6,6g de bromométhyl-4' cyano-2 biphényl sous forme de cristaux de point de fusion 115-118°C.

Exemple 5 : **[(cyano-2' biphényl-yl-4) méthyl]-2 oxo-3 heptanoate d'éthyle**

**Formule (IV)** : $R_1$ = n-Butyl, $R_6$ = Ethyl,

V = 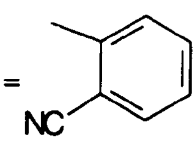

Préparé selon le mode opératoire de l'exemple 3.
Huile utilisée telle quelle pour la suite.

Exemple 6 : **n-Propyl-6 méthyl-2 hydroxy-4 (nitro-4 benzyl)-5 pyrimidine**

**Formule (VII)** : $R_1$ = n-Propyl, $R_2$ = Méthyl, V = $NO_2$

3,5 g de sodium sont dissous dans 175 ml d'éthanol. A cette solution sont ajoutés 9,5 g de chlorhydrate d'acétamidine et le mélange est agité cinq minutes à température ambiante. 20 g de (nitro-4 benzyl)-2 oxo-3 hexanoate d'éthyle, préparé à l'exemple 3, sont alors ajoutés et le mélange est agité durant quatre jours à température ambiante. Les solvants sont ensuite évaporés sous vide et le résidu est repris avec une solution d'acide chlorhydrique et extrait à l'acétate d'éthyle. La phase organique est séchée sur sulfate de magnésium puis évaporée sous vide pour donner un résidu huileux qui cristallise dans un mélange acétone/éther. Les cristaux essorés et séchés donnent 10,9 g de n-propyl-6 méthyl-2 hydroxy-4 (nitro-4 benzyl)-5 pyrimidine sous forme de cristaux de point de fusion 200°C.

Exemple 7 : **n-Propyl-6 méthyl-2 hydroxy-4 (cyano-2' biphényl-yl-4) méthyl-5 pyrimidine**

**Formule (VII) :** $R_1$ = n-Propyl, $R_2$ = Méthyl,

V = 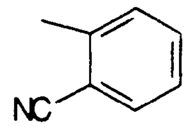

Préparé selon le mode opératoire de l'exemple 6.
Cristaux de point de fusion 206°C.

Exemple 8 : **n-Butyl-6 méthyl-2 hydroxy-4 (cyano-2' biphényl-yl-4) méthyl-5 pyrimidine**

**Formule (VII)** : $R_1$ = n-Butyl, $R_2$ = Méthyl,

V = 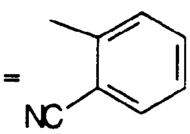

Préparé selon le mode opératoire de l'exemple 6.
Cristaux de point de fusion 173°C.

Exemple 9 : **n-Propyl-6 méthyl-2 (nitro-4 benzyl)-5 chloro-4 pyrimidine**

**Formule (VIII)** : $R_1$ = n-Propyl, $R_2$ = Méthyl, V = $NO_2$

32 g de n-propyl-6 méthyl-2 (nitro-4 benzyl)-5 hydroxy-4 pyrimidine, préparés à l'exemple 6, sont mis en suspension dans 45 ml d'oxychlorure de phosphore. Le mélange est porté au reflux durant 6 heures, puis concentré sous vide. Le résidu est repris à l'eau et extrait au dichlorométhane. La phase organique est lavée par une solution de carbonate de potassium puis séchée sur sulfate de magnésium et évaporée à sec pour donner 24 g de n-propyl-6 méthyl-2 (nitro-4 benzyl)-5 chloro-4 pyrimidine sous forme de cristaux de point de fusion 65°C.

Exemple 10 : **n-Propyl-6 méthyl-2 chloro-4 (cyano-2' biphényl-yl-4) méthyl-5 pyrimidine**

Formule (VIII) : $R_1$ = n-Propyl, $R_2$ = Méthyl,

V = 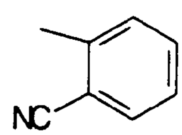

Préparé selon le mode opératoire de l'exemple 9.
Cristaux de point de fusion 95°C.

Exemple 11 : **n-Butyl-6 méthyl-2 chloro-4 (cyano-2' biphényl-yl-4) méthyl-5 pyrimidine**

**Formule (VIII)** : $R_1$ = n-Butyl, $R_2$ = Méthyl,

V = 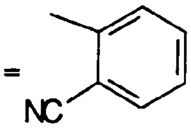

Préparé selon le mode opératoire de l'exemple 9.
Cristaux de point de fusion 75°C.

Exemple 12 : **n-Propyl-6 méthyl-2 hydrazino-4 (cyano-2' biphényl-yl-4) méthyl-5 pyrimidine**

**Formule (IX)** : $R_1$ = n-Propyl, $R_2$ = méthyl,

V = 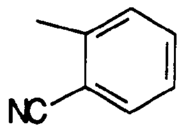

51,7 g de n-Propyl-6 méthyl-2 chloro-4 (cyano-2' biphényl-yl-4) méthyl-5 pyrimidine préparés à l'exemple 10 sont dissous dans 150 ml d'éthanol et 90 ml d'hydrate d'hydrazine. Le mélange est chauffé au reflux pendant 6 heures et le solvant est concentré à demi sous vide puis additionné d'eau. Les cristaux formés sont essorés, lavés à l'eau puis à l'éther et séchés pour donner 46 g de n-Propyl-6 méthyl-2 hydrazino-4 [(cyano-2' biphényl-yl-4) méthyl]-5 pyrimidine sous forme de cristaux de point de fusion 156°C.

Exemple 13 : **n-Propyl-6 méthyl-2 hydrazino-4 (nitro-4 benzyl)-5 pyrimidine**

**Formule (IX)** : $R_1$ = n-Propyl, $R_2$ = méthyl, V = $NO_2$
Préparé selon le mode opératoire de l'exemple 12.
Cristaux de point de fusion 126°C.

Exemple 14 : **n-Butyl-6 méthyl-2 hydrazino-4 (cyano-2' biphényl-yl-4) méthyl-5 pyrimidine**

**Formule (IX)** : $R_1$ = n-Butyl, $R_2$ = méthyl,

V = 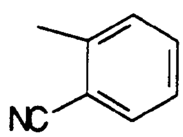

Préparé selon le mode opératoire de l'exemple 12.
Cristaux de point de fusion 154°C.

Exemple 15 : **n-Propyl-7 méthyl-5 (cyano-2' biphényl-yl-4) méthyl-8 1,2,4-triazolo [4,3-c] pyrimidine one-3 (2H)**

**Formule (XII)** : $R_1$ = n-Propyl, $R_2$ = méthyl, X = CO,

V = 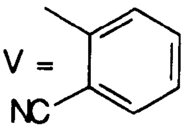 , Y = NH, X ⩦ Y = liaison simple

33,4 g de n-Propyl-6 méthyl-2 hydrazino-4 [(cyano-2' biphényl-yl-4) méthyl]-5 pyrimidine préparés à l'exemple 12 sont dissous dans 600 ml de tétrahydrofurane. 15,2 g de carbonyl diimidazole sont ajoutés et le mélange est chauffé au reflux pendant 1 h 30 . Le solvant est évaporé sous vide et le résidu est repris à l'eau puis extrait au chloroforme. La phase organique est séchée sur sulfate de magnésium et évaporée sous vide ; le résidu obtenu cristallise dans un mélange éther / acétate d'éthyle pour donner 26,4 g de n-Propyl-7 méthyl-5 (cyano-2' biphényl-yl-4) méthyl-8 1,2,4-triazolo [4,3-c] pyrimidine one-3 (2H) sous forme de cristaux de point de fusion 196°C.

Exemple 16 : **n-Propyl-7 méthyl-5 (nitro-4 benzyl)-8 1,2,4-triazolo [4,3-c] pyrimidine one-3 (2H)**

**Formule (XII):** $R_1$ = n-Propyl, $R_2$ = méthyl, X = CO, Y = NH, X ⩦ Y = liaison simple, V = $NO_2$
Préparé selon le mode opératoire de l'exemple 15.
Cristaux de point de fusion 225°C

Exemple 17 : **n-Butyl-7 méthyl-5 (cyano-2' biphényl-yl-4) méthyl-8 1,2,4-triazolo [4,3-c] pyrimidine one-3 (2H)**

**Formule (XII)** : $R_1$ = n-Butyl, $R_2$ = méthyl, X = CO,

Y = NH, X ⩦ Y = liaison simple, V = 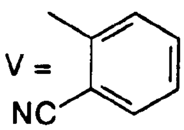

Préparé selon le mode opératoire de l'exemple 15.
Cristaux de point de fusion 173°C

Exemple 18 : **n-Propyl-7 méthyl-5 (cyano-2' biphényl-yl-4) méthyl-8 1,2,4-triazolo [1,5-c] pyrimidine one-2 (3H)**

**Formule (XII)** : $R_1$ = n-Propyl, $R_2$ = méthyl, Y = CO,

X = NH , X ⩦ Y = liaison simple, V = 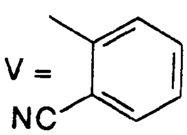

13,8 g de n-Propyl-7 méthyl-5 [(cyano-2' biphényl-yl-4) méthyl]-8 1,2,4-triazolo [4,3-c] pyrimidine one-3 (2H) préparés à l'exemple 15, sont dissous dans 40 ml d'éthanol et 150 ml d'une solution de potasse 3N. Le mélange est chauffé 4 heures à 60°C puis 100 ml d'eau sont rajoutés. La solution est acidifiée par l'acide chlorhydrique concentré et les cristaux obtenus sont essorés, lavés à l'eau et repris dans du chloroforme. La solution chloroformique est séchée sur sulfate de magnésium et évaporée sous vide. Le résidu obtenu cristallise dans un mélange acétate d'éthyle / éther pour donner 10,6 g de cristaux qui sont chromatographiés sur gel de silice avec un éluant chloroforme / méthanol 9/1 pour donner 8,4 g de n-Propyl-7 méthyl-5 [(cyano-2' biphényl-yl-4) méthyl]-8 1,2,4-triazolo [1,5-c] pyrimidine one-2 (3H) sous forme de cristaux de point de fusion 226°C.

Exemple 19 : **n-Propyl-7 méthyl-5 [[(tétrazolyl-5)-2' biphényl-yl-4] méthyl]-8 1,2,4-triazolo [4,3-c] pyrimidine one-3 (2H)**

**Formule (I)** : $R_1$ = n-Propyl $R_2$ = méthyl, X = CO, Y = NH,

X $\overset{--}{-}$ Y = liaison simple, $R_3$ = 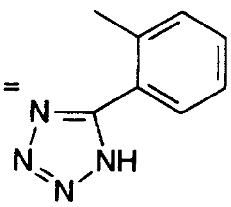

4 g de n-Propyl-7 méthyl-5 [(cyano-2' biphényl-yl-4) méthyl]-8 1,2,4-triazolo [4,3-c] pyrimidine one-3 (2H) préparés à l'exemple 15 sont dissous dans 100 ml de toluène. 2,8 g d'azoture de triméthyl étain sont ajoutés et le mélange est chauffé 24 heures au reflux. Les cristaux formés sont essorés à chaud et lavés à l'éther puis mis en suspension dans 100 ml de tétrahydrofurane. Du gaz chlorhydrique est mis à barbotter dans le mélange et après passage complet en solution un précipité apparaît. Le mélange est abandonné une nuit à température ambiante et les cristaux formés sont essorés, lavés à l'éther et dissous dans une solution de soude diluée. Cette solution est lavée à l'éther puis acififiée par barbottage de dioxyde de soufre et extraite au chloroforme. La phase organique est séchée sur sulfate de magnésium et évaporée sous vide et le résidu cristallise dans un mélange éther / acétone pour donner 1,5 g de n-Propyl-7 méthyl-5 [[(tétrazolyl-5)-2' biphényl-yl-4] méthyl]-8 1,2,4-triazolo [4,3-c] pyrimidine one-3 (2H) sous forme de cristaux de point de fusion 248-249°C.

Exemple 20 : **n-Propyl-7 méthyl-5 [[(tétrazolyl-5)-2' biphényl-yl-4] méthyl]-8 1,2,4-triazolo [1,5-c] pyrimidine one-2 (3H)**

**Formule (I)** : $R_1$ = n-Propyl, $R_2$ = méthyl, X = NH, Y = CO,

X $\overset{--}{-}$ Y = liaison simple, $R_3$ = 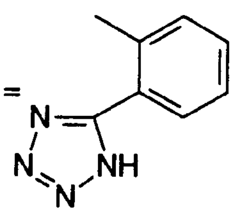

Préparé selon le mode opératoire de l'exemple 19 à partir du composé de l'exemple 18.

Peut être également préparé selon le mode opératoire de l'exemple 18 à partir du composé de l'exemple 19.

Recristallisé dans l'acide acétique, puis lavé à l'acétate d'éthyle.

Cristaux de point de fusion 239°C.

Exemple 21 : **[n-Propyl-7 méthyl-5 oxo-3 [(cyano-2' biphényl-yl-4) méthyl]-8 1,2,4-triazolo [4,3-c] pyrimidine (2H) yl-2] acétate d'éthyle**

**Formule (XII)** : $R_1$ = n-Propyl, $R_2$ = méthyl, X = CO,

Y = N $CH_2CO_2Et$, X $\overset{--}{-}$ Y = liaison simple ,

V = 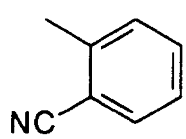

3,8 g de n-Propyl-7 méthyl-5 [(cyano-2' biphényl-yl-4) méthyl]-8 1,2,4-triazolo [4,3-c] pyrimidine one-3 (2H) préparés à l'exemple 15, sont dissous dans 50 ml d'éthanol. Une solution d'éthylate de sodium préparée à partir de 0,25 g de sodium dans 10 ml d'éthanol est ajoutée et le mélange est agité 10 minutes à température ambiante. 1,3 ml de bromoacétate d'éthyle sont ajoutés et le mélange est chauffé au reflux pendant 7 heures. Le solvant est concentré sous vide et le résidu est repris à l'eau et extrait à l'éther. La phase organique est

lavée avec une solution froide de soude diluée puis séchée et évaporée sous vide pour donner 4,3 g de [n-Propyl-7 méthyl-5 oxo-3 [(cyano-2' biphényl-yl-4) méthyl]-8 1,2,4-triazolo [4,3-c] pyrimidine (2H) yl-2] acétate d'éthyle sous forme d'huile utilisée telle quelle pour la suite.

Exemple 22 : **[n-Propyl-7 méthyl-5 oxo-3 [[(tétrazolyl-5)-2' biphényl-yl-4] méthyl]-8 1,2,4-triazolo [4,3-c] pyrimidine (2H) yl-2] acétate d'éthyle**

**Formule (I)** : $R_1$ = n-Propyl, $R_2$ = méthyl, X = CO,
Y = $NCH_2CO_2Et$,

X ⩦ Y = liaison simple, $R_3$ = 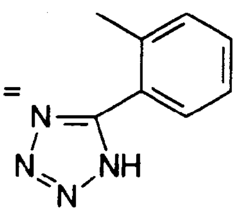

Préparé selon le mode opératoire de l'exemple 19.
Cristaux de point de fusion 173-174°C.

Exemple 23 : **[n-Propyl-7 méthyl-5 oxo-3 [(cyano-2' biphényl-yl-4) méthyl]-8 1,2,4-triazolo [4,3-c] pyrimidine (2H) yl-2]-2 éthanol**

**Formule (XII)** : $R_1$ = n-Propyl, $R_2$ = méthyl, X = CO,
Y = N $CH_2CH_2OH$ , X ⩦ Y = liaison simple ,
V = 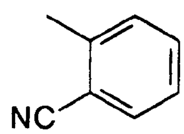

Préparé selon le mode opératoire de l'exemple 21 à partir du bromo-2 éthanol.
Cristaux de point de fusion 112°C.

Exemple 24 : **n-Propyl-7 diméthyl-2,5 [(cyano-2' biphényl-yl-4) méthyl]-8 1,2,4-triazolo [4,3-c] pyrimidine one-3 (2H)**

**Formule (XII)** : $R_1$ = n-Propyl, $R_2$ = méthyl, X = CO,
Y = $NCH_3$ , X ⩦ Y = liaison simple ,
V = 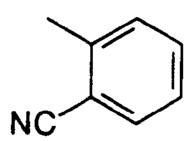

Préparé selon le mode opératoire de l'exemple 21 à partir de l'iodure de méthyle.
Cristaux de point de fusion 145°C.

Exemple 25 : **[n-Propyl-7 méthyl-5 oxo-3 [[(tétrazolyl-5)-2' biphényl-yl-4] méthyl]-8 1,2,4-triazolo [4,3-c] pyrimidine (2H) yl-2]-2 éthanol**

**Formule (I)** : $R_1$ = n-Propyl, $R_2$ = méthyl, X = CO,
Y = $NCH_2CH_2OH$,

X $\overset{--}{-}$ Y = liaison simple, $R_3$ =

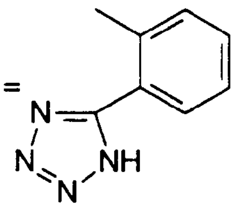

Préparé selon le mode opératoire de l'exemple 19.
Cristaux de point de fusion 149-150°C.

Exemple 26 : **n-Propyl-7 diméthyl-2,5 [[(tétrazolyl-5)-2' biphényl-yl-4] méthyl]-8 1,2,4-triazolo [4,3-c] pyrimidine one-3 (2H)**

**Formule (I)** : $R_1$ = n-Propyl, $R_2$ = méthyl, X = CO,
Y = $NCH_3$,

X $\overset{--}{-}$ Y = liaison simple, $R_3$ =

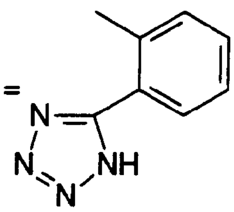

Préparé selon le mode opératoire de l'exemple 19.
Cristaux de point de fusion 205-206°C.

Exemple 27 : **n-Propyl-7 méthyl-5 méthoxy-2 [(cyano-2' biphényl-yl-4) méthyl]-8 1,2,4-triazolo [1,5-c] pyrimidine**

**Formule (XII)** : $R_1$ = n-Propyl, $R_2$ = méthyl, X = N,
Y = $C^{-}OCH_3$ , X $\overset{--}{-}$ Y = double liaison

V =

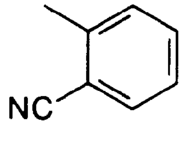

4,4 g de n-Propyl-7 méthyl-5 [(cyano-2' biphényl-yl-4) méthyl]-8 pyrimidine one-2 (3H) préparés à l'exemple 18 sont dissous dans 50 ml d'acétone et 2 g de carbonate de potassium sont ajoutés. Après addition de 2 ml d'iodure de méthyle, le mélange est porté au reflux pendant 5 heures, refroidi et concentré sous vide, puis additionné d'eau et extrait au dichlorométhane. La phase organique est séchée sur sulfate de magnésium et évaporée sous vide, le résidu est chromatographié sur gel de silice dans un éluant chloroforme / acétone 80/20 pour donner 3 g de n-Propyl-7 méthyl-5 méthoxy-2 [(cyano-2' biphényl-yl-4) méthyl]-8 1,2,4-triazolo [1,5-c] pyrimidine (premier produit élué) sous forme de cristaux de point de fusion 89°C.

Exemple 28 : **n-Propyl-7 méthyl-5 méthoxy-2 [[(tétrazolyl-5)-2' biphényl-yl-4] méthyl]-8 1,2,4-triazolo [1,5-c] pyrimidine**

**Formule (I)** : $R_1$ = n-Propyl, $R_2$ = méthyl, X = N,
Y = C-OCH$_3$,

X ---- Y = double liaison, $R_3$ =

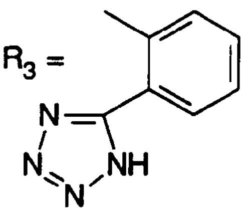

Préparé selon le mode opératoire de l'exemple 19.
Cristaux de point de fusion 189-190°C.

Exemple 29 : **n-Propyl-7 diméthyl-3,5 [(cyano-2' biphényl-yl-4) méthyl]-8 1,2,4-triazolo [1,5-c] pyrimidine one-2 (3H)**

**Formule (XII)** : $R_1$ = n-Propyl, $R_2$ = méthyl, X = N-CH$_3$,
Y = CO , X ---- Y = liaison simple ,

V =

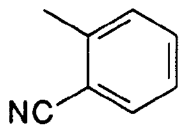

Préparé selon le mode opératoire de l'exemple 27, purifié par chromatographie sur gel de silice avec un éluant chloroforme/méthanol 90/10 (deuxième produit élué).
Cristaux de point de fusion 194°C.

Exemple 30 : **n-Propyl-7 méthyl-5 (amino-4 benzyl)-8 1,2,4-triazolo [4,3-c] pyrimidine one-3 (2H)**

**Formule (XII)** : $R_1$ = n-Propyl, $R_2$ = méthyl, X = CO, Y = NH, X ---- Y = liaison simple , V = NH$_2$

5,4 g de n-Propyl-7 méthyl-5 (nitro-4 benzyl)-8 1,2,4-triazolo [4,3-c] pyrimidine one-3 (2H) préparés à l'exemple 16, sont dissous dans 100 ml de méthanol et sont mis à hydrogéner à pression atmosphérique et température ambiante en présence de 0,8 g de Nickel de Raney. Quand la prise d'hydrogène a cessé, le catalyseur est filtré et le solvant évaporé sous vide pour donner 4,6 g de n-Propyl-7 méthyl-5 (amino-4 benzyl)-8 1,2,4-triazolo [4,3-c] pyrimidine one-3 (2H) sous forme de cristaux de point de fusion 180°C.

Exemple 31 : **Acide [[n-Propyl-7 méthyl-5 oxo-3 1,2,4-triazolo [4,3-c] pyrimidine (2H) yl-8] méthyl-4 phényl] amino carbonyl-2 benzène sulfonique**

**Formule (I)** : $R_1$ = n-Propyl, $R_2$ = méthyl, X = CO,

Y = NH , X $\overline{--}$ Y = liaison simple ,

$R_3$ =

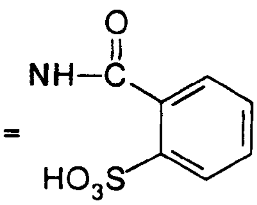

4,6 g de n-Propyl-7 méthyl-5 (amino-4 benzyl)-8 1,2,4-triazolo [4,3-c] pyrimidine one-3 (2H) préparés à l'exemple 30, sont dissous dans 300 ml d'acétonitrile et une solution de 2,9 g d'anhydride sulfobenzoïque dans 30 ml d'acétonitrile est ajoutée. Le mélange est agité pendant 15 minutes et les cristaux formés sont essorés et lavés à l'éther, puis dissous dans une solution aqueuse de bicarbonate de soude. La phase aqueuse est alors acidifiée par barbottage de dioxyde de soufre pour donner 4 g d'acide [[n-Propyl-7 méthyl-5 oxo-3 1,2,4-triazolo [4,3-c] pyrimidine (2H) yl-8] méthyl-4 phényl] amino carbonyl-2 benzène sulfonique sous forme de cristaux de point de fusion 283-286°C.

Exemple 32 : **[n-Propyl-7 méthyl-5 oxo-3 (nitro-4 benzyl)-8 1,2,4-triazolo [4,3-c] pyrimidine (2H) yl-2] acétate d'éthyle**

**Formule (XII)** : $R_1$ = n-Propyl, $R_2$ = méthyl, X = CO, Y = N $CH_2CO_2Et$, X $\overline{--}$ Y = liaison simple, V = $NO_2$
Préparé selon le mode opératoire de l'exemple 21.
Cristaux de point de fusion 144°C.

Exemple 33 : **[n-Propyl-7 méthyl-5 oxo-3 (amino-4 benzyl)-8 1,2,4-triazolo [4,3-c] pyrimidine (2H) yl-2] acétate d'éthyle**

**Formule (XII)** : $R_1$ = n-Propyl, $R_2$ = méthyl, X = CO, Y = N $CH_2CO_2Et$, X $\overline{--}$ Y = liaison simple, V = $NH_2$
Préparé selon le mode opératoire de l'exemple 30.
Cristaux de point de fusion 130°C.

Exemple 34 : **Acide [[n-Propyl-7 méthyl-5 oxo-3 (éthoxycarbonyl méthyl)-2 1,2,4-triazolo [4,3-c] pyrimidine (2H) yl-8] méthyl-4 phényl] amino carbonyl-2 benzène sulfonique**

**Formule (I)** : $R_1$ = n-Propyl, $R_2$ = méthyl, X = CO,

Y = N $CH_2CO_2Et$, X $\overline{--}$ Y = liaison simple ,

$R_3$ =

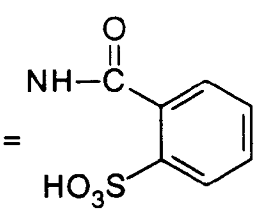

Préparé selon le mode opératoire de l'exemple 31.
Cristaux de point de fusion 282-284°C.

Exemple 35 : **Acide [[n-Propyl-7 méthyl-5 oxo-3 (carboxy méthyl)-2 1,2,4-triazolo [4,3-c] pyrimidine (2H) yl-8] méthyl-4 phényl] amino carbonyl-2 benzène sulfonique**

**Formule (I)** : $R_1$ = n-Propyl, $R_2$ = méthyl, X = CO,

Y = $NCH_2CO_2H$ , X --- Y = liaison simple ,

$R_3$ = 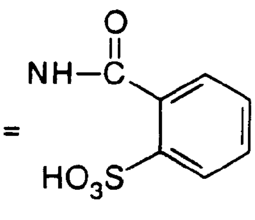

2,5 g d'acide [[n-Propyl-7 méthyl-5 oxo-3 (éthoxycarbonyl méthyl)-2 1,2,4-triazolo [4,3-c] pyrimidine (2H) yl-8] méthyl-4 phényl] amino carbonyl-2 benzène sulfonique, préparé à l'exemple 34 sont dissous dans 30 ml d'eau contenant 1 g de soude. Le mélange est chauffé 2 h à 60°C, refroidi et acidifié par l'acide chlorhydrique pour donner 2 g d'acide [[n-Propyl-7 méthyl-5 oxo-3 (carboxy méthyl)-2 1,2,4-triazolo [4,3-c] pyrimidine (2H) yl-8] méthyl-4 phényl] amino carbonyl-2 benzène sulfonique sous forme de cristaux de point de fusion 296-300°C.

Exemple 36 : **n-Butyl-7 méthyl-5 [[(tétrazolyl-5)-2' biphényl-yl-4] méthyl]-8 1,2,4-triazolo [4,3-c] pyrimidine one-3 (2H)**

**Formule (I)** : $R_1$ = n-Butyl, $R_2$ = méthyl, X = CO, Y = NH,

X --- Y = liaison simple, $R_3$ = 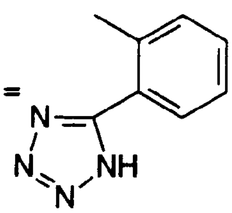

Préparé selon le mode opératoire de l'exemple 19.
Cristaux de point de fusion 233-235°C.

Exemple 37 : **n-Propyl-7 méthyl-5 mercapto-2 (nitro-4 benzyl)-8 1,2,4-triazolo [1,5-c] pyrimidine**

**Formule (XII)** : $R_1$ = n-Propyl, $R_2$ = méthyl, X = N, Y = C-SH, X -- Y = double liaison V = $NO_2$

3,7 g de n-Propyl-6 méthyl-2 hydrazino-4 (nitro-4 benzyl)-5 pyrimidine préparés à l'exemple 13, sont dissous dans 50 ml de n-butanol en présence de 1,5 ml de sulfure de carbone. Le mélange est chauffé au reflux pendant 3 heures, puis refroidi et les cristaux obtenus sont essorés et lavés à l'éther puis séchés pour donner 3,5 g de n-Propyl-7 méthyl-5 mercapto-2 (nitro-4 benzyl)-8 1,2,4-triazolo [1,5-c] pyrimidine sous forme de cristaux de point de fusion 210°C.

Exemple 38 : **n-Propyl-7 méthyl-5 méthylmercapto-2 (nitro-4 benzyl)-8 1,2,4-triazolo [1,5-c] pyrimidine**

**Formule (XII)** : $R_1$ = n-Propyl, $R_2$ = méthyl, X = N, Y = C-$SCH_3$, X -- Y = double liaison V = $NO_2$

5 g de n-Propyl-7 méthyl-5 mercapto-2 (nitro-4 benzyl)-8 1,2,4-triazolo [1,5-c] pyrimidine préparés à l'exemple 37 sont dissous dans 50 ml de chloroforme et 2,2 ml de triéthylamine. 1.5 ml d'iodure de méthyle sont ajoutés et le mélange est agité à température ambiante pendant 2 heures, puis abandonné une nuit. Le mélange est ensuite lavé avec une solution de soude diluée et la phase organique est décantée, séchée sur sulfate de magnésium et évaporée sous vide pour donner un résidu qui cristallise dans un mélange éther/pentane pour fournir 4 g de n-Propyl-7 méthyl-5 méthylmercapto-2 (nitro-4 benzyl)-8 1,2,4-triazolo [1,5-c] pyrimidine sous forme de cristaux de point de fusion 130°C.

Exemple 39 : **n-Propyl-7 méthyl-5 méthylmercapto-2 (amino-4 benzyl)-8 1,2,4-triazolo [1,5-c] pyrimidine**

**Formule (XII)** : $R_1$ = n-Propyl, $R_2$ = méthyl, X = N, Y = $C-SCH_3$, X $\underline{--}$ Y = double liaison V = $NH_2$
Préparé selon le mode opératoire de l'exemple 30.
Huile utilisée telle quelle pour la suite.

Exemple 40 : **Acide [[n-Propyl-7 méthyl-5 méthylmercapto-2 1,2,4-triazolo [1,5-c] pyrimidine-yl-8] méthyl-4 phényl] amino carbonyl-2 benzène sulfonique**

**Formule (I)** : $R_1$ = n-Propyl, $R_2$ = méthyl, X = N,
Y = $C-SCH_3$, X $\underline{--}$ Y = double liaison

$R_3$ = 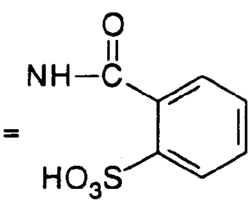

Préparé selon le mode opératoire de l'exemple 31.
Cristaux de point de fusion 250-252°C.

Exemple 41 : **n-Propyl-7 méthyl-5 mercapto-2 [(cyano-2' biphényl-yl-4) méthyl]-8 1,2,4-triazolo [1,5-c] pyrimidine**

**Formule (XII)** : $R_1$ = n-Propyl, $R_2$ = méthyl, X = N,
Y = $C-SH$, X $\underline{--}$ Y = double liaison

V = 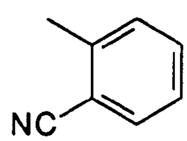

Préparé selon le mode opératoire de l'exemple 37.
Cristaux de point de fusion 202°C.

Exemple 42 : **n-Propyl-7 méthyl-5 mercapto-2 [[(tétrazolyl-5)-2' biphényl-yl-4] méthyl]-8 1,2,4-triazolo [1,5-c] pyrimidine**

**Formule (I)** : $R_1$ = n-Propyl, $R_2$ = méthyl, X = N, Y = C-SH

X $\underline{--}$ Y = double liaison, $R_3$ = 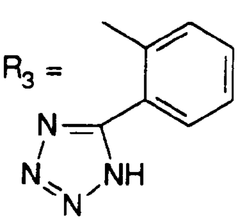

Préparé selon le mode opératoire de l'exemple 19.
Cristaux de point de fusion 223-225°C.

Exemple 43 : **[n-Propyl-7 méthyl-5 [(cyano-2' biphényl-yl-4) méthyl]-8 1,2,4-triazolo [1,5-c] pyrimidine-yl-2] mercapto acétate d'éthyle**

**Formule (XII)** : $R_1$ = n-Propyl, $R_2$ = méthyl, X = N,

Y = $C\text{-}SCH_2\text{-}CO_2\text{-}Et$, X $\stackrel{\text{- -}}{\text{—}}$ Y = double liaison

V = 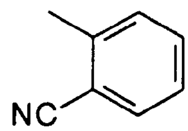

4 g de n-Propyl-7 méthyl-5 [(cyano-2' biphényl-yl-4) méthyl]-8 mercapto-2 1,2,4-triazolo [1,5-c] pyrimidine préparés à l'exemple 41 sont dissous dans 40 ml d'éthanol et une solution d'éthylate de sodium, obtenue par addition de 0,3 g de sodium dans 5 ml d'éthanol, est ajoutée. Le mélange est agité 10 minutes à température ambiante et 1,5 ml de bromoacétate d'éthyle sont ajoutés. Le mélange est alors porté au reflux pendant 2 heures, puis le solvant est évaporé sous vide et le résidu est repris à l'eau et extrait à l'acétate d'éthyle. La phase organique est séchée sur sulfate de magnésium et évaporée sous vide et le résidu obtenu cristallise dans un mélange éther/pentane pour donner 2,9 g de [n-Propyl-7 méthyl-5 [(cyano-2' biphényl-yl-4) méthyl]-8 1,2,4-triazolo [1,5-c] pyrimidine-yl-2] mercapto acétate d'éthyle sous forme de cristaux de point de fusion 103°C.

Exemple 44 : **[n-Propyl-7 méthyl-5 [[(tétrazolyl-5)-2' biphényl-yl-4] méthyl]-8 1,2,4-triazolo [1,5-c] pyrimidine-yl-2] mercapto acétate d'éthyle**

**Formule (I)** : $R_1$ = n-Propyl, $R_2$ = méthyl, X = N,

Y = $C\text{-}SCH_2CO_2Et$,

X $\stackrel{\text{- -}}{\text{—}}$ Y = double liaison, $R_3$ = 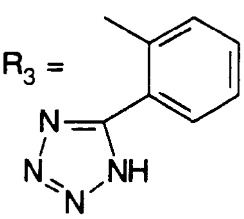

Préparé selon le mode opératoire de l'exemple 19.

Cristaux de point de fusion 127-8°C.

Exemple 45 : **Chlorure de l'acide [n-Propyl-7 méthyl-5 [(cyano-2'-biphényl-yl-4) méthyl]-8 1,2,4-triazolo [1,5-c] pyrimidine-yl-2] sulfonique**

**Formule (XII)** : $R_1$ = n-Propyl, $R_2$ = méthyl, X = N,

Y = $C\text{-}SO_2Cl$,

X $\stackrel{\text{- -}}{\text{—}}$ Y = double liaison, V = 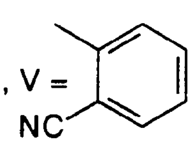

35 g de n-Propyl-7 méthyl-5 mercapto-2 [(cyano-2' biphényl-yl-4) méthyl]-8 1,2,4-triazolo [1,5-c] pyrimidine préparés à l'exemple 41 sont dissous à -5°C dans 300 ml d'acide chlorhydrique concentré. 13 g de chlorate de sodium en solution dans 50 ml d'eau sont ajoutés goutte à goutte en 15 minutes, la température étant maintenue entre -5°C et 0°C. Le mélange est ensuite agité 20 minutes à 0°C puis versé sur un mélange glace/eau ; les cristaux formés sont essorés et lavés à l'eau, puis repris dans 250 ml d'éther et agités cinq minutes puis essorés et séchés à l'air pour donner 30 g de chlorure de l'acide [n-Propyl-7 méthyl-5 [(cyano-2'-biphényl-yl-4) méthyl]-8 1,2,4-triazolo [1,5-c] pyrimidine-yl-2] sulfonique sous forme de cristaux de point de fusion 141°C.

Exemple 46 : **Chlorure de l'acide [n-Butyl-7 méthyl-5 [(cyano-2'-biphényl-yl-4) méthyl]-8 1,2,4-triazolo [1,5-c] pyrimidine-yl-2] sulfonique**

**Formule (XII)** : $R_1$ = n-Butyl, $R_2$ = méthyl, X = N,
Y = C-$SO_2Cl$,

$X \overset{--}{-} Y$ = double liaison, V = 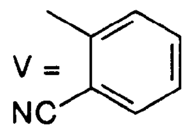

Préparé selon le mode opératoire de l'exemple 45.
Cristaux de point de fusion 112°C.

Exemple 47 : **N,N-diméthyl [n-Propyl-7 méthyl-5 [(cyano-2' biphényl-yl-4) méthyl]-8 1,2,4-triazolo [1,5-c] pyrimidine-yl-2] sulfonamide**

**Formule (XII)** : $R_1$ = n-Propyl, $R_2$ = méthyl, X = N,
Y = C-$SO_2$ $N(CH_3)_2$

$X \overset{--}{-} Y$ = double liaison, V = 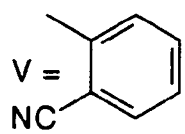

8 g du chlorure de l'acide [n-Propyl-7 méthyl-5 [(cyano-2' biphényl-yl-4) méthyl]-8 1,2,4-triazolo [1,5-c] pyrimidine-yl-2] sulfonique, préparés à l'exemple 45, sont agités avec 40 ml d'une solution aqueuse à 40 % de diméthylamine pendant une heure à 50°C. Le mélange est ensuite extrait au chloroforme et la phase organique est séchée sur sulfate de magnésium puis concentrée sous vide pour donner 5,5 g de N,N-diméthyl [n-Propyl-7 méthyl-5 [(cyano-2' biphényl-yl-4) méthyl]-8 1,2,4-triazolo [1,5-c] pyrimidine-yl-2] sulfonamide sous forme de cristaux de point de fusion 158°C.
Selon ce même mode opératoire les exemples suivants ont été préparés :

Exemple 48 : **N-méthyl [n-Propyl-7 méthyl-5 [(cyano-2' biphényl-yl-4) méthyl]-8 1,2,4-triazolo [1,5-c] pyrimidine-yl-2] sulfonamide**

**Formule (XII)** : $R_1$ = n-Propyl, $R_2$ = méthyl, X = N,
Y = C-$SO_2$ NH $CH_3$

$X \overset{--}{-} Y$ = double liaison, V = 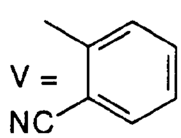

Cristaux de point de fusion 172°C.

Exemple 49 : **N,N-diméthyl [n-Butyl-7 méthyl-5 [(cyano-2' biphényl-yl-4) méthyl]-8 1,2,4-triazolo [1,5-c] pyrimidine-yl-2] sulfonamide**

**Formule (XII)** : $R_1$ = n-Butyl, $R_2$ = méthyl, X = N, Y = C-$SO_2$ $N(CH_3)_2$

X --- Y = double liaison, V = 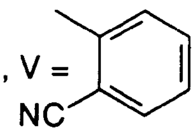

Cristaux de point de fusion 126°C

Exemple 50 : **[n-Propyl-7 méthyl-5 [(cyano-2' biphényl-yl-4) méthyl]-8 1,2,4-triazolo [1,5-c] pyrimidine-yl-2] sulfonamide**

**Formule (XII)** : $R_1$ = n-Propyl, $R_2$ = méthyl, X = N, Y = C-$SO_2$ NH2

X --- Y = double liaison, V = 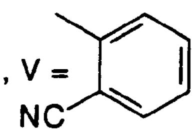

Huile utilisée telle quelle pour la suite.

Exemple 51 : **N-méthyl [n-Butyl-7 méthyl-5 [(cyano-2' biphényl-yl-4) méthyl]-8 1,2,4-triazolo [1,5-c] pyrimidine-yl-2] sulfonamide**

**Formule (XII)** : $R_1$ = n-Butyl, $R_2$ = méthyl, X = N, Y = C-$SO_2$ NH $CH_3$,

X --- Y = double liaison, V = 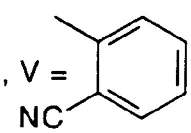

Cristaux de point de fusion 149°C.

Exemple 52 : **N,N-diméthyl [n-Propyl-7 méthyl-5 [[(tétrazolyl-5)-2'-biphényl-yl-4] méthyl]-8 1,2,4-triazolo [1,5-c] pyrimidine-yl-2] sulfonamide**

**Formule (I)** : $R_1$ = n-Propyl, $R_2$ = méthyl, X = N, Y = C-$SO_2$ $N(CH_3)_2$,

X --- Y = double liaison, $R_3$ = 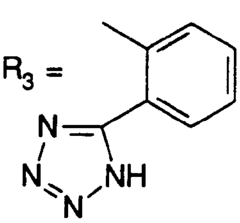

Préparé selon le mode opératoire de l'exemple 19.
Cristaux de point de fusion 176-178°C.

Exemple 53 : **N-méthyl [n-Propyl-7 méthyl-5 [[(tétrazolyl-5)-2'-biphényl-yl-4] méthyl]-8 1,2,4-triazolo [1,5-c] pyrimidine-yl-2] sulfonamide**

**Formule (I)** : $R_1$ = n-Propyl, $R_2$ = méthyl, X = N,
Y = C-$SO_2NH$ $CH_3$,

X ---- Y = double liaison, $R_3$ =

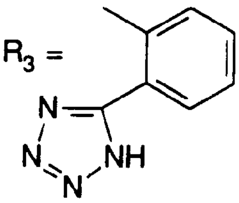

Préparé selon le mode opératoire de l'exemple 19.
Cristaux de point de fusion 163-164°C.

Exemple 54 : **[n-Propyl-7 méthyl-5 [[(tétrazolyl-5)-2'-biphényl-yl-4] méthyl]-8 1,2,4-triazolo [1,5-c] pyrimidine-yl-2] sulfonamide**

**Formule (I)** : $R_1$ = n-Propyl, $R_2$ = méthyl, X = N,
Y = C-$SO_2NH_2$,

X ---- Y = double liaison, $R_3$ =

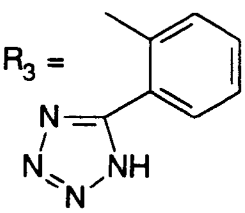

Préparé selon le mode opératoire de l'exemple 19.
Cristaux de point de fusion 200-201°C.

Exemple 55 : **N,N-diméthyl [n-Butyl-7 méthyl-5 [[(tétrazolyl-5)-2'-biphényl-yl-4] méthyl]-8 1,2,4-triazolo [1,5-c] pyrimidine-yl-2] sulfonamide**

**Formule (I)** : $R_1$ = n-Butyl, $R_2$ = méthyl, X = N,
Y = C-$SO_2$ $N(CH_3)_2$,

X ---- Y = double liaison, $R_3$ =

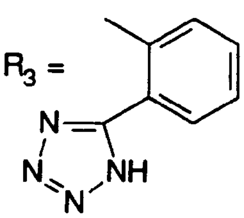

Préparé selon le mode opératoire de l'exemple 19.
Cristaux de point de fusion 147-149°C.

Exemple 56 : **N-méthyl [n-Butyl-7 méthyl-5 [[(tétrazolyl-5)-2'-biphényl-yl-4] méthyl]-8 1,2,4-triazolo [1,5-c] pyrimidine-yl-2] sulfonamide**

**Formule (I)** : $R_1$ = n-Butyl, $R_2$ = méthyl, X = N, Y = C-$SO_2$ NH $CH_3$,

X $\overset{---}{—}$ Y = double liaison, $R_3$ = 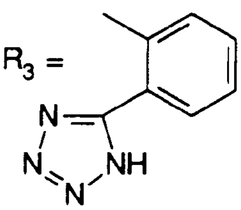

Préparé selon le mode opératoire de l'exemple 19.
Cristaux de point de fusion 179-180°C.

Exemple 57 : **n-Propyl-7 méthyl-5 méthylmercapto-2 [(cyano-2'-biphényl-yl-4) méthyl]-8 1,2,4-triazolo [1,5-c] pyrimidine**

**Formule (XII)** : $R_1$ = n-Propyl, $R_2$ = méthyl, X = N, Y = C-$SCH_3$

X $\overset{---}{—}$ Y = double liaison, V = 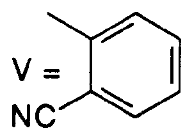

Préparé selon le mode opératoire de l'exemple 38.
Cristaux de point de fusion 107°C.

Exemple 58 : **n-Butyl-7 méthyl-5 méthylmercapto-2 [(cyano-2'-biphényl-yl-4) méthyl]-8 1,2,4-triazolo [1,5-c] pyrimidine**

**Formule (XII)** : $R_1$ = n-Butyl, $R_2$ = méthyl, X = N, Y = C-$SCH_3$

X $\overset{---}{—}$ Y = double liaison, V = 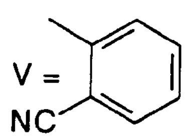

Préparé selon le mode opératoire de l'exemple 38.
Huile utilisée telle quelle pour la suite.

Exemple 59 : **n-Propyl-7 méthyl-5 méthylmercapto-2 [[(tétrazolyl-5)-2'-biphényl-yl-4] méthyl]-8 1,2,4-triazolo [1,5-c] pyrimidine**

**Formule (I)** : $R_1$ = n-Propyl, $R_2$ = méthyl, X = N, Y = C-$SCH_3$,

X --- Y = double liaison, $R_3$ = 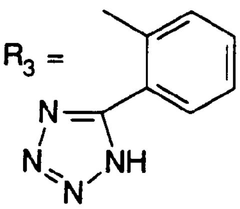

Préparé selon le mode opératoire de l'exemple 19.
Cristaux de point de fusion 169-170°C.

Exemple 60 : **n-Butyl-7 méthyl-5 méthylmercapto-2 [[(tétrazolyl-5)-2'-biphényl-yl-4] méthyl]-8 1,2,4-triazolo [1,5-c] pyrimidine**

**Formule (I)** : $R_1$ = n-Butyl, $R_2$ = méthyl, X = N, Y = C-$SCH_3$,

X --- Y = double liaison, $R_3$ = 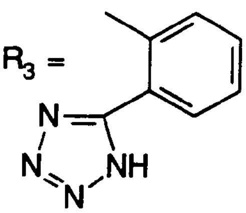

Préparé selon le mode opératoire de l'exemple 19.
Cristaux de point de fusion 194-195°C.

Exemple 61 : **n-Butyl-7 méthyl-5 méthoxy-2 [(cyano-2' biphényl-yl-4) méthyl]-8 1,2,4-triazolo [1,5-c] pyrimidine**

**Formule (XII)** : $R_1$ = n-Butyl, $R_2$ = méthyl, X = N, Y = C-$OCH_3$,

X --- Y = double liaison, V = 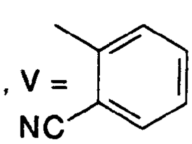

Préparé selon le mode opératoire de l'exemple 27.
Cristaux de point de fusion 88°C.

Exemple 62 : **n-Butyl-7 méthyl-5 méthoxy-2 [[(tétrazolyl-5)-2'-biphényl-yl-4] méthyl]-8 1,2,4-triazolo [1,5-c] pyrimidine**

**Formule (I)** : $R_1$ = n-Butyl, $R_2$ = méthyl, X = N, Y = C-$OCH_3$,

X --- Y = double liaison, $R_3$ =

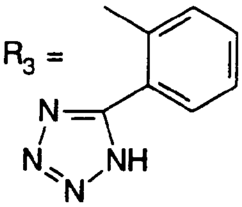

Préparé selon le mode opératoire de l'exemple 19.
Cristaux de point de fusion 195-196°C.

Exemple 63 : **[n-Butyl-7 méthyl-5 oxo-3 [(cyano-2' biphényl-yl-4) méthyl]-8 1,2,4-triazolo [4,3-c] pyrimidine (2H)-yl-2] acétate d'éthyle**

**Formule (XII)** : $R_1$ = n-Butyl, $R_2$ = méthyl, X = CO,

Y = N-$CH_2$-$CO_2$Et,

X --- Y = liaison simple, V =

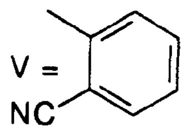

Préparé selon le mode opératoire de l'exemple 21.
Huile utilisée telle quelle pour la suite.

Exemple 64 : **[n-Butyl-7 méthyl-5 oxo-3 [[(tétrazolyl-5)-2' biphényl-yl-4] méthyl]-8 1,2,4-triazolo [4,3-c] pyrimidine (2H)-yl-2] acétate d'éthyle**

**Formule (I)** : $R_1$ = n-Butyl, $R_2$ = méthyl, X = CO, Y = N-$CH_2$-$CO_2$Et,

X --- Y = liaison simple, $R_3$ =

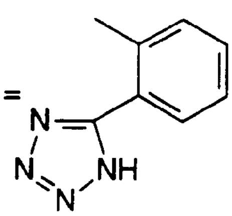

Préparé selon le mode opératoire de l'exemple 19.
Cristaux de point de fusion 174-175°C.

Exemple 65 : **n-Butyl-7 diméthyl-2,5 [(cyano-2' biphényl-yl-4) méthyl]-8 1,2,4-triazolo [4,3-c] pyrimidine one-3 (2H)**

**Formule (XII)** : $R_1$ = n-Butyl, $R_2$ = méthyl, X = CO, Y = N-$CH_3$,

X --- Y = liaison simple, V = 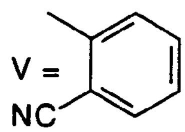

Préparé selon le mode opératoire de l'exemple 24.
Huile utilisée telle quelle pour la suite.

Exemple 66 : **n-Butyl-7 diméthyl-2,5 [[(tétrazolyl-5)-2' biphényl-yl-4) méthyl]-8 1,2,4-triazolo [4,3-c] pyrimidine one-3 (2H)**

**Formule (I)** : $R_1$ = n-Butyl, $R_2$ = méthyl, X = CO, Y = N-$CH_3$,

X --- Y = liaison simple, $R_3$ = 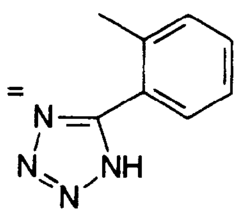

Préparé selon le mode opératoire de l'exemple 19.
Cristaux de point de fusion 192-193°C.

Exemple 67 : **n-Propyl-6 méthyl-2 hydrazino-4 [[(tétrazolyl-5)-2'-biphényl-yl-4] méthyl]-5 pyrimidine**

**Formule (IX)** : $R_1$ = n-Propyl, $R_2$ = méthyl

V = 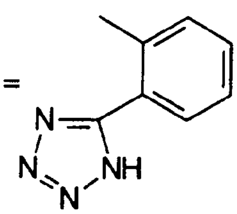

Préparé selon le mode opératoire de l'exemple 19, à partir de la n-Propyl-6 méthyl-2 hydrazino-4 [cyano-2'-biphényl-yl-4] méthyl-5 pyrimidine, préparée à l'exemple 12.
Cristaux de point de fusion 183-185°C.

Exemple 68 : **n-Propyl-7 méthyl-5 mercapto-3 [[(tétrazolyl-5)-2'-biphényl-yl-4] méthyl]-8 1,2,4-triazolo [4,3-c] pyrimidine**

**Formule (I) :** $R_1$ = n-Propyl, $R_2$ = méthyl, X = C-SH, Y = N,

X --- Y = double liaison, $R_3$ = 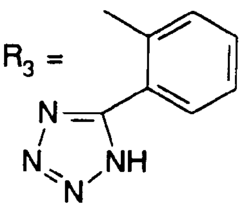

8 g de n-Propyl-6 méthyl-2 hydrazino-4 [[(tétrazolyl-5)-2'-biphényl-yl-4] méthyl]-5 pyrimidine, préparés à l'exemple 67, sont dissous dans un mélange composé de 75 ml de méthanol, 7 ml d'eau et 2,4 g de soude. 2,5 ml de sulfure de carbone sont additionnés goutte à goutte, puis le mélange est porté au reflux pendant une heure et est ensuite évaporé à sec sous vide. Le résidu est repris par 100 ml d'éthanol et le mélange est chauffé une heure au reflux, concentré sous vide puis le résidu est repris à l'eau. Par addition d'acide acétique, le pH est amené à 5 et les cristaux formés sont essorés puis chromatographiés sur gel de silice, avec l'acétate d'éthyle comme éluant, pour donner 1,4 g de n-Propyl-7 méthyl-5 mercapto-3 [[(tétrazolyl-5)-2'-biphényl-yl-4] méthyl]-8 1,2,4-triazolo [4,3-c] pyrimidine sous forme de cristaux de point de fusion 247-248°C.

Exemple 69 : **n-Propyl-7 méthyl-5 [(cyano-2'-biphényl-yl-4) méthyl]-8 1,2,4-triazolo [4,3-c] pyrimidine**

**Formule (XII) :** $R_1$ = n-Propyl, $R_2$ = méthyl, X = CH, Y = N,

X --- Y = double liaison, V = 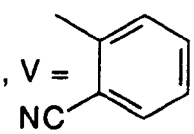

20 g de n-Propyl-6 méthyl-2 hydrazino-4 [(cyano-2'-biphényl-yl-4) méthyl]-5 pyrimidine, préparée à l'exemple 12, sont chauffés durant 6 heures au reflux dans 200 ml de triéthylorthoformate. Le mélange est ensuite concentré sous vide et le résidu repris à l'éther ; les cristaux obtenus sont essorés et lavés à l'éther pour donner 18,8 g de n-Propyl-7 méthyl-5 [(cyano-2'-biphényl-yl-4) méthyl]-8 1,2,4-triazolo [4,3-c] pyrimidine sous forme de cristaux de point de fusion 153°C.

Exemple 70 : **n-Propyl-7 méthyl-5 [(cyano-2'-biphényl-yl-4) méthyl]-8 1,2,4-triazolo [1,5-c] pyrimidine**

**Formule (XII) :** $R_1$ = n-Propyl, $R_2$ = méthyl, X = N, Y = CH,

X --- Y = double liaison, V = 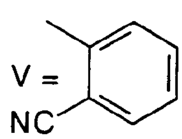

10 g de n-Propyl-7 méthyl-5 [(cyano-2'-biphényl-yl-4) méthyl]-8 1,2,4-triazolo [4,3-c] pyrimidine préparé à l'exemple 69, sont chauffés 4 heures au reflux dans 150 ml d'acide formique. Le mélange est évaporé à sec sous vide et le résidu repris à l'éther et au pentane cristallise pour donner 7,5 g de n-Propyl-7 méthyl-5 [(cyano-2'-biphényl-yl-4) méthyl]-8 1,2,4-triazolo [1,5-c] pyrimidine sous forme de cristaux de point de fusion 112°C.

Exemple 71 : **n-Propyl-7 méthyl-5 [[(tétrazolyl-5)-2'-biphényl-yl-4) méthyl]-8 1,2,4-triazolo [1,5-c] pyrimidine**

**Formule (I)** : $R_1$ = n-Propyl, $R_2$ = méthyl, X = N, Y = CH,

X --- Y = double liaison, $R_3$ = 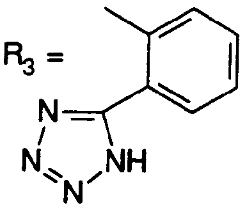

Préparé selon le mode opératoire de l'exemple 19.
Cristaux de point de fusion 183-184°C.

Exemple 72 : **n-Propyl-7 méthyl-5 [[(tétrazolyl-5)-2' biphényl-yl-4] méthyl]-8 1,2,4-triazolo [4,3-c] pyrimidine**

**Formule (I)** : $R_1$ = n-Propyl, $R_2$ = méthyl, X = CH, Y = N,

X --- Y = double liaison, $R_3$ = 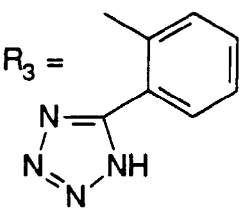

4,8 g de n-Propyl-6 méthyl-2 hydrazino-4 [[(tétrazolyl-5)-2' biphényl-yl-4] méthyl]-5 pyrimidine, préparés à l'exemple 67 sont chauffés 4 heures au reflux dans 40 ml de triéthyl orthoformate. Le mélange est évaporé sous vide et le résidu est cristallisé dans un mélange acétate d'éthyle / éther isopropylique pour donner 1 g de n-Propyl-7 méthyl-5 [[(tétrazolyl-5)-2' biphényl-yl-4] méthyl]-8 1,2,4-triazolo [4,3-c] pyrimidine, sous forme de cristaux de point de fusion 182-184°C.

Exemple 73 : **n-Propyl-7 diméthyl-2,5 [(cyano-2' biphényl-yl-4) méthyl]-8 1,2,4-triazolo [1,5-c] pyrimidine**

**Formule (XII)** : $R_1$ = n-Propyl, $R_2$ = méthyl, X = N, Y = C-$CH_3$,

X --- Y = double liaison, V = 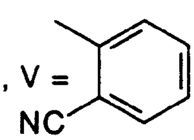

6 g de n-Propyl-6 méthyl-2 hydrazino-4 [(cyano-2' biphényl-yl-4) méthyl]-5 pyrimidine, préparé à l'exemple 12, sont chauffés 5 heures à 90°C dans 100 ml de triéthyl orthoacétate. Le mélange est ensuite évaporé sous vide et le résidu est repris dans 75 ml d'acide formique. La solution obtenue est chauffée 5 heures au reflux, puis l'acide formique est évaporé sous vide et le résidu cristallisé dans un mélange éther/pentane pour donner 5 g de n-Propyl-7 diméthyl-2,5 [(cyano-2' biphényl-yl-4) méthyl]-8 1,2,4-triazolo [1,5-c] pyrimidine sous forme de cristaux de point de fusion 132°C.

Exemple 74 : **n-Propyl-7 diméthyl-2,5 [[(tétrazolyl-5)-2' biphényl-yl-4] méthyl]-8 1,2,4-triazolo [1,5-c] pyrimidine**

**Formule (I)** : $R_1$ = n-Propyl, $R_2$ = méthyl, X = N, Y = C-$CH_3$,

X --- Y = double liaison, $R_3$ =

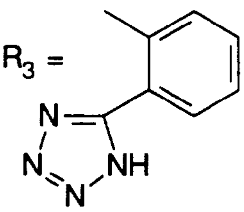

Préparé selon le mode opératoire de l'exemple 19.
Cristaux de point de fusion 188-190°C.

Exemple 75 : **n-Propyl-7 méthyl-5 trifluorométhyl-2 [(cyano-2' biphényl-yl-4) méthyl]-8 1,2,4-triazolo [1,5-c] pyrimidine**

**Formule (XII)** : $R_1$ = n-Propyl, $R_2$ = méthyl, X = N, Y = C-$CF_3$,

X --- Y = double liaison, V =

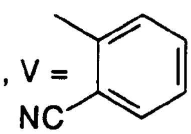

10 g de n-Propyl-6 méthyl-2 hydrazino-4 [(cyano-2' biphényl-yl-4) méthyl]-5 pyrimidine, préparé à l'exemple 12, sont dissous dans 100 ml de tétrahydrofurane anhydre et 5 ml d'anhydride trifluoroacétique sont ajoutés goutte à goutte. Le mélange est chauffé 2 heures au reflux et le solvant est évaporé sous vide. Le résidu obtenu est repris dans 40 ml d'oxychlorure de phosphore et la solution obtenue est chauffée 4 heures au reflux. L'oxychlorure de phosphore est évaporé sous vide et le résidu est alors repris dans 40 ml d'acide formique et chauffé au reflux pendant 3 heures. Après évaporation sous vide de l'acide formique, le résidu huileux est chromatographié sur gel de silice dans l'éther isopropylique pour donner 4,8 g de n-Propyl-7 méthyl-5 trifluorométhyl-2 [(cyano-2' biphényl-yl-4) méthyl]-8 1,2,4-triazolo [1,5-c] pyrimidine sous forme d'une huile utilisée telle quelle pour la suite.

Exemple 76 : **n-Propyl-7 méthyl-5 trifluorométhyl-2 [[(tétrazolyl-5)-2' biphényl-yl-4] méthyl]-5 1,2,4-triazolo [1,5-c] pyrimidine**

**Formule (I)** : $R_1$ = n-Propyl, $R_2$ = méthyl, X = N, Y = C-$CF_3$,

X --- Y = double liaison, $R_3$ =

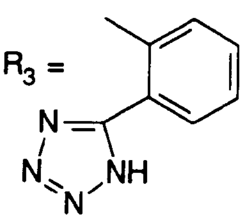

Préparé selon le mode opératoire de l'exemple 19.
Cristaux de point de fusion 161-162°C.

Exemple 77 : **n-Propyl-7 méthyl-5 [(cyano-2' biphényl-yl-4) méthyl]-8 éthyl-2 1,2,4-triazolo [1,5-c] pyrimidine**

**Formule (XII)** : $R_1$ = n-Propyl, $R_2$ = méthyl, X = N, Y = C-$C_2H_5$,

X --- Y = double liaison, V = 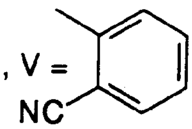

Préparé selon le mode opératoire de l'exemple 73 à partir du triéthyl orthopropionate.
Cristaux de point de fusion 96°C.

Exemple 78 : **n-Propyl-7 méthyl-5 [[(tétrazolyl-5)-2' biphényl-yl-4] méthyl]-8 éthyl-2 1,2,4-triazolo [1,5-c] pyrimidine**

**Formule (I)** : $R_1$ = n-Propyl, $R_2$ = méthyl, X = N, Y = C-$C_2H_5$,

X --- Y = double liaison, $R_3$ = 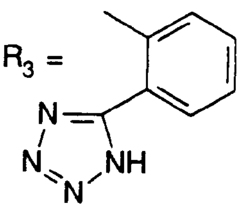

Préparé selon le mode opératoire de l'exemple 19.
Cristaux de point de fusion 190-191°C.

Exemple 79: **n-Propyl-7 méthyl-5 méthylamino-2 [(cyano-2' biphényl-yl-4) méthyl]-8 1,2,4-triazolo [1,5-c] pyrimidine**

**Formule (XII)** : $R_1$ = n-Propyl, $R_2$ = méthyl, X = N, Y = C-NH-$CH_3$,

X --- Y = double liaison, V = 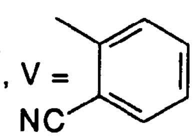

5 g de iodhydrate de n-Propyl-6 méthyl-2 [(cyano-2' biphényl-yl-4) méthyl]-5 (méthyl-4' S-méthyl isothiosemicarbazido)-4 pyrimidine sont chauffés 4 heures au reflux dans 50 ml d'éthoxy-2 éthanol en présence de 1.5 g de carbonate de potassium. Le solvant est ensuite évaporé sous vide et le résidu est repris à l'eau, les cristaux formés sont essorés, lavés à l'eau puis à l'éther pour donner 3.3 g de n-Propyl-7 méthyl-5 méthylamino-2 [(cyano-2' biphényl-yl-4) méthyl]-8 1,2,4-triazolo [1,5-c] pyrimidine sous forme de cristaux de point de fusion 159°C.

Préparation du iodhydrate de n-Propyl-6 méthyl-2 [(cyano-2' biphényl-yl-4) méthyl]-5 (méthyl-4' S-méthyl isothiosemicarbazido)-4 pyrimidine :

10 g de n-Propyl-6 méthyl-2 [(cyano-2' biphényl-yl-4) méthyl]-5 hydrazino-4 pyrimidine, préparé à l'exemple 12, sont dissous dans 100 ml de toluène. 2.1 g d'isothiocyanate de méthyle sont ajoutés et le mélange est chauffé 2 heures au reflux, puis laissé une nuit à température ambiante. 2 ml d'iodure de méthyle sont ajouté et le mélange est porté 2 heures au reflux. Après refroidissement, les cristaux formés sont essorés et lavés à l'éther pour donner 14 g de iodhydrate de n-Propyl-6 méthyl-2 [(cyano-2' biphényl-yl-4) méthyl]-5 (méthyl-4' S-méthyl isothiosemicarbazido)-4 pyrimidine sous forme de cristaux de point de fusion 220°C (décomposition).

Exemple 80 : **n-Propyl-7 méthyl-5 méthylamino-2 [[(tétrazolyl-5)-2' biphényl-yl-4] méthyl]-8 1,2,4-triazolo [1,5-c] pyrimidine**

**Formule (I)** : $R_1$ = n-Propyl, $R_2$ = méthyl, X = N, Y = C-NH-$CH_3$,

X --- Y = double liaison, $R_3$ =

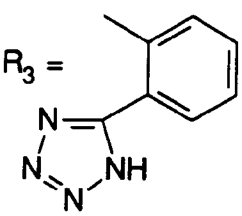

Préparé selon le mode opératoire de l'exemple 19.
Cristaux de point de fusion 229-230°C.

Exemple 81 : **oxo-3 pentanoate d'éthyle**

**Formule (II) :** $R_1$ = éthyl, $R_6$ = éthyl
Préparé selon le mode opératoire de l'exemple 1.
Huile de point d'ébullition $Eb_{15}$ = 86-90°C.

Exemple 82 : **[(cyano-2' biphényl-yl-4) méthyl]-2 oxo-3 pentanoate d'éthyle**

**Formule (IV)** : $R_1$ = éthyl, $R_6$ = éthyl

V =

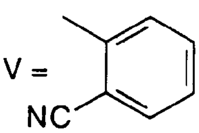

Préparé selon le mode opératoire de l'exemple 3.
Huile utilisée telle quelle pour la suite.

Exemple 83 : **Ethyl-6 méthyl-2 [(cyano-2' biphényl-yl-4) méthyl]-5 hydroxy-4 pyrimidine**

**Formule (VII)** : $R_1$ = éthyl, $R_2$ = méthyl

V = 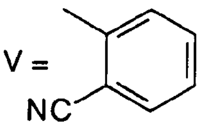

Préparé selon le mode opératoire de l'exemple 6.
Cristaux de point de fusion 188°C.

Exemple 84 : **Ethyl-6 méthyl-2 [(cyano-2' biphényl-yl-4) méthyl]-5 chloro-4 pyrimidine**

**Formule (VIII)** : $R_1$ = éthyl, $R_2$ = méthyl

V = 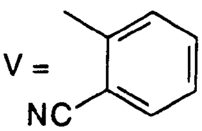

Préparé selon le mode opératoire de l'exemple 9.
Cristaux de point de fusion 80°C.

Exemple 85 : **Ethyl-6 méthyl-2 hydrazino-4 [(cyano-2' biphényl-yl-4) méthyl]-5 pyrimidine**

**Formule (IX)** : $R_1$ = éthyl, $R_2$ = méthyl

V = 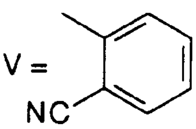

Préparé selon le mode opératoire de l'exemple 12.
Cristaux de point de fusion 190°C.

Exemple 86 : **Ethyl-7 méthyl-5 [(cyano-2' biphényl-yl-4) méthyl]-8 1,2,4-triazolo [4,3-c] pyrimidine**

**Formule (XII)** : $R_1$ = éthyl, $R_2$ = méthyl, X = CH, Y = N,

X $\overset{--}{—}$ Y = double liaison, V = 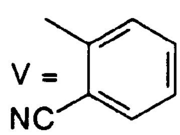

Préparé selon le mode opératoire de l'exemple 69.
Cristaux de point de fusion 166°C.

Exemple 87 : **Ethyl-7 méthyl-5 [(cyano-2' biphényl-yl-4) méthyl]-8 1,2,4-triazolo [1,5-c] pyrimidine**

**Formule (XII)** : $R_1$ = éthyl, $R_2$ = méthyl, X = N, Y = CH,

X --- Y = double liaison, V = 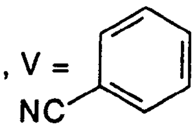

Préparé selon le mode opératoire de l'exemple 70.
Cristaux de point de fusion 117°C.

Exemple 88 : **Ethyl-7 méthyl-5 [[(tétrazolyl-5)-2' biphényl-yl-4] méthyl]-8 1,2,4-triazolo [1,5-c] pyrimidine**

**Formule (I)** : $R_1$ = éthyl, $R_2$ = méthyl, X = N, Y = CH,

X --- Y = double liaison, $R_3$ = 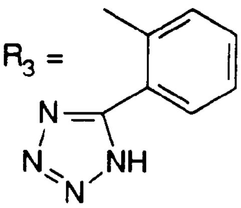

Préparé selon le mode opératoire de l'exemple 19.
Cristaux de point de fusion 146-148°C.

Exemple 89 : **Ethyl-7 diméthyl-2,5 [(cyano-2' biphényl-yl-4) méthyl]-8 1,2,4-triazolo [1,5-c] pyrimidine**

**Formule (XII)** : $R_1$ = éthyl, $R_2$ = méthyl, X = N,
Y = C-$CH_3$,

X --- Y = double liaison, V = 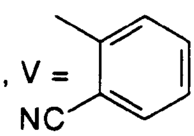

Préparé selon le mode opératoire de l'exemple 73.
Cristaux de point de fusion 126°C.

Exemple 90 : **Ethyl-7 diméthyl-2,5 [[(tétrazolyl-5)-2' biphényl-yl-4] méthyl]-8 1,2,4-triazolo [1,5-c] pyrimidine**

**Formule (I)** : $R_1$ = éthyl, $R_2$ = méthyl, X = N, Y = C-$CH_3$,

X --- Y = double liaison, $R_3$ = 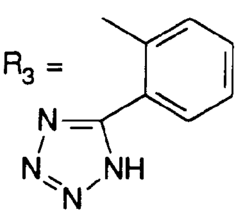

Préparé selon le mode opératoire de l'exemple 19.
Cristaux de point de fusion 230-231°C.

Exemple 91 : **Diéthyl-2,7 méthyl-5 [(cyano-2' biphényl-yl-4) méthyl]-8 1,2,4-triazolo [1,5-c] pyrimidine**

**Formule (XII)** : $R_1$ = éthyl, $R_2$ = méthyl, X = N, Y = C-$C_2H_5$,

X --- Y = double liaison, V = 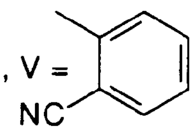

Préparé selon le mode opératoire de l'exemple 73 à partir du triéthylorthopropionate. Cristaux de point de fusion 96°C.

Exemple 92 : **Diéthyl-2,7 méthyl-5 [[(tétrazolyl-5)-2' biphényl-yl-4] méthyl]-8 1,2,4-triazolo [1,5-c] pyrimidine**

**Formule (I)** : $R_1$ = éthyl, $R_2$ = méthyl, X = N, Y = C-$C_2H_5$,

X --- Y = double liaison, $R_3$ = 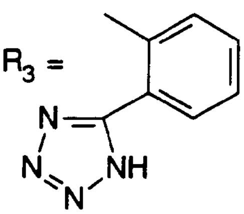

Préparé selon le mode opératoire de l'exemple 19. Cristaux de point de fusion 220-222°C.

Exemple 93 : **n-Propyl-7-méthyl-5 phényl-2 [(cyano-2' biphényl-yl-4) méthyl]-8 1,2,4-triazolo [1,5-c] pyrimidine**

**Formule (XII)** : $R_1$ = n-Propyl, $R_2$ = méthyl, X = N, Y = C-phényl,

X --- Y = double liaison, V = 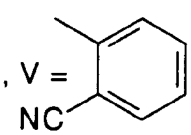

Préparé selon le mode opératoire de l'exemple 73 à partir du triéthyl orthobenzoate. Huile utilisée telle quelle pour la suite.

Exemple 94 : **n-Propyl-7 méthyl-5 phényl-2 [[(tétrazolyl-5)-2' biphényl-yl-4] méthyl]-8 1,2,4-triazolo [1,5-c] pyrimidine**

**Formule (I)** : $R_1$ = n-Propyl, $R_2$ = méthyl, X = N, Y = C-phényl,

X --- Y = double liaison, $R_3$ = 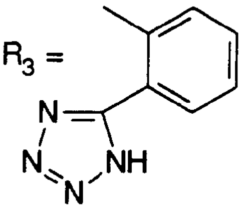

Préparé selon le mode opératoire de l'exemple 19.
Cristaux de point de fusion 196°C.

Exemple 95 : **Ethyl-7 méthyl-5 [(cyano-2' biphényl-yl-4) méthyl]-8 1,2,4-triazolo [4,3-c] pyrimidine one-3 (2H)**

**Formule (XII)** : $R_1$ = éthyl, $R_2$ = méthyl, X = CO, Y = NH,

X --- Y = liaison simple, V = 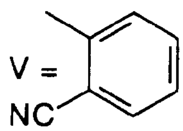

Préparé selon le mode opératoire de l'exemple 15.
Cristaux de point de fusion 174°C.

Exemple 96 : **Ethyl-7 méthyl-5 [(cyano-2' biphényl-yl-4) méthyl]-8 1,2,4-triazolo [1,5-c] pyrimidine-one-2 (3H)**

**Formule (XII)** : $R_1$ = éthyl, $R_2$ = méthyl, X = NH, Y = CO,

X --- Y = liaison simple, V = 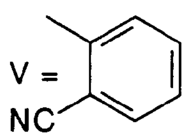

Préparé selon le mode opératoire de l'exemple 18.
Cristaux de point de fusion 246°C.

<u>Exemple 97</u> : **Ethyl-7 méthyl-5 [[(tétrazolyl-5)-2' biphényl-yl-4] méthyl]-8 1,2,4-triazolo [1,5-c] pyrimidine-one-2 (3H)**

**Formule (I)** : $R_1$ = éthyl, $R_2$ = méthyl, X = NH, Y = CO,

X --- Y = liaison simple, $R_3$ = 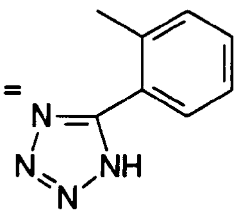

Préparé selon le mode opératoire de l'exemple 19.
Cristaux de point de fusion 254°C.

<u>Exemple 98</u> : **n-Butyl-7 méthyl-5 trifluorométhyl-2 [(cyano-2' biphényl-yl-4) méthyl]-8 1,2,4-triazolo [1,5-c] pyrimidine**

**Formule (XII)** : $R_1$ = n-Butyl, $R_2$ = méthyl, X = N, Y = C-$CF_3$,

X --- Y = double liaison, V = 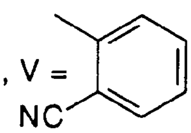

Préparé selon le mode opératoire de l'exemple 75.
Cristaux de point de fusion 110°C.

<u>Exemple 99</u> : **n-Butyl-7 méthyl-5 trifluorométhyl-2 [[(tétrazolyl-5)-2' biphényl-yl-4]méthyl]-8 1,2,4-triazolo [1,5-c] pyrimidine**

**Formule (I)** : $R_1$ = n-Butyl, $R_2$ = méthyl, X = N, Y = C-$CF_3$,

X --- Y = double liaison, $R_3$ = 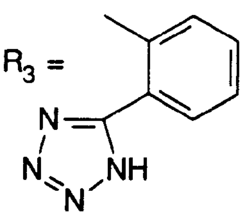

Préparé selon le mode opératoire de l'exemple 19.
Cristaux de point de fusion 179-180°C.

Exemple 100 : **n-Propyl-6 mercapto-2 hydroxy-4 [(cyano-2' biphényl-yl-4) méthyl]-5 pyrimidine**

**Formule (VII)** : $R_1$ = n-Propyl, $R_2$ = SH,

V = 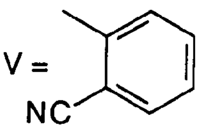

5.7 g de sodium sont dissous dans 150 ml de méthanol et 19 g de thiourée sont ajoutés à cette solution. Le mélange est agité 5 minutes et 58 g d'oxo-3 [(cyano-2' biphényl-yl-4) méthyl]-2 hexanoate d'éthyle, préparé à l'exemple 4, sont ajoutés. Le mélange est ensuite chauffé 10 heures au reflux et le méthanol est évaporé sous vide. Le résidu est repris à l'eau et lavé à l'éther, la phase aqueuse est neutralisée par addition d'acide chlorhydrique dilué et les cristaux obtenus sont filtrés, lavés à l'eau et à l'éther pour donner 26 g de n-Propyl-6 mercapto-2 hydroxy-4 [(cyano-2' biphényl-yl-4) méthyl]-5 pyrimidine sous forme de cristaux de point de fusion 191°C.

Exemple 101 : **n-Propyl-6 méthylthio-2 hydroxy-4 [(cyano-2' biphényl-yl-4) méthyl]-5 pyrimidine**

**Formule (VII)** : $R_1$ = n-Propyl, $R_2$ = $SCH_3$,

V = 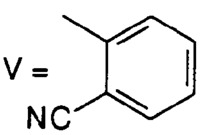

26 g de n-Propyl-6 mercapto-2 hydroxy-4 [(cyano-2' biphényl-yl-4) méthyl]-5 pyrimidine, préparé à l'exemple 100, sont agités 15 minutes dans une solution de 5 g de potasse dans 200 ml de méthanol. 6 ml d'iodure de méthyle sont ajoutés au mélange qui est ensuite agité 4 heures à température ambiante. Les cristaux formés sont essorés, lavés à l'eau puis à l'éther et séchés pour donner 23 g de n-Propyl-6 méthylthio-2 hydroxy-4 [(cyano-2' biphényl-yl-4) méthyl]-5 pyrimidine sous forme de cristaux de point de fusion 218°C.

Exemple 102 : **n-Propyl-6 méthylthio-2 chloro-4 [(cyano-2' biphényl-yl-4) méthyl]-5 pyrimidine**

**Formule (VIII)** : $R_1$ = n-Propyl, $R_2$ = $SCH_3$,

V = 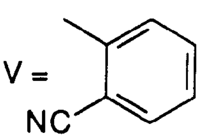

Préparé selon le mode opératoire de l'exemple 9.
Cristaux de point de fusion 88°C.

Exemple 103 : **n-Propyl-6 méthylthio-2 hydrazino-4 [(cyano-2' biphényl-yl-4) méthyl]-5 pyrimidine**

**Formule (IX)** : $R_1$ = n-Propyl, $R_2$ = $SCH_3$,

V = 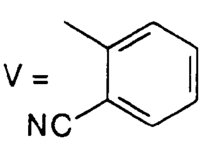

Préparé selon le mode opératoire de l'exemple 12.

Cristaux de point de fusion 106°C.

Exemple 104 : **n-Propyl-6 méthylthio-2 hydrazino-4 [[(tétrazolyl-5)-2' biphényl-yl-4] méthyl]-5 pyrimidine**

**Formule (IX)** : $R_1$ = n-Propyl, $R_2$ = $SCH_3$,

V = 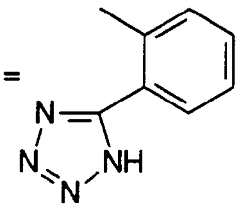

Préparé selon le mode opératoire de l'exemple 19.
Cristaux de point de fusion 224°C.

Exemple 105 : **n-Propyl-7 méthylthio-5 [[(tétrazolyl-5)-2' biphényl-yl-4] méthyl]-8 1,2,4-triazolo [4,3-c] pyrimidine-one-3 (2H)**

**Formule (I)** : $R_1$ = n-Propyl, $R_2$ = $SCH_3$, X = CO,
Y = NH,

X $\overline{--}$ Y = liaison simple, $R_3$ = 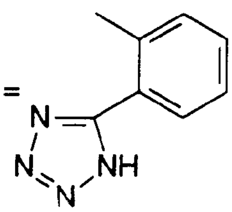

Préparé selon le mode opératoire de l'exemple 15, chromatographié sur gel de silice ($CHCl_3$ 9 / MeOH 1).
Cristaux de point de fusion 259-261°C.

Exemple 106 : **n-Propyl-7 méthyl-5 amino-2 [(cyano-2' biphényl-yl-4) méthyl]-8 1,2,4-triazolo [1,5-c] pyrimidine**

**Formule (XII)** : $R_1$ = n-Propyl, $R_2$ = méthyl, X = N,
Y = C-$NH_2$,

X $\overline{--}$ Y = double liaison, V = 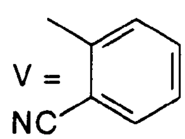

10 g de n-Propyl-6 méthyl-2 hydrazino-4 [(cyano-2' biphényl-yl-4) méthyl]-5 pyrimidine, préparé à l'exemple 12, et 5 g de sulfate de méthyl-2 thio-2 pseudourée sont chauffés au reflux pendant 16 heures. Après addition d'eau, les cristaux formés sont essorés et lavés à l'éther puis à l'acétate d'éthyle avant d'être repris dans une solution de soude diluée et extraits au chloroforme. La phase organique est séchée sur sulfate de magnésium et évaporée sous vide pour donner un résidu qui cristallise dans un mélange d'éther isopropylique et d'acétate d'éthyle pour donner 1,8 g de n-Propyl-7 méthyl-5 amino-2 [(cyano-2' biphényl-yl-4) méthyl]-8 1,2,4-triazolo [1,5-c] pyrimidine sous forme de cristaux de point de fusion 150°C.

Exemple 107 : **n-Propyl-7 méthyl-5 amino-2 [[(tétrazolyl-5)-2' biphényl-yl-4] méthyl]-8 1,2,4-triazolo [1,5-c] pyrimidine**

**Formule (I)** : $R_1$ = n-Propyl, $R_2$ = méthyl, X = N,
Y = C-$NH_2$,

X --- Y = double liaison, $R_3$ = 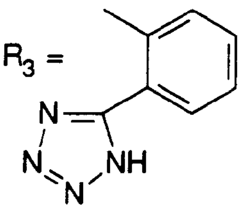

Préparé selon le mode opératoire de l'exemple 19.
Cristaux de point de fusion 170-174°C.

Exemple 108 : **[n-Propyl-7 méthyl-5 [(cyano-2' biphényl-yl-4) méthyl]-8 1,2,4-triazolo [1,5-c] pyrimidine-yl-2] carboxylate d'éthyle**

**Formule (XII)** : $R_1$ = n-Propyl, $R_2$ = méthyl, X = N,
Y = C-$CO_2$Et,

X --- Y = double liaison, V = 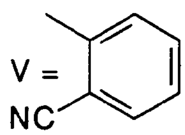

34.6 g de n-Propyl-6 méthyl-2 hydrazino-4 [(cyano-2' biphényl-yl-4) méthyl]-5 pyrimidine, préparé à l'exemple 12, sont dissous dans 500 ml de chloroforme stabilisé à l'amylène en présence de 13.9 g de triéthylamine. 13.2 ml de chlorure d'éthoxalyle sont ajoutés goutte à goutte et le mélange est agité 1 heure à température ambiante, puis 2 heures au reflux. Après lavage à l'eau, la phase chloroformique est séchée et évaporée sous vide et le résidu qui cristallise dans un mélange acétone/éther donne 25 g d'hydrazide de point de fusion 176°C. Cet hydrazide est ensuite chauffé 6 heures au reflux dans 60 ml d'oxychlorure de phosphore. Le mélange est concentré sous vide puis le résidu est repris au chloroforme et la solution obtenue est lavée à l'eau et avec une solution de bicarbonate de sodium, avant d'être séchée sur sulfate de magnésium et évaporée sous vide. Le résidu obtenu cristallise dans un mélange éther/éther isopropylique pour donner 15.7 g de [n-Propyl-7 méthyl-5 [(cyano-2' biphényl-yl-4) méthyl]-8 1,2,4-triazolo [1,5-c] pyrimidine-yl-2] carboxylate d'éthyle sous forme de cristaux de point de fusion 108°C.

Exemple 109 : **[n-Propyl-7 méthyl-5 [[(tétrazolyl-5)-2' biphényl-yl-4] méthyl]-8 1,2,4-triazolo [1,5-c] pyrimidine-yl-2] carboxylate d'éthyle**

**Formule (I)** : $R_1$ = n-Propyl, $R_2$ = méthyl, X = N,
Y = C-$CO_2$Et,

X --- Y = double liaison, $R_3$ = 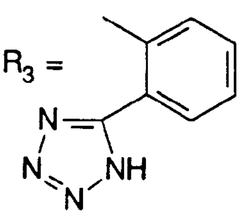

Préparé selon le mode opératoire de l'exemple 19.
Cristaux de point de fusion 168-170°C.

Exemple 110 : **Acide [n-Propyl-7 méthyl-5 [[(tétrazolyl-5)-2' biphényl-yl-4] méthyl]-8 1,2,4-triazolo [1,5-c] pyrimidine-yl-2] carboxylique**

**Formule (I)** : $R_1$ = n-Propyl, $R_2$ = méthyl, X = N,
Y = C-$CO_2$H,

X --- Y = double liaison, $R_3$ =

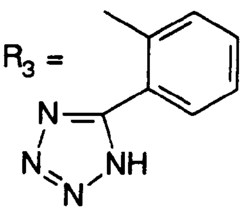

2.8 g de [n-Propyl-7 méthyl-5 [[(tétrazolyl-5)-2' biphényl-yl-4) méthyl]-8 1,2,4-triazolo [1,5-c] pyrimidine-yl-2] carboxylate d'éthyle préparés à l'exemple 109, sont dissous dans une solution de 1.8 g de carbonate de sodium dans 30 ml d'eau. La solution est agitée 30 heures à température ambiante puis acidifiée par barbotage de dioxyde de soufre et extraite au dichlorométhane. La phase organique est séchée sur sulfate de magnésium et évaporée à sec sous vide. Le résidu cristallise dans un mélange acétone/éther pour donner 2.3 g d'acide [n-Propyl-7 méthyl-5 [[(tétrazolyl-5)-2' biphényl-yl-4) méthyl]-8 1,2,4-triazolo [1,5-c] pyrimidine-yl-2] carboxylique sous forme de cristaux de point de fusion 193-194°C.

Exemple 111 : **n-Butyl-7 méthyl-5 [(cyano-2' biphényl-yl-4) méthyl]-8 1,2,4-triazolo [1,5-c] pyrimidine-one-2 (3H)**

**Formule (XII)** : $R_1$ = n-Butyl, $R_2$ = méthyl, X = NH,
Y = CO,

X --- Y = liaison simple, V =

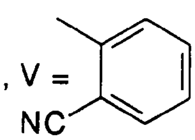

Préparé selon le mode opératoire de l'exemple 18.
Cristaux de point de fusion 235°C.

Exemple 112 : **n-Butyl-7 méthyl-5 [[(tétrazolyl-5)-2' biphényl-yl-4] méthyl]-8 1,2,4-triazolo [1,5-c] pyrimidine-one-2 (3H)**

**Formule (I)** : $R_1$ = n-Butyl, $R_2$ = méthyl, X = NH,
Y = CO,

X --- Y = liaison simple, $R_3$ =

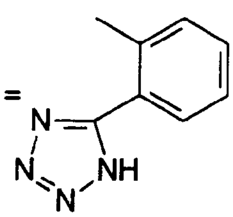

Préparé selon le mode opératoire de l'exemple 19.

Cristaux de point de fusion 236-238°C.

Exemple 113 : **Acide [[[n-Butyl-7 méthyl-5 oxo-2 1,2,4-triazolo [1,5-c] pyrimidine (3H)-yl-8] méthyl]-4' biphényl yl-2] carboxylique**

**Formule (I)** : $R_1$ = n-Butyl, $R_2$ = méthyl, X = NH, Y = CO,

X --- Y = liaison simple, $R_3$ = 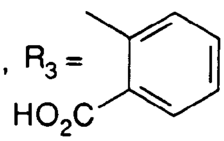

8 g de n-Butyl-7 méthyl-5 [(cyano-2' biphényl-yl-4) méthyl]-8 1,2,4-triazolo [1,5-c] pyrimidine-one-2 (3H) préparés à l'exemple 111, sont chauffés 10 heures au reflux dans une solution de 6 g de soude dans 30 ml d'éthylène glycol et 2 ml d'eau. Cette solution, après refroidissement, est acidifiée par l'acide chlorhydrique à pH = 5 et les cristaux formés sont essorés et séchés puis lavés à l'acétone pour donner 5 g d'acide [[[n-Butyl-7 méthyl-5 oxo-2 1,2,4-triazolo [1,5-c] pyrimidine (3H)-yl-8] méthyl]-4' biphénylyl-2] carboxylique sous forme de cristaux de point de fusion 210-211°C.

Exemple 114 : **n-Propyl-6 hydroxy-4 [(cyano-2' biphényl-yl-4) méthyl]-5 pyrimidine**

**Formule (VII)** : $R_1$ = n-Propyl, $R_2$ = H,

V = 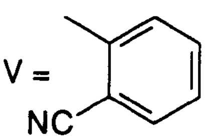

29 g de n-Propyl-6 méthylthio-2 hydroxy-4 [(cyano-2' biphényl-yl-4) méthyl]-5 pyrimidine, préparés à l'exemple 101, sont dissous dans 250 ml de diglyme et 60 g de Nickel de Raney sont ajoutés. Le mélange est chauffé 3 heures au reflux. Le catalyseur est filtré et lavé à l'éthanol, le filtrat est évaporé sous vide et le résidu est chromatographié sur gel de silice dans un éluant chloroforme 8 / acétone 2 pour donner 14.2 g de n-Propyl-6 hydroxy-4 [(cyano-2' biphényl-yl-4) méthyl]-5 pyrimidine sous forme de cristaux de point de fusion 158°C.

Exemple 115 : **n-Propyl-6 chloro-4 [(cyano-2' biphényl-yl-4) méthyl]-5 pyrimidine**

**Formule (VIII)** : $R_1$ = n-Propyl, $R_2$ = H,

V = 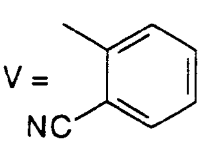

Préparé selon le mode opératoire de l'exemple 9.
Cristaux de point de fusion 95°C.

Exemple 116 : **n-Propyl-6 hydrazino-4 [(cyano-2' biphenyl-yl-4) méthyl]-5 pyrimidine**

**Formule (IX)** : $R_1$ = n-Propyl, $R_2$ = H,

V = 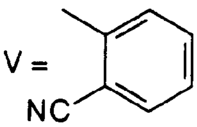

Préparé selon le mode opératoire de l'exemple 12.
Cristaux de point de fusion 120°C.

Exemple 117 : **n-Propyl-7 [(cyano-2' biphényl-yl-4) méthyl]-8 1,2,4-triazolo [4,3-c] pyrimidine-one-3 (2H)**

**Formule (XII)** : $R_1$ = n-Propyl, $R_2$ = H, X = CO,
Y = NH,

X ⎓ Y = liaison simple, V = 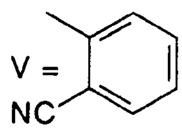

Préparé selon le mode opératoire de l'exemple 15.
Cristaux de point de fusion 124°C.

Exemple 118 : **n-Propyl-7 [(cyano-2' biphényl-yl-4) méthyl]-8 1,2,4-triazolo [1,5-c] pyrimidine-one-2 (3H)**

**Formule (XII)** : $R_1$ = n-Propyl, $R_2$ = H, X = NH,
Y = CO,

X ⎓ Y = liaison simple, V = 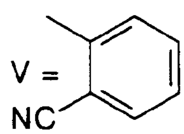

Préparé selon le mode opératoire de l'exemple 18.
Cristaux de point de fusion 199°C.

Exemple 119 : **n-Propyl-7 [[(tétrazolyl-5)-2' biphényl-yl-4] méthyl]-8 1,2,4-triazolo [1,5-c] pyrimidine-one-2 (3H)**

**Formule (I)** : $R_1$ = n-Propyl, $R_2$ = H, X = NH, Y = CO,

X $\overset{\text{- -}}{\text{—}}$ Y = liaison simple, $R_3$ = 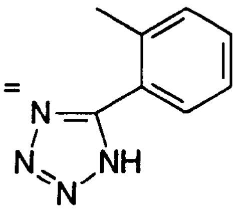

Préparé selon le mode opératoire de l'exemple 19.
Cristaux de point de fusion 190-192°C.

Exemple 120 : **n-Propyl-7 [(cyano-2' biphényl-yl-4) méthyl]-8 1,2,4-triazolo [1,5-c] pyrimidine**

**Formule (XII)** : $R_1$ = n-Propyl, $R_2$ = H, X = N, Y = CH,

X $\overset{\text{- -}}{\text{—}}$ Y = double liaison, V = 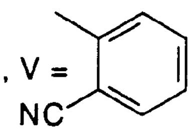

Préparé selon les modes opératoires des exemples 69 et 70.
Cristaux de point de fusion 104°C.

Exemple 121 : **n-Propyl-7 [[(tétrazolyl-5)-2' biphényl-yl-4] méthyl]-8 1,2,4-triazolo [1,5-c] pyrimidine**

**Formule (I)** : $R_1$ = n-Propyl, $R_2$ = H, X = N, Y = CH,

X $\overset{\text{- -}}{\text{—}}$ Y = double liaison, $R_3$ = 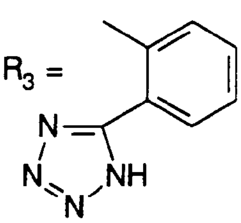

Préparé selon le mode opératoire de l'exemple 19.
Cristaux de point de fusion 131-133°C.

Exemple 122 : **n-Butyl-6 méthyl-2 hydrazino-4 [[(tétrazolyl-5)-2' biphényl-yl-4] méthyl]-5 pyrimidine**

**Formule (IX)** : $R_1$ = n-Butyl, $R_2$ = méthyl,

V = 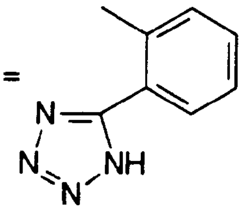

Préparé selon le mode opératoire de l'exemple 67.
Cristaux de point de fusion 166°C.

<u>Exemple 123</u> : **n-Butyl-7 méthyl-5 [[(tétrazolyl-5)-2' biphényl-yl-4] méthyl]-8 1,2,4-triazolo [4,3-c] pyrimidine**

**Formule (I)** : $R_1$ = n-Butyl, $R_2$ = méthyl, X = CH, Y = N,

X --- Y = double liaison, $R_3$ = 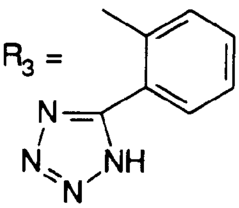

Préparé selon le mode opératoire de l'exemple 72, purifié par chromatographie sur gel de silice dans un éluant dichlorométhane 95 / méthanol 5 (2ème produit élué).
Cristaux de point de fusion 185-186°C.

<u>Exemple 124</u> : **n-Butyl-7 méthyl-5 [[(tétrazolyl-5)-2' biphényl-yl-4] méthyl]-8 1,2,4-triazolo [1,5-c] pyrimidine**

**Formule (I)** : $R_1$ = n-Butyl, $R_2$ = méthyl, Y = CH, X = N,

X --- Y = double liaison, $R_3$ = 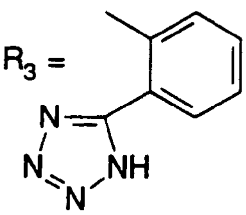

Préparé selon le mode opératoire de l'exemple 123, purifié par chromatographie sur gel de silice dans un éluant dichlorométhane 95 / méthanol 5 (1er produit élué).
Cristaux de point de fusion 172-173°C.

Exemple 125 : **n-Butyl-7 méthyl-5 [[(tétrazolyl-5)-2'biphényl-yl-4] méthyl]-8 mercapto-3 1,2,4-triazolo [4,3-c] pyrimidine**

**Formule (I)** : $R_1$ = n-Butyl, $R_2$ = méthyl, X = C-SH,
Y = N,

X ⎓ Y = double liaison, $R_3$ =

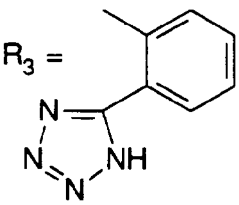

5.9 g de n-Butyl-6 méthyl-2 hydrazino-4 [[(tétrazolyl-5)-2'biphényl-yl-4] méthyl]-5 pyrimidine préparé à l'exemple 122, sont ajoutés à un mélange contenant 3.1 ml de sulfure de carbone, 1.4 g de soude, 36 ml de méthanol et 2 ml d'eau. Ce mélange est porté 1 heure au reflux, puis évaporé à sec ; 80 ml d'éthanol sont ajoutés et le mélange obtenu est chauffé une heure au reflux, puis concentré sous vide, repris à l'eau, acidifié par l'acide chlorhydrique et extrait au dichlorométhane. La phase organique est évaporée et le résidu est chromatographié sur gel de silice dans un éluant dichlorométhane 95 / méthanol 5 pour donner 3.2 g de n-Butyl-7 méthyl-5 [[(tétrazolyl-5)-2' biphényl-yl-4] méthyl]-8 mercapto-3 1,2,4-triazolo [4,3-c] pyrimidine sous forme de cristaux de point de fusion 172-173°C.

Exemple 126 : **n-Butyl-7 méthyl-5 [[(tétrazolyl-5)-2'biphényl-yl-4] méthyl]-8 mercapto-2 1,2,4-triazolo [1,5-c] pyrimidine**

**Formule (I)** : $R_1$ = n-Butyl, $R_2$ = méthyl, X = N
Y = C-SH,

X ⎓ Y = double liaison, $R_3$ =

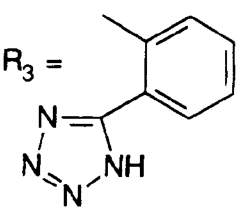

2.7 g de n-Butyl-7 méthyl-5 [[(tétrazolyl-5)-2' biphényl-yl-4] méthyl]-8 mercapto-31,2,4-triazolo [4,3-c] pyrimidine, sont dissous dans 100 ml d'eau et 0.6 g de soude. Le mélange est chauffé 3 heures au reflux puis refroidi et acidifié par l'acide chlorhydrique concentré et extrait au dichlorométhane. La phase organique est séchée sur sulfate de magnésium et évaporée sous vide. Le résidu est chromatographié sur gel de silice dans un éluant dichlorométhane 95 / méthanol 5 pour donner 1 g de n-Butyl-7 méthyl-5 [[(tétrazolyl-5)-2' biphényl-yl-4] méthyl]-8 mercapto-2 1,2,4-triazolo [1,5-c] pyrimidine sous forme de cristaux de point de fusion 135-137°C.

Exemple 127 : **[n-Butyl-7 méthyl-5 [(cyano-2' biphényl-yl-4) méthyl]-8 1,2,4-triazolo [1,5-c] pyrimidine-yl-2] oxy-2 éthanol**

**Formule (XII) :** $R_1$ = n-Butyl, $R_2$ = méthyl, X = N,
Y = C-$OCH_2$-$CH_2$-OH,

V = 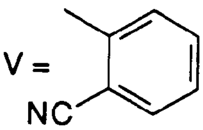

Préparé selon le mode opératoire de l'exemple 27 à partir du bromo-2 éthanol.
Huile utilisée telle quelle pour la suite.

Exemple 128 : **Acétate de [n-Butyl-7 méthyl-5 [(cyano-2' biphényl-yl-4) méthyl]-8 1,2,4-triazolo [1,5-c] pyrimidine-yl-2] oxy-2 éthanol**

**Formule (XII) :** $R_1$ = n-Butyl, $R_2$ = méthyl, X = N,
Y = C-$OCH_2$-$CH_2$-O-CO-$CH_3$

X --- Y = double liaison, V = 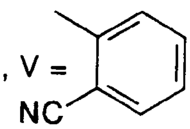

4.4 g de [n-Butyl-7 méthyl-5 [(cyano-2' biphényl-yl-4) méthyl]-8 1,2,4-triazolo [1,5-c] pyrimidine-yl-2] oxy-2 éthanol préparé à l'exemple 127 sont chauffés 2 heures au reflux dans 45 ml d'anhydride acétique. Le mélange est ensuite évaporé à sec pour donner 5 g d'acétate de [n-Butyl-7 méthyl-5 [(cyano-2' biphényl-yl-4) méthyl]-8 1,2,4-triazolo [1,5-c] pyrimidine-yl-2] oxy-2 éthanol sous forme d'huile utilisée telle quelle pour la suite.

Exemple 129 : **Acétate de [n-Butyl-7 méthyl-5 [[(tétrazolyl-5)-2' biphényl-yl-4] méthyl]-8 1,2,4-triazolo [1,5-c] pyrimidine-yl-2] oxy-2 éthanol**

**Formule (I) :** $R_1$ = n-Butyl, $R_2$ = méthyl, X = N,
Y = C-$OCH_2$-$CH_2$-O-CO-$CH_3$

X --- Y = double liaison, $R_3$ = 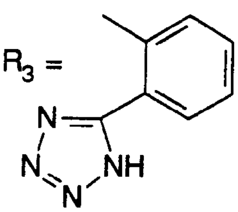

Préparé selon le mode opératoire de l'exemple 19.
Cristaux de point de fusion 141-143°C.

<u>Exemple 130</u> : **[n-Butyl-7 méthyl-5 [(cyano-2' biphényl-yl-4) méthyl]-8 1,2,4-triazolo [1,5-c] pyrimidine-yl-2] mercapto acétate d'éthyle**

**Formule (XII)** : $R_1$ = n-Butyl, $R_2$ = méthyl, X = N,
Y = C-$SCH_2$-$CO_2Et$,

X ---- Y = double liaison, V = 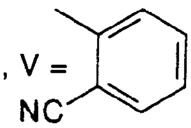

Préparé selon le mode opératoire de l'exemple 27 à partir du bromoacétate d'éthyle et de la n-Butyl-7 méthyl-5 mercapto-2 [(cyano-2' biphényl-yl-4) méthyl]-8 1,2,4-triazolo [1,5-c] pyrimidine.
Cristaux de point de fusion 93°C.

<u>Exemple 131</u> : **[n-Butyl-7 méthyl-5 [[(tétrazolyl-5)-2' biphényl-yl-4] méthyl]-8 1,2,4-triazolo [1,5-c] pyrimidine-yl-2] mercaptoacétate d'éthyle**

**Formule (I)** : $R_1$ = n-Butyl, $R_2$ = méthyl, X = N,
Y = C-$SCH_2$-$CO_2Et$,

X ---- Y = double liaison, $R_3$ = 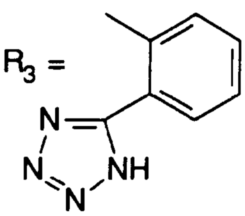

Préparé selon le mode opératoire de l'exemple 19.
Cristaux de point de fusion 155-156°C.

<u>Exemple 132</u> : **Acétate de [n-Butyl-7 méthyl-5 [[(tétrazolyl-5)-2' biphényl-yl-4] méthyl]-8 1,2,4-triazolo [1,5-c] pyrimidine-yl-2] mercapto-2 éthanol**

**Formule (I)** : $R_1$ = n-Butyl, $R_2$ = méthyl, X = N,
Y = C-$SCH_2$-$CH_2$-O-CO-$CH_3$

X ---- Y = double liaison, $R_3$ = 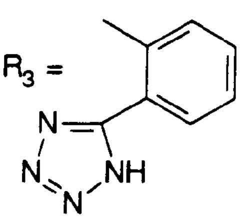

Préparé selon le mode opératoire de l'exemple 27 à partir de l'acétate du bromo-2 éthanol et de la n-Butyl-7 méthyl-5 mercapto-2 [[(tétrazolyl-5)-2' biphényl-yl-4] méthyl]-8 1,2,4-triazolo [1,5-c] pyrimidine, préparé à l'exemple 126.
Cristaux de point de fusion 173-175°C.

Exemple 133 : **n-Propyl-6 éthyl-2 hydroxy-4 [(cyano-2' biphényl-yl-4) méthyl]-5 pyrimidine**

**Formule (VII) :** $R_1$ = n-Propyl, $R_2$ = éthyl,

V = 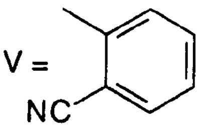

Préparé selon le mode opératoire de l'exemple 6.
Cristaux de point de fusion 216°C.

Exemple 134 : **n-Propyl-6 éthyl-2 chloro-4 [(cyano-2' biphényl-yl-4) méthyl]-5 pyrimidine**

**Formule (VIII) :** $R_1$ = n-Propyl, $R_2$ = éthyl,

V = 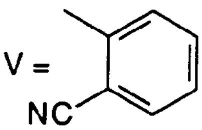

Préparé selon le mode opératoire de l'exemple 9.
Huile utilisée telle quelle pour la suite.

Exemple 135 : **n-Propyl-6 éthyl-2 hydrazino-4 [(cyano-2' biphényl yl-4) méthyl]-5 pyrimidine**

**Formule (IX) :** $R_1$ = n-Propyl, $R_2$ = éthyl,

V = 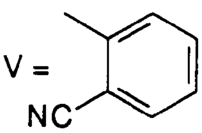

Préparé selon le mode opératoire de l'exemple 12.
Cristaux de point de fusion 80°C.

Exemple 136 : **n-Propyl-7 éthyl-5 [(cyano-2' biphényl-yl-4) méthyl]-8 1,2,4-triazolo [4,3-c] pyrimidine one-3 (2H)**

**Formule (XII) :** $R_1$ = n-Propyl, $R_2$ = éthyl, X = CO, Y = NH,

X ⎓ Y = liaison simple, V = 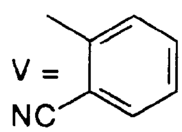

Préparé selon le mode opératoire de l'exemple 15.
Cristaux de point de fusion 170°C.

Exemple 137 : **n-Propyl-7 éthyl-5 [(cyano-2' biphényl-yl-4) méthyl]-8 1,2,4-triazolo [1,5-c] pyrimidine one-2 (3H)**

**Formule (XII) :** $R_1$ = n-Propyl, $R_2$ = éthyl, X = NH, Y = CO,

X --- Y = liaison simple, V =
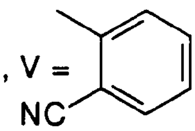

Préparé selon le mode opératoire de l'exemple 18.
Cristaux de point de fusion 208°C.

Exemple 138 : **n-Propyl-7 éthyl-5 [[(tétrazolyl-5)-2' biphényl-yl-4] méthyl]-8 1,2,4-triazolo [1,5-c] pyrimidine one-2 (3H)**

**Formule (I) :** $R_1$ = n-Propyl, $R_2$ = éthyl, X = NH, Y = CO,

X --- Y = liaison simple, $R_3$ =
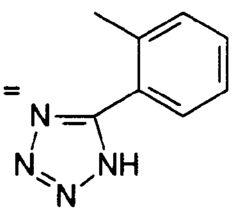

Préparé selon le mode opératoire de l'exemple 19.
Cristaux de point de fusion 255-256°C.

Exemple 139 : **di-n-propyl-2,6-hydroxy-4 [(cyano-2' biphényl-yl-4) méthyl]-5 pyrimidine**

**Formule (VII)** : $R_1$ = $R_2$ = n-Propyl,

V =
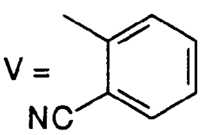

Préparé selon le mode opératoire de l'exemple 6.
Cristaux de point de fusion 150°C.

Exemple 140 : **di-n-propyl-2,6 chloro-4 [(cyano-2' biphényl-yl-4) méthyl]-5 pyrimidine**

**Formule (VIII)** : $R_1$ = $R_2$ = n-Propyl,

V =
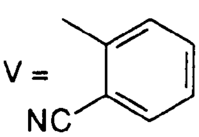

Préparé selon le mode opératoire de l'exemple 9.
Huile utilisée telle quelle pour la suite.

Exemple 141 : **di-n-propyl-2,6 hydrazino-4 [(cyano-2' biphényl-yl-4) méthyl]-5 pyrimidine**

**Formule (IX)** : $R_1 = R_2 =$ n-Propyl,

V = 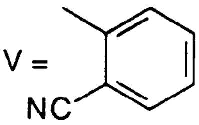

Préparé selon le mode opératoire de l'exemple 12.
Huile utilisée telle quelle pour la suite.

Exemple 142 : **di-n-propyl-5,7 [(cyano-2' biphényl-yl-4) méthyl]-8 1,2,4-triazolo [4,3-c] pyrimidine-one-3 (2H)**

**Formule (XII)** : $R_1 = R_2 =$ n-Propyl, X = CO, Y = NH

X $\overset{--}{—}$ Y = liaison simple, V = 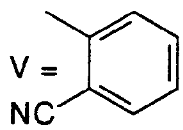

Préparé selon le mode opératoire de l'exemple 15.
Cristaux de point de fusion 149°C.

Exemple 143 : **di-n-propyl-5,7 [(cyano-2' biphényl-yl-4) méthyl]-8 1,2,4-triazolo [1,5-c] pyrimidine-one-2 (3H)**

**Formule (XII)** : $R_1 = R_2 =$ n-Propyl, X = NH, Y = CO,

X $\overset{--}{—}$ Y = liaison simple, V = 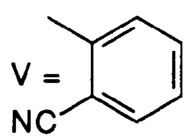

Préparé selon le mode opératoire de l'exemple 18.
Cristaux de point de fusion 184°C.

Exemple 144 : **di-n-propyl-5,7 [[(tétrazolyl-5)-2' biphényl-yl-4] méthyl]-8 1,2,4-triazolo [1,5-c] pyrimidine-one-2 (3H)**

**Formule (I)** : $R_1 = R_2 =$ n-Propyl, X = NH, Y = CO,

X $\overset{--}{—}$ Y = liaison simple, $R_3$ = 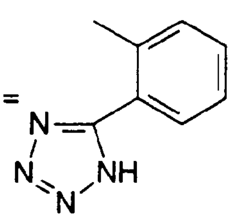

Préparé selon le mode opératoire de l'exemple 19.
Cristaux de point de fusion 258-259°C.

Exemple 145 : **[n-Propyl-7 méthyl-5 [(cyano-2' biphényl-yl-4) méthyl]-8 1,2,4-triazolo [1,5-c] pyrimidine-yl-2] acétate d'éthyle**

**Formule (XII) :** $R_1$ = n-Propyl, $R_2$ = méthyl, X = N,
Y = C-$CH_2CO_2Et$,

X ⎓ Y = double liaison, V = 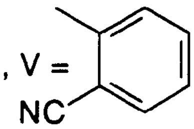

Préparé selon le mode opératoire de l'exemple 108 à partir du chlorure d'acide ester éthylique de l'acide malonique.

Cristaux de point de fusion 100°C.

Exemple 146 : **[n-Propyl-7 méthyl-5 [[(tétrazolyl-5)-2' biphényl-yl-4] méthyl]-8 1,2,4-triazolo [1,5-c] pyrimidine-yl-2] acétate d'éthyle**

**Formule (I) :** $R_1$ = n-Propyl, $R_2$ = méthyl, X = N,
Y = C-$CH_2CO_2Et$,

X ⎓ Y = double liaison, $R_3$ = 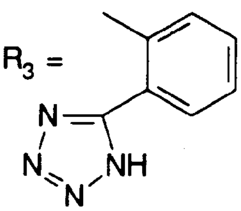

Préparé selon le mode opératoire de l'exemple 19.

Cristaux de point de fusion 150°C.

Exemple 147 : **[n-Propyl-7 méthyl-5 [(cyano-2' biphényl-yl-4) méthyl]-8 1,2,4-triazolo [1,5-c] pyrimidine-yl-2] amino acétate d'éthyle**

**Formule (XII) :** $R_1$ = n-Propyl, $R_2$ = méthyl, X = N,
Y = C-NH $CH_2CO_2Et$,

X ⎓ Y = double liaison, V = 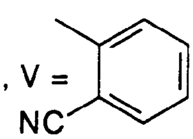

Préparé selon le mode opératoire de l'exemple 79 à partir de l'isothiocyanatoacétate d'éthyle.

Cristaux de point de fusion 132°C.

Exemple 148 : **[n-Propyl-7 méthyl-5 [[(tétrazolyl-5)-2' biphényl-yl-4] méthyl]-8 1,2,4-triazolo [1,5-c] pyrimidine-yl-2] amino acétate d'éthyle**

**Formule (I) :** $R_1$ = n-Propyl, $R_2$ = méthyl, X = N,
Y = C-NH $CH_2CO_2Et$,

X --- Y = double liaison, $R_3$ =

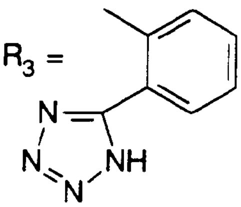

Préparé selon le mode opératoire de l'exemple 19.
Cristaux de point de fusion 180-181°C.

Exemple 149 : **[Ethyl-7 méthyl-5 [(cyano-2' biphényl-yl-4) méthyl]-8 1,2,4-triazolo [1,5-c] pyrimidine-yl-2] carboxylate d'éthyle**

**Formule (XII) :** $R_1$ = Ethyl, $R_2$ = méthyl, X = N,
Y = C-$CO_2Et$,

X --- Y = double liaison, V =

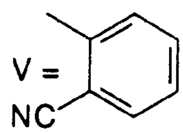

Préparé selon le mode opératoire de l'exemple 108.
Cristaux de point de fusion 160°C.

Exemple 150 : **[Ethyl-7 méthyl-5 [[(tétrazolyl-5)-2' biphényl-yl-4] méthyl]-8 1,2,4-triazolo [1,5-c] pyrimidine-yl-2] carboxylate d'éthyle**

**Formule (I) :** $R_1$ = Ethyl, $R_2$ = méthyl, X = N,
Y = C-$CO_2Et$,

X --- Y = double liaison, $R_3$ =

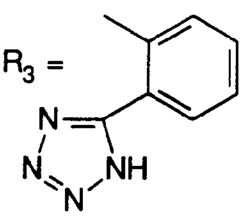

Préparé selon le mode opératoire de l'exemple 19.
Cristaux de point de fusion 207-208°C.

Exemple 151 : **n-Propyl-7 méthyl-5 méthoxyméthyl-2 [(cyano-2' biphényl-yl-4) méthyl]-8 1,2,4-triazolo [1,5-c] pyrimidine**

**Formule (XII) :** $R_1$ = n-Propyl, $R_2$ = méthyl, X = N,
Y = C-$CH_2$-$OCH_3$,

X --- Y = double liaison, V = 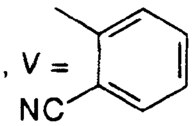

Préparé selon le mode opératoire de l'exemple 108 à partir du chlorure de l'acide méthoxy-2 acétique.
Huile utilisée telle quelle pour la suite.

Exemple 152 : **n-Propyl-7 méthyl-5 méthoxyméthyl-2 [[(tétrazolyl-5)-2' biphényl-yl-4) méthyl]-8 1,2,4-triazolo [1,5-c] pyrimidine**

**Formule (I) :** $R_1$ = n-Propyl, $R_2$ = méthyl, X = N,
Y = C-$CH_2$-$OCH_3$,

X --- Y = double liaison, $R_3$ = 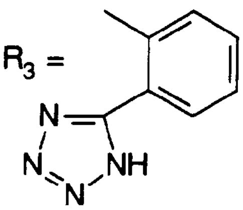

Préparé selon le mode opératoire de l'exemple 19.
Cristaux de point de fusion 130-131°C.

Exemple 153 : **Acide [Ethyl-7 méthyl-5 [[(tétrazolyl-5)-2' biphényl-yl-4] méthyl]-8 1,2,4-triazolo [1,5-c] pyrimidine-yl-2] carboxylique**

**Formule (I) :** $R_1$ = Ethyl, $R_2$ = méthyl, X = N,
Y = C-$CO_2$H,

X --- Y = double liaison, $R_3$ = 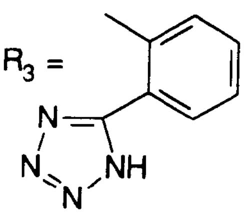

2.2 g de [Ethyl-7 méthyl-5 [[(tétrazolyl-5)-2' biphényl-yl-4] méthyl]-8 1,2,4-triazolo [1,5-c] pyrimidine-yl-2] carboxylate d'éthyle, préparé à l'exemple 150, sont dissous dans 50 ml d'eau contenant 0.56 g de soude. Le mélange est chauffé à 80°C pendant 3 heures, puis refroidi et acidifié par barbotage de dioxyde de soufre. Les cristaux formés sont essorés, lavés à l'éther et à l'acétate d'éthyle pour donner 1.4 g d'acide [Ethyl-7 méthyl-5 [[(tétrazolyl-5)-2' biphényl-yl-4] méthyl]-8 1,2,4-triazolo [1,5-c] pyrimidine-yl-2] carboxylique sous forme de cristaux de point de fusion 194-198°C.

Exemple 154 : **cyclopropyl-3 oxo-3 propionate d'éthyle**

**Formule (II)** : $R_1$ = cyclopropyl, $R_6$ = éthyl

Préparé selon le mode opératoire de l'exemple 1.
Huile de point d'ébullition $Eb_{20}$ = 115-118°C.

Exemple 155 : **[(cyano-2' biphényl-yl-4) méthyl]-2 cyclopropyl-3 oxo-3 propionate d'éthyle**

**Formule (IV) :** $R_1$ = cyclopropyl, $R_6$ = éthyl,

V = 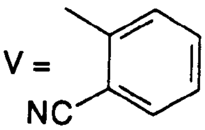

Préparé selon le mode opératoire de l'exemple 3.
Huile utilisée telle quelle pour la suite.

Exemple 156 : **cyclopropyl-6 méthyl-2 hydroxy-4 [(cyano-2' biphényl-yl-4) méthyl]-5 pyrimidine**

**Formule (VII) :** $R_1$ = cyclopropyl, $R_2$ = méthyl,

V = 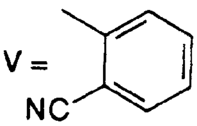

Préparé selon le mode opératoire de l'exemple 6.
Cristaux de point de fusion 230°C.

Exemple 157 : **cyclopropyl-6 méthyl-2 chloro-4 [(cyano-2' biphényl-yl-4) méthyl]-5 pyrimidine**

**Formule (VIII) :** $R_1$ = cyclopropyl, $R_2$ = méthyl,

V = 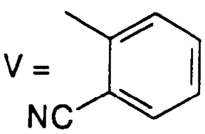

Préparé selon le mode opératoire de l'exemple 9.
Huile utilisée telle quelle pour la suite.

Exemple 158 : **cyclopropyl-6 méthyl-2 hydrazino-4 [(cyano-2' biphényl-yl-4) méthyl]-5 pyrimidine**

**Formule (IX) :** $R_1$ = cyclopropyl, $R_2$ = méthyl,

V = 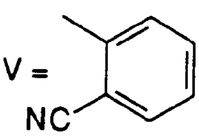

Préparé selon le mode opératoire de l'exemple 12.
Cristaux de point de fusion 170°C.

Exemple 159 : **cyclopropyl-7 méthyl-5 [(cyano-2' biphényl-yl-4) méthyl]-8 1,2,4-triazolo [4,3-c] pyrimidine one-3 (2H)**

**Formule (XII) :** $R_1$ = cyclopropyl, $R_2$ = méthyl, X = CO, Y = NH,

X $\stackrel{--}{—}$ Y = liaison simple, V = 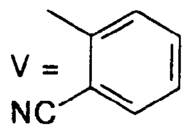

Préparé selon le mode opératoire de l'exemple 15. Cristaux de point de fusion 204°C.

Exemple 160 : **cyclopropyl-7 méthyl-5 [(cyano-2' biphényl-yl-4) méthyl]-8 1,2,4-triazolo [1,5-c] pyrimidine one-2 (3H)**

**Formule (XII) :** $R_1$ = cyclopropyl, $R_2$ = méthyl, Y = CO, X = NH,

X $\stackrel{--}{—}$ Y = liaison simple, V = 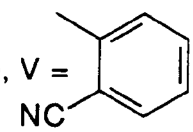

Préparé selon le mode opératoire de l'exemple 18. Cristaux de point de fusion 270°C.

Exemple 161 : **cyclopropyl-7 méthyl-5 [[(tétrazolyl-5)-2' biphényl-yl-4] méthyl]-8 1,2,4-triazolo [1,5-c] pyrimidine one-2 (3H)**

**Formule (I) :** $R_1$ = cyclopropyl, $R_2$ = méthyl, Y = CO, X = NH,

X $\stackrel{--}{—}$ Y = liaison simple, $R_3$ = 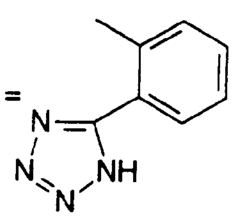

Préparé selon le mode opératoire de l'exemple 19. Cristaux de point de fusion 264-265°C.

Exemple 162 : **cyclopropyl-7 diméthyl-2,5 [(cyano-2' biphényl-yl-4) méthyl]-8 1,2,4-triazolo [1,5-c] pyrimidine**

**Formule (XII) :** $R_1$ = cyclopropyl, $R_2$ = méthyl,
Y = C-$CH_3$, X = N,

X --- Y = double liaison, V = 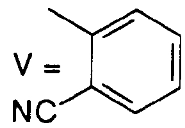

Préparé selon le mode opératoire de l'exemple 73.
Cristaux de point de fusion 120°C.

Exemple 163 : **cyclopropyl-7 diméthyl-2,5 [[(tétrazolyl-5)-2' biphényl-yl-4] méthyl]-8 1,2,4-triazolo [1,5-c] pyrimidine**

**Formule (I) :** $R_1$ = cyclopropyl, $R_2$ = méthyl,
Y = C-$CH_3$, X = N,

X --- Y = double liaison, $R_3$ = 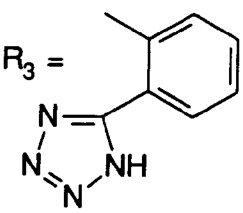

Préparé selon le mode opératoire de l'exemple 19.
Cristaux de point de fusion 186-188°C.

Exemple 164 : **n-Propyl-6 méthoxyméthyl-2 hydroxy-4 [(cyano-2' biphényl-yl-4) méthyl]-5 pyrimidine**

**Formule (VII) :** $R_1$ = n-Propyl, $R_2$ = $CH_2OCH_3$,

V = 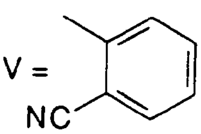

Préparé selon le mode opératoire de l'exemple 6 à partir du chlorhydrate de méthoxyacétamidine dont on peut trouver une préparation dans la référence C.A : 63 , P 9963e.
Cristaux de point de fusion 134°C.

Exemple 165 : **n-Propyl-6 méthoxyméthyl-2 chloro-4 [(cyano-2' biphényl-yl-4) méthyl]-5 pyrimidine**

**Formule (VIII) :** $R_1$ = n-Propyl, $R_2$ = $CH_2OCH_3$,

V = 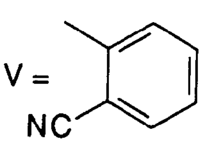

Préparé selon le mode opératoire de l'exemple 9.
Huile utilisée telle quelle pour la suite.

Exemple 166 : **n-Propyl-6 méthoxyméthyl-2 hydrazino-4 [(cyano-2' biphényl-yl-4) méthyl]-5 pyrimidine**

**Formule (IX) :** $R_1$ = n-Propyl, $R_2$ = $CH_2OCH_3$,

V = 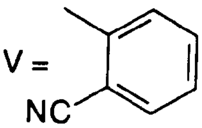

Préparé selon le mode opératoire de l'exemple 12.
Huile utilisée telle quelle pour la suite.

Exemple 167 : **n-Propyl-7 méthoxyméthyl-5 [(cyano-2' biphényl-yl-4) méthyl]-8 1,2,4-triazolo [4,3-c] pyrimidine one-3 (2H)**

**Formule (XII) :** $R_1$ = n-Propyl, $R_2$ = $CH_2OCH_3$,
X = CO, Y = NH,

X --- Y = liaison simple, V = 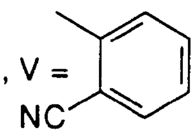

Préparé selon le mode opératoire de l'exemple 15.
Cristaux de point de fusion 108°C.

Exemple 168 : **n-Propyl-7 méthoxyméthyl-5 [(cyano-2' biphényl-yl-4) méthyl]-8 1,2,4-triazolo [1,5-c] pyrimidine one-2 (3H)**

**Formule (XII) :** $R_1$ = n-Propyl, $R_2$ = $CH_2OCH_3$,
Y = CO, X = NH,

X --- Y = liaison simple, V = 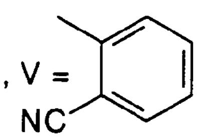

Préparé selon le mode opératoire de l'exemple 18.
Huile utilisée telle quelle pour la suite.

Exemple 169 : **n-Propyl-7 méthoxyméthyl-5 [[(tétrazolyl-5)-2' biphényl-yl-4] méthyl]-8 1,2,4-triazolo [1,5-c] pyrimidine one-2 (3H)**

**Formule (I) :** $R_1$ = n-Propyl, $R_2$ = $CH_2OCH_3$,
Y = CO, X = NH,

X ⎓ Y = liaison simple, $R_3$ = 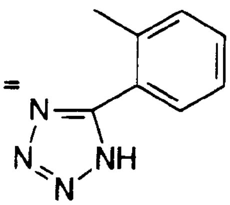

Préparé selon le mode opératoire de l'exemple 19.
Cristaux de point de fusion 166-168°C.

Exemple 170 : **n-Propyl-7 méthoxyméthyl-5 méthyl-2 [(cyano-2' biphényl-yl-4) méthyl]-8 1,2,4-triazolo [1,5-c] pyrimidine**

**Formule (XII) :** $R_1$ = n-Propyl, $R_2$ = $CH_2OCH_3$,
Y = C-$CH_3$, X = N,

X ⎓ Y = double liaison, V = 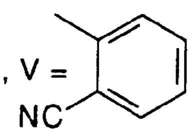

Préparé selon le mode opératoire de l'exemple 73.
Huile utilisée telle quelle pour la suite.

Exemple 171 : **n-Propyl-7 méthoxyméthyl-5 méthyl-2 [[(tétrazolyl-5)-2' biphényl-yl-4] méthyl]-8 1,2,4-triazolo [1,5-c] pyrimidine**

**Formule (I) :** $R_1$ = n-Propyl, $R_2$ = $CH_2OCH_3$,
Y = C-$CH_3$, X = N,

X ⎓ Y = double liaison, $R_3$ = 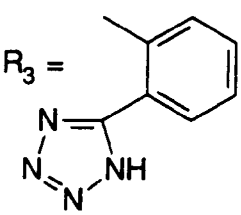

Préparé selon le mode opératoire de l'exemple 19.
Cristaux de point de fusion 137-138°C.

Exemple 172 : **bromure de (cyano-3 thiényl-2)-4 benzyle**

A) Méthyl-4' chloro-4 butyrophénone :

53 ml de toluène et 70,5 g de chlorure de l'acide chloro-4 butyrique sont dissous dans 100 ml de dichlorométhane et la solution est ajoutée à 10°C à une suspension de 74 g de chlorure d'aluminium dans 200 ml de dichlorométhane. On laisse ensuite la température s'élever pendant un quart d'heure, le mélange est traité par de l'eau glacée. La phase organique est séchée sur sulfate de magnésium et évaporée sous vide pour donner 96,9 g de méthyl-4' chloro-4 butyrophénone sous forme d'une huile utilisée telle quelle pour la suite.

B) α-chloro β-(chloro-2 éthyl) méthyl-4 cinnamaldéhyde :

130 ml d'oxychlorure de phosphore sont ajoutés lentement, à 0°C, sur 130 ml de diméthyl formamide, puis 117,5 g de méthyl-4' chloro-4 butyrophénone, préparée en A), en solution dans 50 ml de diméthyl formamide sont ajoutés goutte à goutte. Le mélange est ensuite agité à température ambiante pendant une heure, puis à 50°C pendant 2 heures et à 70°C pendant 1 heure. Le mélange est alors versé sur de la glace et repris à l'éther, la phase éthérée est lavée avec une solution saturée de bicarbonate de sodium, séchée sur sulfate de sodium et évaporée sous vide pour donner 133,8 g d'α-chloro β-(chloro-2 éthyl) méthyl-4 cinnamaldéhyde sous forme d'une huile utilisée telle quelle pour la suite.

C) (méthyl-4 phényl)-2 dihydro-4,5-thiophène-3 carboxaldéhyde :

15,9 g d'α-chloro β-(chloro-2 éthyl) méthyl-4 cinnamaldéhyde préparés en B) et 22 g de sulfure de sodium (9 $H_2O$) sont ajoutés à 200 ml de THF. Une quantité d'eau suffisante est ajoutée afin que le sulfure de sodium passe entièrement en solution et le mélange est ensuite chauffé pendant 3 heures au reflux, refroidi puis repris à l'éther. La phase organique est décantée, lavée à l'eau puis séchée sur sulfate de magnésium et évaporée sous vide pour donner 13,5 g de (méthyl-4 phényl)-2 dihydro-4,5-thiophène-3 carboxaldéhyde sous forme d'une huile utilisée telle quelle pour la suite.

D) (Méthyl-4 phényl)-2 cyano-3 dihydro-4,5-thiophène :

15 g de (méthyl-4 phényl)-2 dihydro-4,5-thiophène-3 carboxaldéhyde, préparés en C) et 6,5 g de chlorhydrate d'hydroxylamine sont mélangés dans 40 ml d'éthanol et 10 ml d'eau. Une solution de 4,7 g de carbonate de sodium dans 10 ml d'eau est ajoutée. Le mélange est agité à température ambiante pendant une demi-heure, puis extrait à l'éther. La phase éthérée est lavée à l'eau, puis séchée sur sulfate de sodium et évaporée sous vide pour donner 15,2 g d'un résidu jaune gommeux. Ce résidu est ajouté à 13 ml d'anhydride acétique et le mélange chauffe légèrement, brunit et devient liquide. Le mélange est ensuite chauffé 1 heure au reflux, puis versé sur de la glace et extrait au dichlorométhane et lavé par une solution saturée de bicarbonate de soude, puis la phase organique est séchée sur sulfate de magnésium et évaporée sous vide, le résidu obtenu est chromatographié sur gel de silice dans le dichlorométhane pour donner 10 g de (méthyl-4 phényl)-2 cyano-3 dihydro-4,5-thiophène sous forme d'une huile utilisée telle quelle pour la suite.

E) (Méthyl-4 phényl)-2 cyano-3 thiophène :

49,9 g de (Méthyl-4 phényl)-2 cyano-3 dihydro-4,5-thiophène, préparés en D) sont dissous dans 200 ml de tétrachlorure de carbone, le mélange est chauffé au reflux et, au bout de deux heures, 11 g de brome en solution dans 200 ml de tétrachlorure de carbone sont ajoutés goutte à goutte. Le reflux est poursuivi jusqu'à cessation du dégagement d'acide bromhydrique puis le solvant est évaporé sous vide. Le résidu est repris dans 200 ml de tétrahydrofurane anhydre et 28 g de tertiobutylate de potassium sont ajoutés. Le mélange est chauffé une heure au reflux puis refroidi et additionné d'eau et de chlorure de sodium et extrait à l'éther. La phase organique est évaporée sous vide pour donner 31,8 g de (méthyl-4 phényl)-2 cyano-3 thiophène sous forme d'huile utilisée telle quelle pour la suite.

F) Bromure de (cyano-3 thiényl-yl-2)-4 benzyle :

24,5 g de (méthyl-4 phényl)-2 cyano-3 thiophène, préparés en E), sont dissous dans 200 ml de tétrachlorure de carbone. 21,9 g de N-bromo succinimide sont ajoutés ainsi que 0,1 g de péroxyde de benzoyle. Le mélange est chauffé 24 heures au reflux. Les cristaux de succinimide sont filtrés et le solvant évaporé sous vide. Le résidu est repris dans un mélange d'hexane et d'acétate d'éthyle et la solution est gardée 24 heures au congélateur. Les cristaux formés sont essorés pour donner 14 g de bromure de (cyano-3 thiényl-yl-2)-4 benzyle sous forme de cristaux de point de fusion 80°C.

Exemple 173 : **[(cyano-3 thiényl-yl-2)-4 benzyl]-2 oxo-3 hexanoate d'éthyle**

**Formule (IV)** : $R_1$ = n-Propyl, $R_6$ = Ethyl,

V = 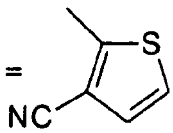

11 g d'oxo-3 hexanoate d'éthyle sont mis en solution dans 150 ml de tétrahydrofurane. 12,9 g de bromure de (cyano-3 thiényl-yl-2)-4 benzyle et 6,1 g de bromure de lithium sont ajoutés et le mélange est agité à température ambiante. On introduit alors goutte à goutte, 24,2 ml de diisopropyl éthyl amine. Après la fin de l'addition le mélange réactionnel est porté 24 heures au reflux. Après évaporation sous vide, le résidu obtenu est repris à l'eau puis extrait au chloroforme. La phase organique est séchée puis évaporée sous vide. L'excès d'oxo-3 hexanoate d'éthyle est enlevé à la pompe à palette. On obtient ainsi 16,4 g de [(cyano-3 thiényl-yl-2)-4 benzyl]-2 oxo-3 hexanoate d'éthyle sous forme d'une huile jaune pâle utilisée telle quelle pour la suite.

Exemple 174 : **n-Propyl-6 méthyl-2 hydroxy-4 [(cyano-3 thiényl-yl-2)-4 benzyl]-5 pyrimidine**

**Formule (VII)** : $R_1$ = n-Propyl, $R_2$ = Méthyl,

V = 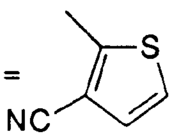

0,23 g de sodium sont dissous dans 10 ml d'éthanol. A cette solution est ajouté 1 g de chlorhydrate d'acétamidine et le mélange est agité cinq minutes à température ambiante. 2,4 g de [(cyano-3 thiényl-yl-2)-4 benzyl]-2 oxo-3 hexanoate d'éthyle sont alors ajoutés et le mélange est agité 48 heures à température ambiante puis trois heures au reflux. Après refroidissement, de l'eau acidifiée par une solution d'acide chlorhydrique est ajoutée et on laisse se déposer un solide. Ce précipité est essoré, lavé à l'eau puis avec un peu d'éther et séché. On récupère ainsi 1,4 g de n-Propyl-6 méthyl-2 hydroxy-4 [(cyano-3 thiényl-yl-2)-4 benzyl]-5 pyrimidine sous forme de cristaux blancs de point de fusion 180°C.

Exemple 175 : **n-Propyl-6 méthyl-2 chloro-4 [(cyano-3 thiényl-yl-2)-4 benzyl]-5 pyrimidine**

**Formule (VIII)** : $R_1$ = n-Propyl, $R_2$ = Méthyl,

V = 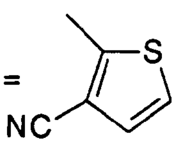

1,6 g de n-Propyl-6 méthyl-2 hydroxy-4[(cyano-3 thiényl-yl-2) 4-benzyl]-5 pyrimidine sont mis en suspension dans 1,7 ml d'oxychlorure de phosphore. Le mélange est porté à reflux durant 7 heures puis concentré sous vide. Le résidu obtenu est dissous dans du dichlorométhane puis lavé par une solution aqueuse de carbonate de sodium. La phase organique est ensuite séchée puis évaporée. On récupère ainsi 1,8 g de n-Propyl-6 méthyl-2 chloro-4 [(cyano-3 thiényl-yl-2)-4 benzyl]-5 pyrimidine sous forme d'une huile utilisée telle quelle pour la suite des opérations.

Exemple 176 : **n-Propyl-6 méthyl-2 hydrazino-4 [(cyano-3 thiényl-yl-2)-4 benzyl]-5 pyrimidine**

**Formule (IX)** : $R_1$ = n-Propyl, $R_2$ = Méthyl,

V = 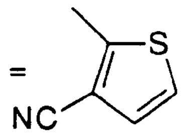

Préparé selon le mode opératoire de l'exemple 12.
Huile utilisée telle quelle pour la suite.

Exemple 177 : **n-Propyl-7 méthyl-5 [(cyano-3 thiényl-yl-2)-4 benzyl]-8 1,2,4-triazolo [4,3-c] pyrimidine one-3 (2H)**

**Formule (XII)** : $R_1$ = n-Propyl, $R_2$ = méthyl, X = CO,
Y = NH,

X $\overset{--}{—}$ Y = liaison simple, V = 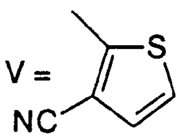

Préparé selon le mode opératoire de l'exemple 19.
Cristaux de point de fusion 170°C

Exemple 178 : **n-Propyl-7 méthyl-5 [[(tétrazolyl-5)-3 thiényl-yl-2]-4 benzyl]-8 1,2,4-triazolo [4,3-c] pyrimidine one-3 (2H)**

**Formule (I)** : $R_1$ = n-Propyl, $R_2$ = méthyl, X = CO,
Y = NH,

X $\overset{--}{—}$ Y = liaison simple, V = 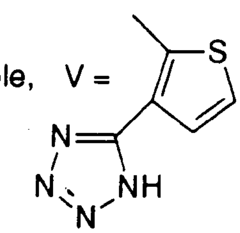

Préparé selon le mode opératoire de l'exemple 19.
Cristaux de point de fusion 240-242°C.

Exemple 179 : **n-Propyl-7 méthyl-5 (pyridyl-4)-2 [(cyano-2' biphényl-yl-4) méthyl]-8 1,2,4-triazolo [1,5-c] pyrimidine**

**Formule (XII)** : $R_1$ = n-Propyl, $R_2$ = méthyl, X = N,

Y = C 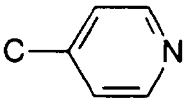 ,

X ⚋ Y = double liaison, V = 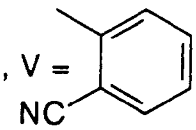

Préparé selon le mode opératoire de l'exemple 108 à partir du chlorure de l'acide pyridine-4 carboxylique. Cristaux de point de fusion 166°C.

Exemple 180 : **n-Propyl-7 hydroxyméthyl-5 [[(tétrazolyl-5)-2' biphényl-yl-4] méthyl]-8 1,2,4-triazolo [1,5-c] pyrimidine one-2 (3H)**

**Formule (I)** : $R_1$ = n-Propyl, $R_2$ = $CH_2OH$, Y = CO,
X = NH,

X ⚋ Y = liaison simple, $R_3$ = 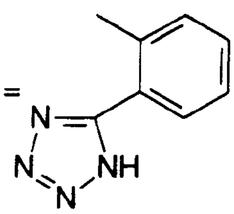

1 g de n-Propyl-7 méthoxyméthyl-5 [[(tétrazolyl-5)-2'biphényl-yl-4] méthyl]-8 1,2,4-triazolo [1,5-c] pyrimidine one-2 (3H) préparé à l'exemple 169, sont dissous dans 50 ml de chloroforme stabilisé à l'amylène. 0.7 ml de tribromure de bore sont ajoutés et le mélange est agité 8 heures à température ambiante, le dérivé n-Propyl-7 bromo méthyl-5 [[(tétrazolyl-5)-2' biphényl-yl-4] méthyl]-8 1,2,4-triazolo [1,5-c] pyrimidine one-2 (3H) ainsi formé est repris avec une solution de soude diluée et agité de nouveau 6 heures. La phase aqueuse est ensuite décantée et acidifiée par barbotage de dioxyde de soufre, les cristaux formés sont essorés et lavés à l'acétone puis séchés pour donner 0.6 g de n-Propyl-7 hydroxyméthyl-5 [[(tétrazolyl-5)-2' biphényl-yl-4] méthyl]-8 1,2,4-triazolo [1,5-c] pyrimidine one-2 (3H) sous forme de cristaux de point de fusion 182-183°C.

Exemple 181 : **n-Propyl-7 hydroxyméthyl-5 méthyl-2 [[(tétrazolyl-5)-2' biphényl-yl-4] méthyl]-8 1,2,4-triazolo [1,5-c] pyrimidine**

**Formule (I)** : $R_1$ = n-Propyl, $R_2$ = $CH_2OH$, Y = C-$CH_3$,
X = N,

X ⚋ Y = double liaison, $R_3$ = 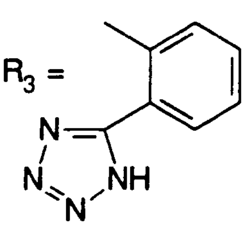

Préparé selon le mode opératoire de l'exemple 180 à partir du n-Propyl-7 méthoxyméthyl-5 méthyl-2 [[(tétrazolyl-5)-2' biphényl-yl-4] méthyl]-8 1,2,4-triazolo [1,5-c] pyrimidine préparé à l'exemple 171.

Cristaux de point de fusion 190-191°C.

Exemple 182 : **n-Propyl-7 hydroxyméthyl-2 méthyl-5 [[(tétrazolyl-5)-2' biphényl-yl-4] méthyl]-8 1,2,4-triazolo [1,5-c] pyrimidine**

**Formule (I)** : $R_1$ = n-Propyl, $R_2$ = $CH_3$, Y = C-$CH_2$-OH, X = N,

X ---- Y = double liaison, $R_3$ =

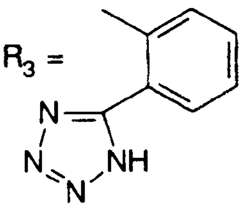

Préparé selon le mode opératoire de l'exemple 180 à partir du n-Propyl-7 méthoxyméthyl-2 méthyl-5 [[(tétrazolyl-5)-2' biphényl-yl-4] méthyl]-8 1,2,4-triazolo [1,5-c] pyrimidine préparé à l'exemple 152.

Cristaux de point de fusion 226-227°C.

Exemple 183 : **[(diméthyl-4,4-oxazoline-yl-2)-2' biphényl-yl-4] méthyl-2 oxo-3 hexanoate d'éthyle**

**Formule (IV)** : $R_1$ = n-Propyl, $R_6$ = Ethyl,

V =

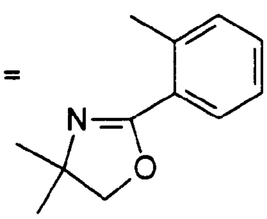

Préparé selon le mode opératoire de l'exemple 3 à partir du chlorhydrate de bromométhyl-4' (diméthyl-4,4-oxazoline-yl-2)-2 biphényl en utilisant un équivalent supplémentaire de N,N,N-diisopropylethylamine afin de libérer le chlorhydrate in situ.

Huile chromatographiée sur gel de silice dans l'éluant chloroforme / pentane 70/30 et utilisée telle quelle pour la suite.

Préparation du chlorhydrate de bromométhyl-4' (diméthyl-4,4-oxazoline-yl-2)-2 biphényl :

A/ Méthoxyméthyl-4 bromobenzène :

100 g de bromo-4 bromure de benzyle sont dissous dans 250 ml de méthanol. Une solution de méthylate de sodium, obtenue par dissolution de 10 g de sodium dans 500 ml de méthanol est ajoutée et le mélange est agité 3 heures à température ambiante. Le méthanol est évaporé et le résidu est repris à l'éther et lavé à l'eau, la phase éthérée est séchée sur sulfate de magnésium, et évaporée à sec, le résidu est distillé sous vide pour donner 74.3 g de méthoxyméthyl-4 bromobenzène sous forme d'une huile de point d'ébullition $Eb_{20}$ = 112-114°C.

B/ Chlorhydrate de méthoxyméthyl-4' (diméthyl-4,4-oxazoline-yl-2)-2 biphényl :

7.5 g de magnésium en copeaux sont mis en suspension dans 15 ml de tétrahydrofurane anhydre. Une solution de 49 g de méthoxyméthyl-4 bromobenzène , préparée en A), dans 50 ml de tétrahydrofurane anhydre, est ajoutée goutte à goutte de façon à maintenir la température en dessous de 40°C. Quand tout le magnésium a disparu, une solution de 28 g de (diméthyl-4,4-oxazoline-yl-2)-2 méthoxy benzène préparé selon la référence

MEYERS A.I.; MIHELICH E.D. ; J. Am. Chem. Soc., 1975, 97 (25), 7383, dans 100 ml de tétrahydrofurane anhydre est ajouté goutte à goutte en maintenant la température en dessous de 50°C. Le mélange est ensuite agité 2 heures à température ambiante et abandonné 48 heures. Le solvant est alors concentré de moitié sous vide et le résidu est versé sur 1.5 l d'une solution saturée de chlorure d'ammonium et extrait à l'éther, lavé à l'eau puis la phase organique est séchée sur sulfate de magnésium puis acidifiée par addition d'éther chlorhydrique. Le précipité gommeux orange qui se forme est décanté, puis repris à l'eau et cristallise, les cristaux sont lavés à l'eau puis à l'éther pour donner 26 g de chlorhydrate de méthoxyméthyl-4' (diméthyl-4,4-oxazoline-yl-2)-2 biphényl sous forme de cristaux de point de fusion 108-110°C.

C/ Chlorhydrate de bromométhyl-4' (diméthyl-4,4-oxazoline-yl-2)-2 biphényl

5 g de chlorhydrate de méthoxyméthyl-4' (diméthyl-4,4-oxazoline-yl-2)-2 biphényl préparés en B/, sont dissous dans 75 ml de chloroforme stabilisé à l'amylène et 3.2 ml de tribromure de bore sont ajoutés en refroidissant à 0°C. Le mélange est agité une heure à 0°C et lavé à l'eau froide. La phase organique est décantée, puis séchée sur sulfate de magnésium et évaporée sous vide pour donner 5.2 g de chlorhydrate de bromométhyl-4' (diméthyl-4,4-oxazoline-yl-2)-2 biphényl sous forme de cristaux de point de fusion 126-127°C.

Exemple 184 : **n-Propyl-6 méthyl-2 hydroxy-4 [[(diméthyl-4,4-oxazoline-yl-2)-2'biphényl-yl-4] méthyl]-5 pyrimidine**

**Formule (VII)** : $R_1$ = n-Propyl, $R_2$ = méthyl,

V = 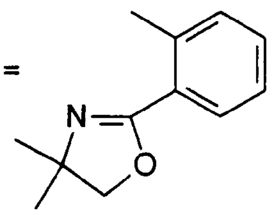

Préparé selon le mode opératoire de l'exemple 6.
Cristaux de point de fusion 126°C.

Exemple 185 : **n-Propyl-6 méthyl-2 chloro-4 [[(diméthyl-4,4-oxazoline-yl-2)-2'biphényl-yl-4] méthyl]-5 pyrimidine**

**Formule (VIII)** : $R_1$ = n-Propyl, $R_2$ = méthyl,

V = 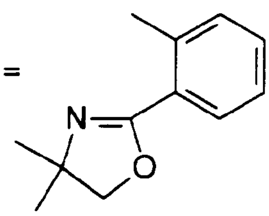

1.5 g de n-Propyl-6 méthyl-2 hydroxy-4 [[(diméthyl-4,4-oxazoline-yl-2)-2' biphényl-yl-4] méthyl]-5 pyrimidine préparé à l'exemple 184, sont dissous dans 3 ml de chlorure de thionyle. 0.1 ml de diméthyl formamide sont ajoutés et le mélange est agité 1 heure à température ambiante. Le chlorure de thionyle est évaporé sous vide sans chauffer, le résidu est lavé à l'éther puis alcalinisé par une solution d'hydroxyde d'ammonium et lavé à l'eau. La phase éthérée est évaporée sous vide pour donner 0.6 g de n-Propyl-6 méthyl-2 chloro-4 [[(diméthyl-4,4-oxazoline-yl-2)-2' biphényl-yl-4]-méthyl]-5 pyrimidine sous forme d'une huile utilisée telle quelle pour la suite.

Exemple 186 : **n-Propyl-6 méthyl-2 hydrazino-4 [[(diméthyl-4,4-oxazoline-yl-2)-2'biphényl-yl-4] méthyl]-5 pyrimidine**

**Formule (IX)** : $R_1$ = n-Propyl, $R_2$ = méthyl,

V = 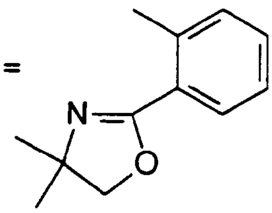

Préparé selon le mode opératoire de l'exemple 12.
Huile utilisée telle quelle pour la suite.

Exemple 187 : **n-Propyl-7 diméthyl-2,5 [[(diméthyl-4,4-oxazoline-yl-2)-2'biphényl-yl-4] méthyl]-8 1,2,4-triazolo [1,5-c] pyrimidine**

**Formule (I)** : $R_1$ = n-Propyl, $R_2$ = méthyl, X = N, Y = C-$CH_3$,

X ⎓ Y = double liaison, $R_3$ = 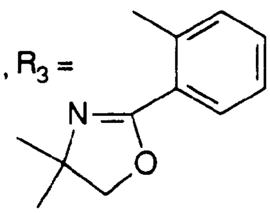

Préparé selon le mode opératoire de l'exemple 73.
Cristaux de point de fusion 135-136°C.

Exemple 188 : **n-Propyl-7 diméthyl-2,5 [(cyano-2' biphényl-yl-4) méthyl]-8 1,2,4-triazolo [1,5-c] pyrimidine**

**Formule (XII)** : $R_1$ = n-Propyl, $R_2$ = méthyl, X = N, Y = C-$CH_3$,

X ⎓ Y = double liaison, V = 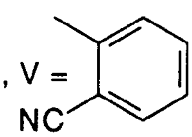

10 g de n-Propyl-7 diméthyl-2,5 [[(diméthyl-4,4-oxazoline-yl-2)-2' biphényl-yl-4] méthyl]-8 1,2,4-triazolo [1,5-c] pyrimidine préparé à l'exemple 187 sont dissous dans 50 ml de pyridine et 10 ml d'oxychlorure de phosphore sont additionnés goutte à goutte en maintenant la température en dessous de 15°C. Le mélange est ensuite chauffé 3 h à 100°C, puis évaporé sous vide et le résidu est jeté dans un mélange glace/eau et extrait au chloroforme. La phase organique est séchée sur sulfate de magnésium et évaporée sous vide pour donner 6 g de n-Propyl-7 diméthyl-2,5 [(cyano-2' biphényl-yl-4) méthyl]-8 1,2,4-triazolo [1,5-c] pyrimidine sous forme de cristaux de point de fusion 132°C. Ce composé est identique à celui de l'exemple 73.

Exemple 189 : **n-Propyl-6 méthyl-2 hydroxy-4 pyrimidine**

**Formule (XIII)** : $R_1$ = n-Propyl, $R_2$ = $CH_3$

Préparé selon le mode opératoire de l'exemple 6 en utilisant l'éthylbutyryl acétate et le chlorhydrate d'acétamidine dans l'éthanol en présence d'éthylate de sodium.

Cristaux de point de fusion : 95°C.

Exemple 190 : **n-Propyl-6 méthyl-2 chloro-4 pyrimidine**

Préparé selon le mode opératoire de l'exemple 9.
Cristaux de point de fusion : 55°C.

Exemple 191 : **n-Propyl-6 méthyl-2 hydrazino-4 pyrimidine**

Préparé selon le mode opératoire de l'exemple 12.
Cristaux de point de fusion : 101°C.

Exemple 192 : **n-Propyl-7 méthyl-5 1,2,4-triazolo [4,3-c] pyrimidine one-3 (2H)**

**Formule (XVII) :** $R_1$ = n-Propyl, $R_2$ = $CH_3$, Y = NH, X = CO,
X --- Y = liaison simple
Préparé selon le mode opératoire de l'exemple 15.
Cristaux de point de fusion: 145°C.

Exemple 193 : **n-Propyl-7 méthyl-5 1,2,4-triazolo [1,5-c] pyrimidine one-2 (3H)**

**Formule (XVII)** : $R_1$ = n-Propyl, $R_2$ = $CH_3$, Y = C = O, X = NH,
X --- Y = liaison simple

On chauffe au reflux durant 20 h une solution de 15,5 g de n-Propyl-7 méthyl-5 1,2,4-triazolo [4,3-c] pyrimidine one-3 (2H) préparés à l'exemple 192 dans 100 ml d'acide acétique. Le mélange réactionnel est ensuite évaporé sous vide et le résidu obtenu cristallise dans l'éther éthylique. Les cristaux essorés sont lavés à l'éther éthylique et séchés. On obtient 12 g de n-Propyl-7 méthyl-5 1,2,4-triazolo [1,5-c] pyrimidine one-2 (3H) sous forme de cristaux de point de fusion 173°C.

Exemple 194 : **n-Propyl-7 méthyl-5 bromo-8 1,2,4-triazolo [1,5-c] pyrimidine one-2 (3H)**

**Formule (XVI)** : $R_1$ = n-Propyl, $R_2$ = $CH_3$, Y = C = O, X = NH,
X --- Y = liaison simple

On agite à température ambiante durant 3 heures, une solution de 10 g de n-Propyl-7 méthyl-5 1,2,4-triazolo [1,5-c] pyrimidine one-2 (3H)-préparés à l'exemple 193 dans 120 ml d'acide acétique contenant 12 g d'acétate de sodium et à laquelle on a additionné goutte à goutte une solution de 2,6 ml de brome dans 50 ml d'acide acétique. Le mélange réactionnel est ensuite concentré sous vide puis additionné d'eau. Les cristaux formés sont essorés, lavés à l'eau et séchés. On obtient ainsi 7 g de n-Propyl-7 méthyl-5 bromo-8 1,2,4-triazolo [1,5-c] pyrimidine one-2 (3H) sous forme de cristaux de point de fusion 221°C.

Exemple 195 : **n-Proyl-7 méthyl-5 [(cyano-2' biphényl-yl-4) méthyl]-8 1,2,4-triazolo [1,5-c] pyrimidine one-2 (3H)**

**Formule (XII)** : $R_1$ = n-Propyl, $R_2$ = $CH_3$, Y = C = O,
X = NH,

X --- Y = liaison simple, V =

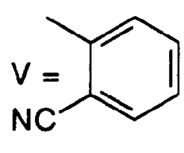

A une solution de 14 g de bromométhyl-4' cyano-2 biphényl dans 60 ml de tétrahydrofurane anhydre on ajoute à température ambiante 7,5 g de poudre de zinc activée. Le mélange est agité à température ambiante pendant 4 h. Une solution de 7 g de n-Propyl-7 méthyl-5 bromo-8 1,2,4-triazolo [1,5-c] pyrimidine one-2 (3H)

préparés à l'exemple 194 dans 40 ml de tétrahydrofurane anhydre est ensuite ajouté au mélange réactionnel puis on additionne une solution de 527 mg de tris (dibenzylidène acétone) dipalladium (o) et de 1032 g de trio-tolylphosphine dans 30 ml de tétrahydrofurane anhydre. Le mélange réactionnel est agité 1 h à température ambiante puis porté au reflux 3 h et agité encore 20 h à température ambiante. Le mélange réactionnel est ensuite additionné d'eau et extrait au chloroforme qu'on lave à l'eau, sèche et évapore.

Le résidu obtenu est chromatographié sur gel de silice avec comme éluant le mélange chloroforme/méthanol 9/1 pour conduire à 3.95 g de n-Propyl-7 méthyl-5 [(cyano-2' biphényl-yl-4) méthyl]-8 1,2,4-triazolo [1,5-c] pyrimidine one-2 (3H) sous forme de cristaux de point de fusion 215-216°C. Ce composé est identique à celui de l'exemple 18.

Exemple 196 : **n-Propyl-7 hydrazino-5 [[(tétrazolyl-5)-2' biphényl-yl-4 ] méthyl]-8 1,2,4 triazolo [1,5-c] pyrimidine-one-2 (3H)**

**Formule (I) :** $R_1$ = n-Propyl, $R_2$ = NH-$NH_2$, X = NH, Y = CO,

X --- Y = liaison simple, $R_3$ = 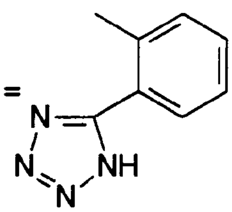

On porte au reflux durant 3 heures 30 une solution de 14,1 g de n-Propyl-7 méthylthio-5 [[(tétrazoly-5)-2' biphényl-yl-4] méthyl]-8 1,2,4 triazolo [4,3-c] pyrimidine-one-3 (2H) préparés à l'exemple 105 et de 30 ml d'hydrate d'hydrazine dans 100 ml de méthoxy-2 éthanol. Le mélange réactionnel est ensuite concentré sous vide, additionné d'eau et neutralisé par un barbotage de dioxyde de soufre ; les cristaux formés sont essorés, lavés à l'eau et séchés. On obtient 9 g de n-Propyl-7 hydrazino-5 [[(tétrazolyl-5)-2' biphényl-yl-4 ] méthyl]-8 1,2,4 triazolo [1,5-c] pyrimidine-one-2 (3H) sous forme de cristaux de point de fusion 287-288°C.

**PHARMACOLOGIE**

I. Principe

L'affinité des produits des exemples pour les récepteurs de l'angiotensine II est évaluée par technique de déplacement d'un radioligand spécifiquement fixé sur les récepteurs surrénaliens de l'angiotensine II, chez le rat.

II. Mode opératoire

Une aliquote d'un homogénat de surrénales de rat incube en présence d'une concentration unique de [ $^{125}$I ] - SIAII (Sar$^1$, Tyr$^4$, Ile$^8$ -angiotensine II), antagoniste des récepteurs de l'angiotensine II, et de deux concentrations d'agents compétiteurs ($10^{-5}$ M, $10^{-7}$ M), durant 60 min à 25°C.

La réaction est achevée par ajout de tampon, puis rapide filtration à travers des filtres de papier de verre. La liaison non spécifique est déterminée en présence d'angiotensine II.

III. Expression des résultats

Les résultats sont exprimés, pour les concentrations testées, en pourcentage de déplacement du radioligand spécifiquement fixé sur les récepteurs surrénaliens de l'angiotensine II.

IV. Résultats

| Produit de l'exemple | % de déplacement du ligand marqué | |
|---|---|---|
| | 1E-5M | 1E-7M |
| Exemple 19 | 66 | 48 |
| Exemple 20 | 60 | 45 |
| Exemple 25 | 68 | 54 |
| Exemple 36 | 65 | 43 |
| Exemple 42 | 75 | 46 |
| Exemple 68 | 67 | 33 |
| Exemple 71 | 73 | 60 |
| Exemple 74 | 69 | 54 |
| Exemple 78 | 67 | 52 |
| Exemple 80 | 74 | 59 |
| Exemple 90 | 63 | 48 |
| Exemple 97 | 60 | 38 |
| Exemple 107 | 71 | 58 |
| Exemple 109 | 67 | 46 |
| Exemple 112 | 60 | 41 |
| Exemple 138 | 61 | 17 |
| Exemple 146 | 74 | 56 |
| Exemple 152 | 69 | 57 |

**TOXICOLOGIE**

Les produits des exemples décrits présentent, après administration par voie orale, une excellente tolérance.

Leur dose létale 50 chez le rat a été évaluée supérieure à 300 mg/kg.

**CONCLUSION**

Les produits des exemples décrits présentent une bonne affinité pour les récepteurs à l'angiotensine II. A ce titre ils pourront être utilisés avec bénéfice dans les diverses pathologies où l'angiotensine II est impliquée, en particulier dans les traitements de l'hypertension artérielle, de l'insuffisance cardiaque, des maladies de la paroi artérielle, à des posologies de 1 à 400 mg par voie orale et 0.01 à 50 mg par voie intraveineuse, en une ou plusieurs prises par jour.

## Revendications

**Revendications pour les Etats contractants suivants : AT, BE, CH, DE, DK, FR, GB, IT, LI, LU, MC, NL, PT, SE**

1. Dérivés de triazolopyrimidine caractérisés en ce qu'ils répondent à la formule générale (I) :

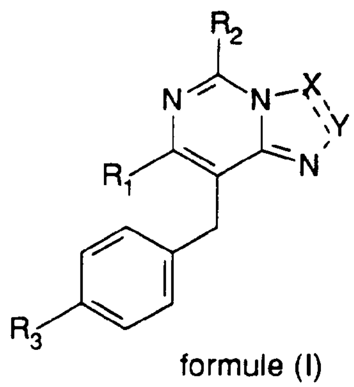

formule (I)

dans laquelle :

$R_1$ représente un radical alkyle inférieur de 1 à 6 atomes de carbone ou un radical cycloalkyle en $C_3$-$C_7$,

$R_2$ représente l'atome d'hydrogène, un radical alkyle inférieur de 1 à 6 atomes de carbone, un radical halogéno alkyle inférieur de 1 à 6 atomes de carbone, un groupement $NR_4R'_4$, un groupement $NH$-$NH_2$, un groupement $(CH_2)_m\ OR_4$, $(CH_2)_m\ SR_4$.

$R_4$ et $R'_4$ identiques ou différents représentent un atome d'hydrogène, un radical alkyle inférieur de 1 à 6 atomes de carbone et m représente un nombre entier de 0 à 5,

l'ensemble —X---Y— ou —Y---X— représente l'un des radicaux bivalents suivants :

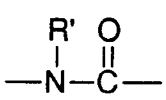 , 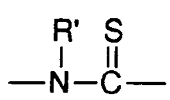 , 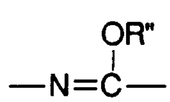 , 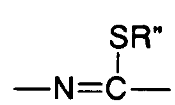 , 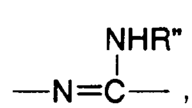 ,

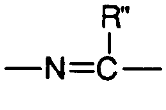 , 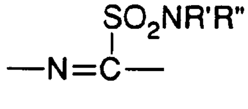

dans lesquels R' et R" identiques ou différents représentent :

un atome d'hydrogène, un radical alkyle inférieur de 1 à 6 atomes de carbone, un radical halogénoalkyle inférieur de 1 à 6 atomes de carbone, un groupement $(CH_2)_n\ COOR_5$, $(CH_2)_{n'}$-$OR_5$, $(CH_2)_{n'}O$-$CO$-$R_5$, $(CH_2)_{n'}SR_5$ ; n étant un nombre entier de 0 à 5, n' étant un nombre entier de 1 à 5 et $R_5$ étant un atome d'hydrogène ou radical alkyle inférieur de 1 à 6 atomes de carbone;

un radical phényle, pyridyle, thiényle ou furyle,

$R_3$ représente un groupement $NO_2$, $NH_2$ ou représente l'un des radicaux suivants :

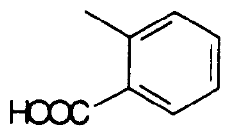 , 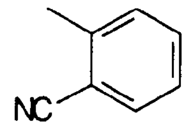 , 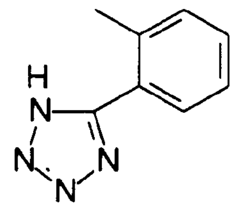 , 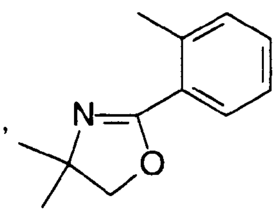 ,

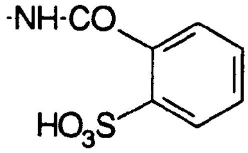 , 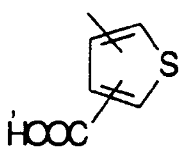 , 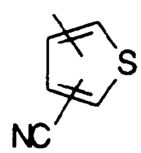 , 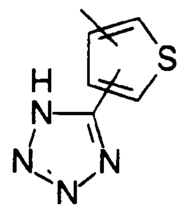

Les dérivés précités doivent être considérés également sous leur forme tautomères et peuvent se présenter sous la forme de sels d'addition, en particulier des sels d'addition pharmaceutiquement acceptables.

**2.** Dérivés de triazolo pyrimidine selon la revendication 1, caractérisés en ce que :

$R_1$ représente un radical alkyle inférieur de 1 à 6 atomes de carbone ou un radical cycloalkyle en $C_3$-$C_7$ ;

$R_2$ représente l'atome d'hydrogène, un radical alkyle inférieur de 1 à 6 atomes de carbone, un groupement NH-$NH_2$, un groupement $(CH_2)_mOR_4$, $(CH_2)_m$ $SR_4$ ; $R_4$ représentant un atome d'hydrogène, un radical alkyle inférieur de 1 à 6 atomes de carbone et m représentant un nombre entier de 0 à 2;

L'ensemble —X----Y— représente un radical choisi parmi les radicaux bivalents suivants :

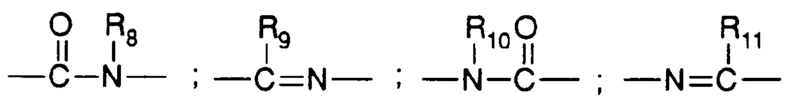

dans lesquels

$R_8$ représente un radical choisi dans le groupe constitué par l'atome d'hydrogène, -$(CH_2)_nOH$, -$(CH_2)_nCOOH$, -$R_{12}$, -$(CH_2)_nCOOR_{12}$ ; $R_{12}$ représentant un radical alkyle inférieur de 1 à 6 atomes de carbone et n un nombre entier égal à 1 ou 2 ;

$R_9$ représente l'atome d'hydrogène ou un radical -SH ;

$R_{10}$ représente l'atome d'hydrogène ou un radical alkyle inférieur de 1 à 6 atomes de carbone ;

$R_{11}$ représente un radical choisi dans le groupe constitué par l'atome d'hydrogène, un radical alkyle inférieur de 1 à 6 atomes de carbone, un radical halogénoalkyle inférieur de 1 à 6 atomes de carbone, phényle, pyridinyle, -O $(CH_2)_nOH$, -$OR_{12}$, -O $(CH_2)_n$ $OCOR_{12}$, SH, -$SR_{12}$, -$S(CH_2)_nCOOR_{12}$, -$S(CH_2)_n$ $OCOR_{12}$, -NH $(CH_2)_n$ $COOR_{12}$, -$NR_{13}R_{14}$, $SO_2NR_{13}R_{14}$, $(CH_2)_nOH$, $(CH_2)_n$ $OR_{12}$, COOH, $COOR_{12}$, $(CH_2)_nCOOH$, $(CH_2)_nCOOR_{12}$ ; n et $R_{12}$ étant définis comme indiqué précédemment, $R_{13}$ et $R_{14}$ identiques ou différents représentant l'atome d'hydrogène ou un radical alkyle inférieur ayant de 1 à 6 atomes de carbone ;

$R_3$ représente l'un des radicaux suivants :

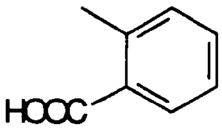 , 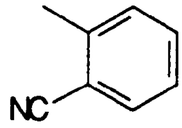 , 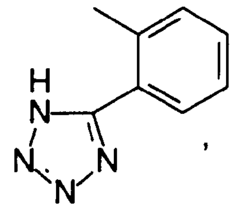 , 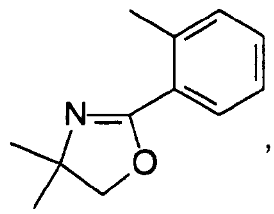 ,

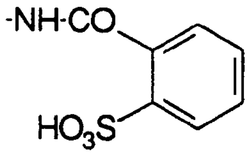 , 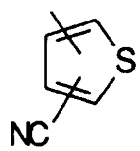 , 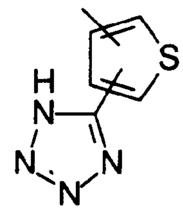

3. Dérivés selon la revendication 1 ou 2, caractérisés en ce que $R_1$ est un groupement choisi parmi éthyl, n-propyl et n-butyl.

4. Dérivés selon l'une quelconque des revendications 1 à 3, caractérisés en ce que $R_2$ est un groupement choisi parmi méthyl, éthyl ou méthoxy méthyl.

5. Dérivés selon l'une quelconque des revendications 1 à 4, caractérisés en ce que l'ensemble —X--Y— représente l'un des radicaux bivalents suivants :

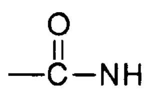 , 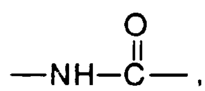 , 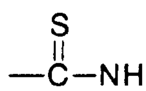 ,

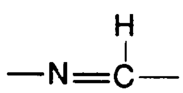 , 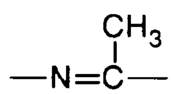 , 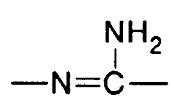 , 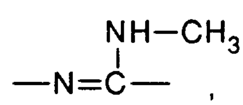 ,

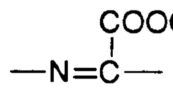 ou 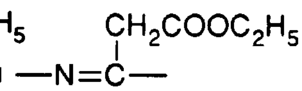

ou leur forme tautomère.

6. Dérivés selon l'une quelconque des revendications 1 à 5 caractérisés en ce que $R_3$ est un groupement (tétrazolyl-5)-2 phényl.

7. Dérivé selon la revendication 1 ou 2 caractérisé en ce qu'il s'agit du dérivé :
- n-Propyl-7 méthyl-5 [[(tétrazolyl-5)-2' biphényl-yl-4] méthyl]-8 1,2,4-triazolo [1,5-c] pyrimidine one-2 (3H)

8. Dérivés selon la revendication 1 ou 2 caractérisés en ce qu'ils sont choisis parmi les dérivés :
   - n-Propyl-7 méthyl-5 [[(tétrazolyl-5)-2' biphényl-yl-4] méthyl]-8 1,2,4-triazolo [4,3-c] pyrimidine one-3 (2H)
   - n-Propyl-7 méthyl-5 mercapto-3 [[(tétrazolyl-5)-2' biphényl-yl-4] méthyl]-8 1,2,4-triazolo [4,3-c] pyrimidine
   - n-Propyl-7 diméthyl-2,5 [[(tétrazolyl-5)-2' biphényl-yl-4] méthyl]-8 1,2,4-triazolo [1,5-c] pyrimidine
   - n-Propyl-7 méthyl-5 [[(tétrazolyl-5)-2' biphényl-yl-4] méthyl]-8 1,2,4-triazolo [1,5-c] pyrimidine
   - n-Propyl-7 méthyl-5 amino-2 [[(tétrazolyl-5)-2' biphényl-yl-4] méthyl]-8 1,2,4-triazolo [1,5-c] pyrimidine
   - n-Propyl-7 méthyl-5 méthylamino-2 [[(tétrazolyl-5)-2' biphényl-yl-4] méthyl]-8 1,2,4-triazolo [1,5-c] pyrimidine

- [n-Propyl-7 méthyl-5 [[(tétrazolyl-5)-2' biphényl-yl-4] méthyl]-8 1,2,4-triazolo [1,5-c] pyrimidine-yl-2] carboxylate d'éthyle
- [n-Propyl-7 méthyl-5 [[(tétrazolyl-5)-2' biphényl-yl-4] méthyl]-8 1,2,4-triazolo [1,5-c] pyrimidine-yl-2] acétate d'éthyle
- Ethyl-7 diméthyl-2,5 [[(tétrazolyl-5)-2' biphényl-yl-4] méthyl]-8 1,2,4-triazolo [1,5-c] pyrimidine
- n-Butyl-7 méthyl-5 [[(tétrazolyl-5)-2' biphényl-yl-4] méthyl]-8 1,2,4-triazolo [1,5-c] pyrimidine one-2 (3H)
- n-Propyl-7 éthyl-5 [[(tétrazolyl-5)-2' biphényl-yl-4] méthyl]-8 1,2,4-triazolo [1,5-c] pyrimidine one-2 (3H)
- n-Propyl-7 méthoxyméthyl-5 [[(tétrazolyl-5)-2' biphényl-yl-4] méthyl]-8 1,2,4-triazolo [1,5-c] pyrimidine one-2 (3H)

**9.** Procédé de préparation des composés de formule (I) selon l'une quelconque des revendications 1 à 8, caractérisé en ce qu'il comprend la préparation d'un composé intermédiaire de formule (X) ou (X") :

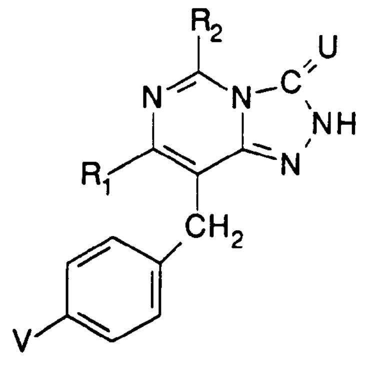

Formule (X)

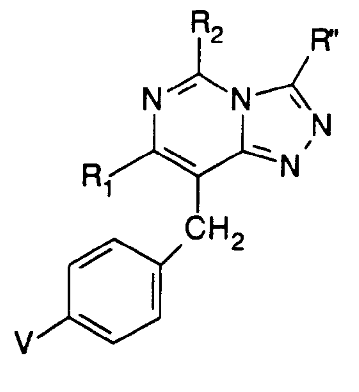

Formule (X")

dans lesquelles $R_1$, $R_2$ sont tels que définis à la revendication 1 ; V représente un groupe choisi parmi les radicaux $NO_2$,

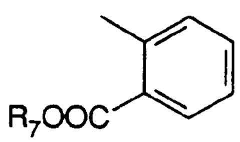
,
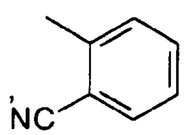
,
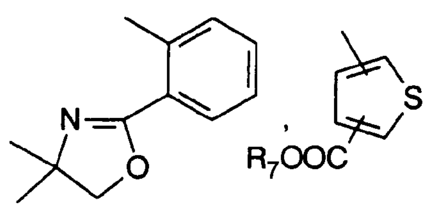
,
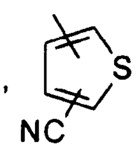

$R_7$ représentant un radical alkyle inférieur ou un benzyle ; U représente l'atome d'oxygène ou de soufre ou un groupement N-R", R" est tel que défini à la revendication 1 ; par cyclisation des composés de formule (IX) :

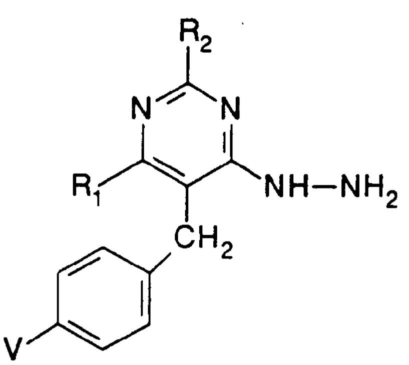

dans laquelle $R_1$, $R_2$ et V sont définis comme ci-dessus ; ladite cyclisation étant réalisée par l'action sur les composés de formule (IX) :
soit du carbonyl diimidazole au reflux du tétrahydrofurane ;
soit de l'urée par chauffage sans solvant ou dans un solvant comme la N-méthyl pyrrolidone ;
soit du xanthogénate de potassium au reflux d'un alcool comme le méthoxy éthanol ;
soit du sulfure de carbone dans un alcool en présence ou non d'une amine comme la triéthylamine ;
soit d'un isocyanate suivie d'un traitement au $POCl_3$ ;
soit d'un isothiocyanate suivie d'une méthylation en $SCH_3$ par l'iodure de méthyle puis cyclisation thermique ;
soit d'un ortho ester par chauffage ;
soit encore d'un chlorure d'acide suivie d'une cyclisation à l'aide de $POCl_3$.

**10.** Procédé de préparation selon la revendication 9, caractérisé en ce qu'il comprend la préparation d'un composé intermédiaire de formule (XI) ou (XI") :

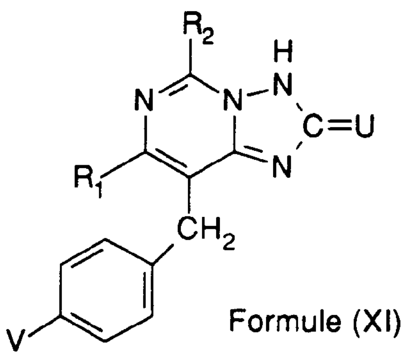
Formule (XI)

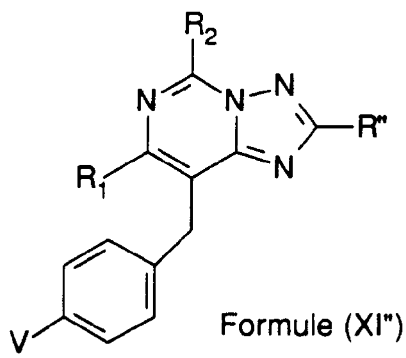
Formule (XI")

dans lesquelles $R_1$, $R_2$, V, U et R" sont définis comme ci-dessus, par isomérisation des composés précités de formule (X) ou (X") en milieu basique dans l'eau ou dans un mélange eau-alcool à une température comprise entre 20 et 100°C; cette isomérisation pouvant être encore réalisée en milieu acide par chauffage dans l'acide acétique en présence ou non d'acétate de sodium ou de potassium ou encore dans le dichloro benzène en présence d'acide formique.

**11.** Procédés de préparation des composés de formule (I) dans laquelle $R_3$ représente un groupement

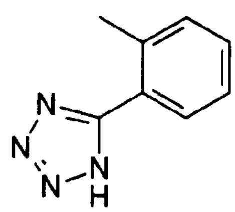

caractérisés en ce qu'on fait réagir un composé de formule :

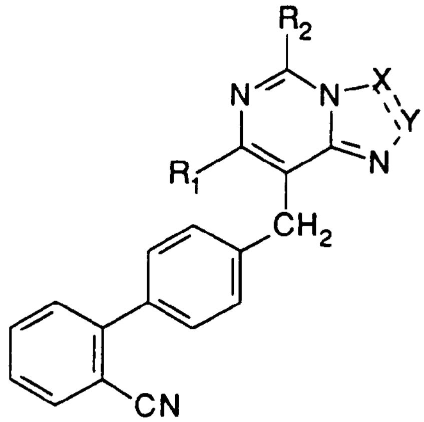

dans laquelle $R_1$, $R_2$, X et Y sont définis comme ci-dessus avec l'azoture de sodium dans le diméthyl formamide en présence d'un sel d'ammonium comme le chlorure d'ammonium à une température comprise entre 100 et 150°C ou par chauffage dans le toluène ou le xylène avec l'azoture de triméthyl étain suivi d'un traitement par le gaz chlorhydrique dans le tétrahydrofurane par exemple.

**12.** Composition pharmaceutique, caractérisée en ce qu'elle comprend une quantité pharmaceutiquement ef-

ou un de ses sels d'addition pharmaceutiquement acceptables, éventuellement incorporé dans un excipient, véhicule ou support pharmaceutiquement acceptable.

**13.** Composition pharmaceutique à activité antagoniste des récepteurs à l'angiotensine II permettant de traiter

dans laquelle :

$R_1$ représente un radical alkyle inférieur de 1 à 6 atomes de carbone ou un radical cycloalkyle en $C_3$-$C_7$,

$R_2$ représente l'atome d'hydrogène, un radical alkyle inférieur de 1 à 6 atomes de carbone, un radical halogéno alkyle inférieur de 1 à 6 atomes de carbone, un groupement $NR_4R'_4$, un groupement NH-$NH_2$, un groupement $(CH_2)_m$ $OR_4$, $(CH_2)_m$ $SR_4$.

$R_4$ et $R'_4$ identiques ou différents représentent un atome d'hydrogène, un radical alkyle inférieur de 1 à 6 atomes de carbone et m représente un nombre entier de 0 à 5,

l'ensemble —X---Y— ou —Y---X— représente l'un des radicaux bivalents suivants :

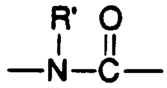 , 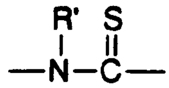 , 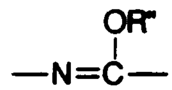 , 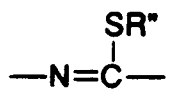 , 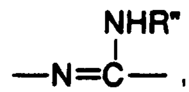 ,

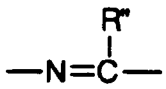 , 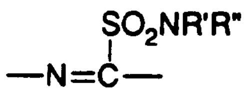

dans lesquels R' et R" identiques ou différents représentent :

un atome d'hydrogène, un radical alkyle inférieur de 1 à 6 atomes de carbone, un radical halogénoalkyle inférieur de 1 à 6 atomes de carbone, un groupement $(CH_2)_n$ $COOR_5$, $(CH_2)_{n'}$-$OR_5$, $(CH_2)_{n'}$O-CO-$R_5$, $(CH_2)_{n'}SR_5$ ; n étant un nombre entier de 0 à 5, n' étant un nombre entier de 1 à 5 et $R_5$ étant un atome d'hydrogène ou radical alkyle inférieur de 1 à 6 atomes de carbone ;

un radical phényle, pyridyle, thiényle ou furyle,

$R_3$ représente un groupement $NO_2$, $NH_2$ ou représente l'un des radicaux suivants :

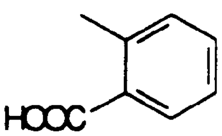 , 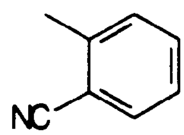 , 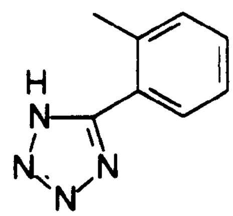 , 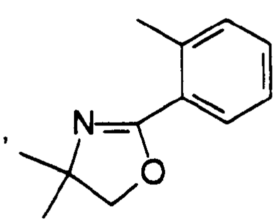 ,

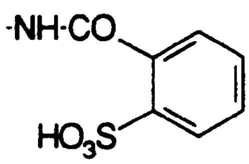 , 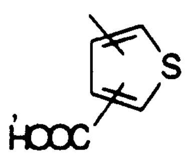 , 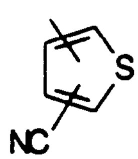 , 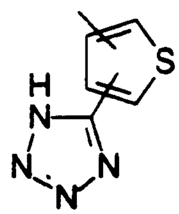 ,

les dérivés précités devant être considérés également sous leur forme tautomères et pouvant se présenter sous la forme de sels d'addition, en particulier des sels d'addition pharmaceutiquement acceptables, caractérisé en ce qu'il comprend la préparation d'un composé intermédiaire de formule (X) ou (X") :

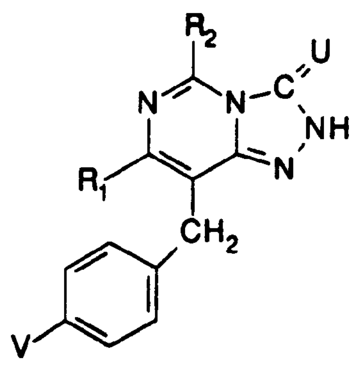

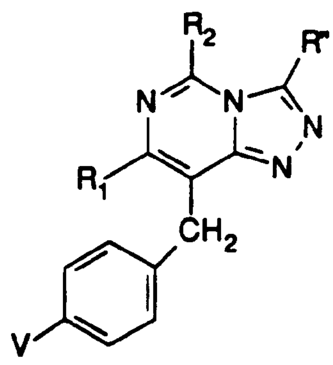

**Formule (X)** **Formule (X")**

dans lesquelles $R_1$, $R_2$ sont tels que définis précédemment ; V représente un groupe choisi parmi les radicaux $NO_2$,

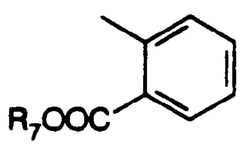 , 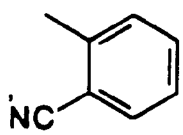 , 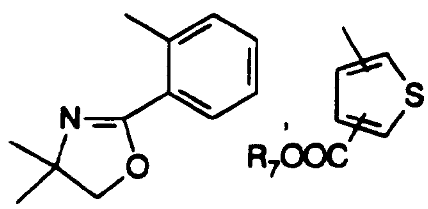 , 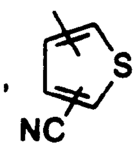

$R_7$ représentant un radical alkyle inférieur ou un benzyle ; U représente l'atome d'oxygène ou de soufre ou un groupement N-R", R" est tel que défini précédemment ; par cyclisation des composés de formule (IX) :

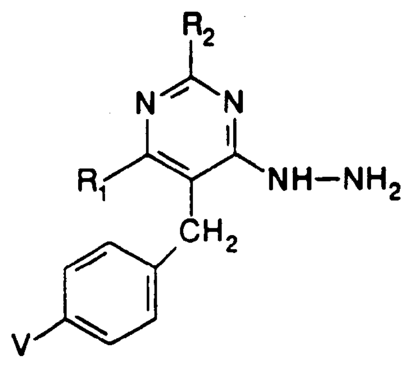

dans laquelle $R_1$, $R_2$ et V sont définis comme ci-dessus ; ladite cyclisation étant réalisée par l'action sur les composés de formule (IX) :
soit du carbonyl diimidazole au reflux du tétrahydrofurane ;
soit de l'urée par chauffage sans solvant ou dans un solvant comme la N-méthyl pyrrolidone ;
soit du xanthogénate de potassium au reflux d'un alcool comme le méthoxy éthanol ;
soit du sulfure de carbone dans un alcool en présence ou non d'une amine comme la triéthylamine ;
soit d'un isocyanate suivie d'un traitement au $POCl_3$ ;
soit d'un isothiocyanate suivie d'une méthylation en $SCH_3$ par l'iodure de méthyle puis cyclisation thermique ;
soit d'un ortho ester par chauffage ;
soit encore d'un chlorure d'acide suivie d'une cyclisation à l'aide de $POCl_3$.

2. Procédé de préparation selon la revendication 1, caractérisé en ce qu'il comprend la préparation d'un composé intermédiaire de formule (XI) ou (XI") :

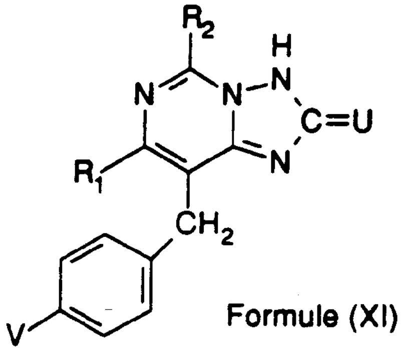

Formule (XI)

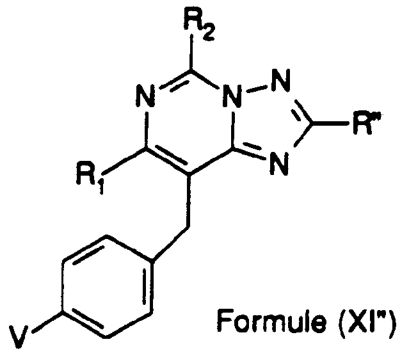

Formule (XI")

dans lesquelles $R_1$, $R_2$, V, U et R" sont définis comme ci-dessus, par isomérisation des composés précités de formule (X) ou (X") en milieu basique dans l'eau ou dans un mélange eau-alcool à une température comprise entre 20 et 100°C ; cette isomérisation pouvant être encore réalisée en milieu acide par chauffage dans l'acide acétique en présence ou non d'acétate de sodium ou de potassium ou encore dans le dichloro benzène en présence d'acide formique.

3. Procédé de préparation selon la revendication 1 des composés de formule (I) dans laquelle $R_3$ représente un groupement :

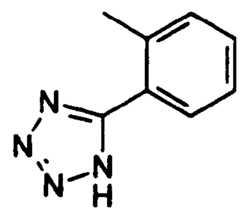

caractérisé en ce qu'on fait réagir un composé de formule :

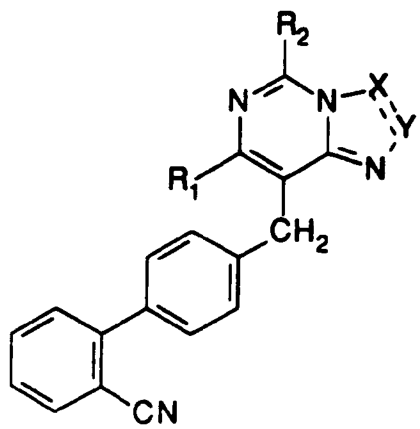

dans laquelle $R_1$, $R_2$, X et Y sont définis comme ci-dessus avec l'azoture de sodium dans le diméthyl formamide en présence d'un sel d'ammonium comme le chlorure d'ammonium à une température comprise entre 100 et 150°C ou par chauffage dans le toluène ou le xylène avec l'azoture de triméthyl étain suivi d'un traitement par le gaz chlorhydrique dans le tétrahydrofurane par exemple.

4. Procédé selon l'une des revendications 1 à 3, pour la préparation des composés de formule (I) dans laquelle :

$R_1$ représente un radical alkyle inférieur de 1 à 6 atomes de carbone ou un radical cycloalkyle en $C_3$-$C_7$ ;

$R_2$ représente l'atome d'hydrogène, un radical alkyle inférieur de 1 à 6 atomes de carbone, un groupement NH-$NH_2$, un groupement $(CH_2)_mOR_4$, $(CH_2)_m$ $SR_4$ ; $R_4$ représentant un atome d'hydrogène, un radical alkyle inférieur de 1 à 6 atomes de carbone et m représentant un nombre entier de 0 à 2 ;
L'ensemble —X----Y— représente un radical choisi parmi les radicaux bivalents suivants :

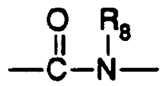 ; 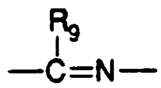 ; 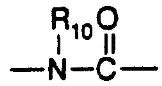 ; 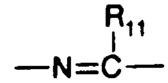

dans lesquels
$R_8$ représente un radical choisi dans le groupe constitué par l'atome d'hydrogène, $-(CH_2)_nOH$, $-(CH_2)_nCOOH$, $-R_{12}$, $-(CH_2)_nCOOR_{12}$ ; $R_{12}$ représentant un radical alkyle inférieur de 1 à 6 atomes de carbone et n un nombre entier égal à 1 ou 2 ;
$R_9$ représente l'atome d'hydrogène ou un radical -SH ;
$R_{10}$ représente l'atome d'hydrogène ou un radical alkyle inférieur de 1 à 6 atomes de carbone ;
$R_{11}$ représente un radical choisi dans le groupe constitué par l'atome d'hydrogène, un radical alkyle inférieur de 1 à 6 atomes de carbone, un radical halogénoalkyle inférieur de 1 à 6 atomes de carbone, phényle, pyridinyle, $-O(CH_2)_nOH$, $-OR_{12}$, $-O(CH_2)_n\ OCOR_{12}$, SH, $-SR_{12}$, $-S(CH_2)_nCOOR_{12}$, $-S(CH_2)_n\ OCOR_{12}$, $-NH(CH_2)_n\ COOR_{12}$, $-NR_{13}R_{14}$, $SO_2NR_{13}R_{14}$, $(CH_2)_nOH$, $(CH_2)_n\ OR_{12}$, COOH, $COOR_{12}$, $(CH_2)_nCOOH$, $(CH_2)_nCOOR_{12}$ ; n et $R_{12}$ étant définis comme indiqué précédemment, $R_{13}$ et $R_{14}$ identiques ou différents représentant l'atome d'hydrogène ou un radical alkyle inférieur ayant de 1 à 6 atomes de carbone ;
$R_3$ représente l'un des radicaux suivants :

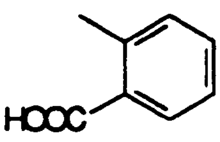 , 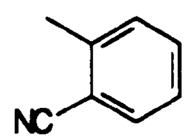 , 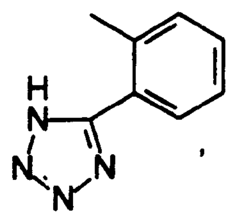 , 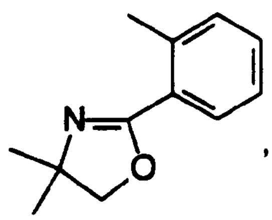 ,

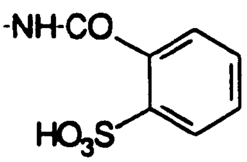 , 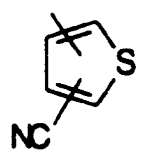 , 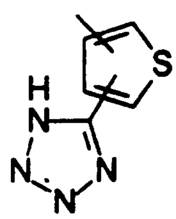

5. Procédé selon l'une des revendications 1 à 3, caractérisé en ce que $R_1$ est un groupement choisi parmi éthyl, n-propyl et n-butyl.

6. Procédé selon l'une des revendications 1 à 3, caractérisé en ce que $R_2$ est un groupement choisi parmi méthyl, éthyl ou méthoxy méthyl.

7. Procédé selon l'une des revendications 1 à 3, caractérisé en ce que l'ensemble —X--Y— représente l'un des radicaux bivalents suivants :

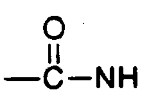 , 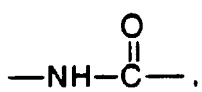 , 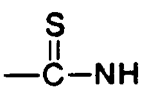 ,

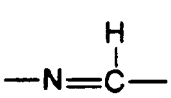 , 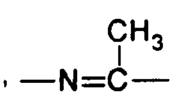 , 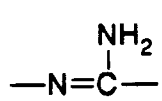 , 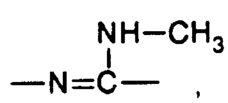 ,

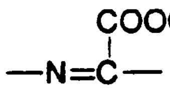 ou 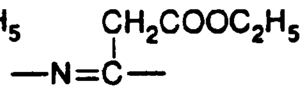

ou leur forme tautomère.

**8.** Procédé selon l'une des revendications 1 à 3, caractérisé en ce que $R_3$ est un groupement (tétrazolyl-5)-2 phényl.

**9.** Procédé selon l'une des revendications 1 à 3, pour la préparation du dérivé :
- n-Propyl-7 méthyl-5 [[(tétrazolyl-5)-2' biphényl-yl-4] méthyl]-8 1,2,4-triazolo [1,5-c] pyrimidine one-2 (3H)

**10.** Procédé selon l'une des revendications 1 à 3, pour la préparation des dérivés :
- n-Propyl-7 méthyl-5 [[(tétrazolyl-5)-2' biphényl-yl-4] methyl]-8 1,2,4-triazolo [4,3-c] pyrimidine one-3 (2H)
- n-Propyl-7 méthyl-5 mercapto-3 [[(tétrazolyl-5)-2' biphényl-yl-4] méthyl]-8 1,2,4-triazolo [4,3-c] pyrimidine
- n-Propyl-7 diméthyl-2,5 [[(tétrazolyl-5)-2' biphényl-yl-4] méthyl]-8 1,2,4-triazolo [1,5-c] pyrimidine
- n-Propyl-7 méthyl-5 [[(tétrazolyl-5)-2' biphényl-yl-4] méthyl]-8 1,2,4-triazolo [1,5-c] pyrimidine
- n-Propyl-7 méthyl-5 amino-2 [[(tétrazolyl-5)-2' biphényl-yl-4] méthyl]-8 1,2,4-triazolo [1,5-c] pyrimidine
- n-Propyl-7 méthyl-5 méthylamino-2 [[(tétrazolyl-5)-2' biphényl-yl-4] méthyl]-8 1,2,4-triazolo [1,5-c] pyrimidine
- [n-Propyl-7 méthyl-5 [[(tétrazolyl-5)-2' biphényl-yl-4] méthyl]-8 1,2,4-triazolo [1,5-c] pyrimidine-yl-2] carboxylate d'éthyle
- [n-Propyl-7 méthyl-5 [[(tétrazolyl-5)-2' biphényl-yl-4] méthyl]-8 1,2,4-triazolo [1,5-c] pyrimidine-yl-2] acétate d'éthyle
- Ethyl-7 diméthyl-2,5 [[(tétrazolyl-5)-2' biphényl-yl-4] méthyl]-8 1,2,4-triazolo [1,5-c] pyrimidine
- n-Butyl-7 méthyl-5 [[(tétrazolyl-5)-2' biphényl-yl-4] méthyl]-8 1,2,4-triazolo [1,5-c] pyrimidine one-2 (3H)
- n-Propyl-7 éthyl-5 [[(tétrazolyl-5)-2' biphényl-yl-4] méthyl]-8 1,2,4-triazolo [1,5-c] pyrimidine one-2 (3H)
- n-Propyl-7 méthoxyméthyl-5 [[(tétrazolyl-5)-2' biphényl-yl-4] méthyl]-8 1,2,4-triazolo [1,5-c] pyrimidine one-2 (3H)

**11.** Procédé de préparation d'une composition pharmaceutique, caractérisé en ce qu'on incorpore une quantité pharmaceutiquement efficace d'au moins un composé de formule (I) obtenu selon l'une quelconque des revendications 1 à 9, dans un excipient, véhicule ou support pharmaceutiquement acceptable.

**12.** Procédé selon la revendication 10, caractérisé en ce que la composition pharmaceutique est formulée sous forme de gélules, de comprimés dosés de 1 à 400 mg ou sous forme de préparations injectables dosées de 0,01 à 50 mg.

**Revendications pour l'Etat contractant suivant : GR**

**1.** Dérivés de triazolopyrimidine caractérisés en ce qu'ils répondent à la formule générale (I) :

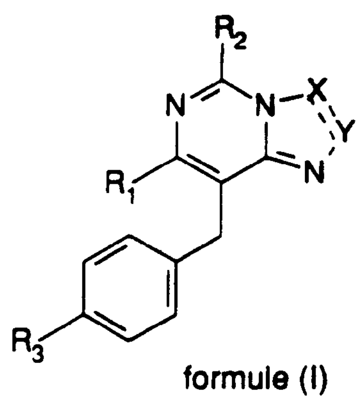

formule (I)

dans laquelle :

$R_1$ représente un radical alkyle inférieur de 1 à 6 atomes de carbone ou un radical cycloalkyle en $C_3$-$C_7$,

$R_2$ représente l'atome d'hydrogène, un radical alkyle inférieur de 1 à 6 atomes de carbone, un radical halogéno alkyle inférieur de 1 à 6 atomes de carbone, un groupement $NR_4R'_4$, un groupement NH-$NH_2$, un groupement $(CH_2)_m$ $OR_4$, $(CH_2)_m$ $SR_4$.

$R_4$ et $R'_4$ identiques ou différents représentent un atome d'hydrogène, un radical alkyle inférieur de 1 à 6 atomes de carbone et m représente un nombre entier de 0 à 5,

l'ensemble —X---Y— ou —Y---X— représente l'un des radicaux bivalents suivants :

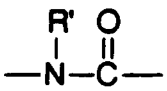 , 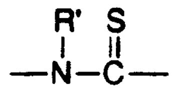 , 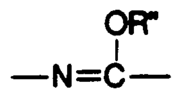 , 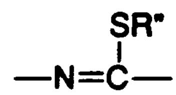 , 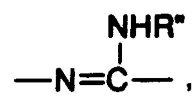 ,

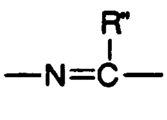 , 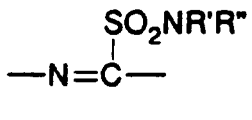

dans lesquels R' et R" identiques ou différents représentent :

un atome d'hydrogène, un radical alkyle inférieur de 1 à 6 atomes de carbone, un radical halogénoalkyle inférieur de 1 à 6 atomes de carbone, un groupement $(CH_2)_n$ $COOR_5$, $(CH_2)_{n'}$-$OR_5$, $(CH_2)_n$ O-CO-$R_5$, $(CH_2)_{n'}SR_5$ ; n étant un nombre entier de 0 à 5, n' étant un nombre entier de 1 à 5 et $R_5$ étant un atome d'hydrogène ou radical alkyle inférieur de 1 à 6 atomes de carbone ;

un radical phényle, pyridyle, thiényle ou furyle,

$R_3$ représente un groupement $NO_2$, $NH_2$ ou représente l'un des radicaux suivants :

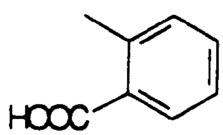 , 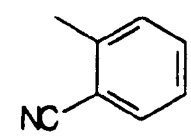 , 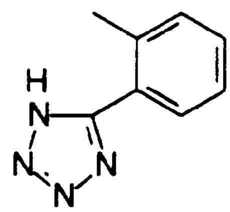 , 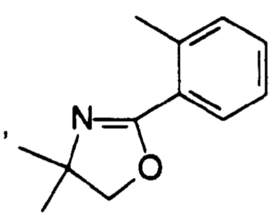 ,

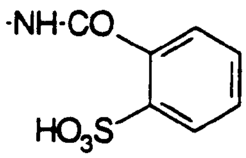 , 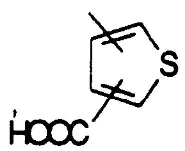 , 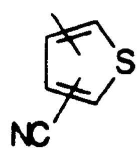 , 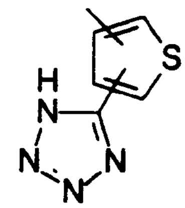

Les dérivés précités doivent être considérés également sous leur forme tautomères et peuvent se présenter sous la forme de sels d'addition, en particulier des sels d'addition pharmaceutiquement acceptables.

**2.** Dérivés de triazolo pyrimidine selon la revendication 1, caractérisés en ce que:

$R_1$ représente un radical alkyle inférieur de 1 à 6 atomes de carbone ou un radical cycloalkyle en $C_3$-$C_7$ ;

$R_2$ représente l'atome d'hydrogène, un radical alkyle inférieur de 1 à 6 atomes de carbone, un groupement NH-$NH_2$, un groupement $(CH_2)_mOR_4$, $(CH_2)_m$ $SR_4$ ; $R_4$ représentant un atome d'hydrogène, un radical alkyle inférieur de 1 à 6 atomes de carbone et m représentant un nombre entier de 0 à 2 ;

L'ensemble —X----Y— représente un radical choisi parmi les radicaux bivalents suivants :

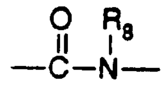 ; 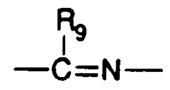 ; 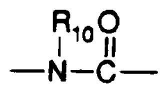 ; 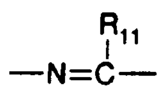

dans lesquels

$R_8$ représente un radical choisi dans le groupe constitué par l'atome d'hydrogène, $(CH_2)_nOH$, $(CH_2)_nCOOH$, $-R_{12}$, $-(CH_2)_nCOOR_{12}$ ; $R_{12}$ représentant un radical alkyle inférieur de 1 à 6 atomes de carbone et n un nombre entier égal à 1 ou 2 ;

$R_9$ représente l'atome d'hydrogène ou un radical -SH ;

$R_{10}$ représente l'atome d'hydrogène ou un radical alkyle inférieur de 1 à 6 atomes de carbone ;

$R_{11}$ représente un radical choisi dans le groupe constitué par l'atome d'hydrogène, un radical alkyle inférieur de 1 à 6 atomes de carbone, un radical halogénoalkyle inférieur de 1 à 6 atomes de carbone, phényle, pyridinyle, $-O(CH_2)_nOH$, $-OR_{12}$, $-O(CH_2)_nOCOR_{12}$, SH, $-SR_{12}$, $-S(CH_2)_nCOOR_{12}$, $-S(CH_2)_nOCOR_{12}$, $-NH(CH_2)_nCOOR_{12}$, $-NR_{13}R_{14}$, $SO_2NR_{13}R_{14}$, $(CH_2)_nOH$, $(CH_2)_nOR_{12}$, COOH, $COOR_{12}$, $(CH_2)_nCOOH$, $(CH_2)_nCOOR_{12}$ ; n et $R_{12}$ étant définis comme indiqué précédemment, $R_{13}$ et $R_{14}$ identiques ou différents représentant l'atome d'hydrogène ou un radical alkyle inférieur ayant de 1 à 6 atomes de carbone ;

$R_3$ représente l'un des radicaux suivants :

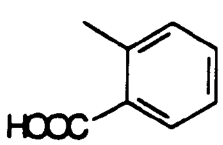 , 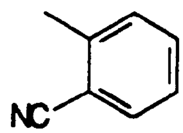 , 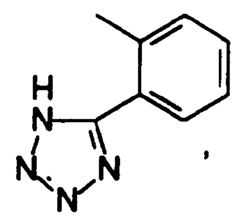 , 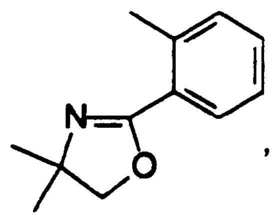 ,

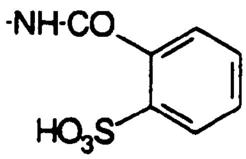 , 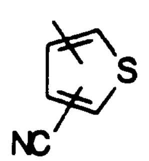 , 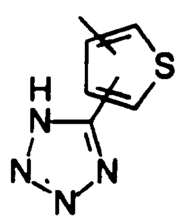

3. Dérivés selon la revendication 1 ou 2, caractérisés en ce que $R_1$ est un groupement choisi parmi éthyl, n-propyl et n-butyl.

4. Dérivés selon l'une quelconque des revendications 1 à 3, caractérisés en ce que $R_2$ est un groupement choisi parmi méthyl, éthyl ou méthoxy méthyl.

5. Dérivés selon l'une quelconque des revendications 1 à 4, caractérisés en ce que l'ensemble —X--Y— représente l'un des radicaux bivalents suivants :

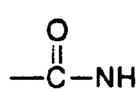 , 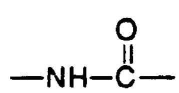 , 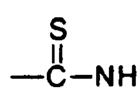 ,

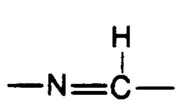 , 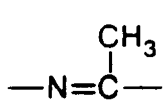 , 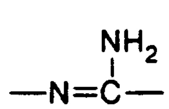 , 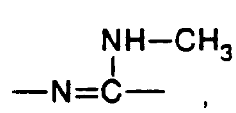 ,

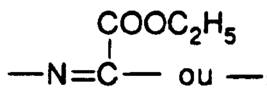 ou 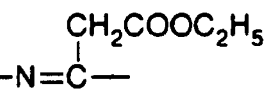

ou leur forme tautomère.

6. Dérivés selon l'une quelconque des revendications 1 à 5 caractérisés en ce que $R_3$ est un groupement (tétrazolyl-5)-2 phényl.

7. Dérivé selon la revendication 1 ou 2 caractérisé en ce qu'il s'agit du dérivé :
   - n-Propyl-7 méthyl-5 [[(tétrazolyl-5)-2' biphényl-yl-4] méthyl]-8 1,2,4-triazolo [1,5-c] pyrimidine one-2 (3H)

8. Dérivés selon la revendication 1 ou 2 caractérisés en ce qu'ils sont choisis parmi les dérivés :
   - n-Propyl-7 méthyl-5 [[(tétrazolyl-5)-2' biphényl-yl-4] méthyl]-8 1,2,4-triazolo [4,3-c] pyrimidine one-3 (2H)
   - n-Propyl-7 méthyl-5 mercapto-3 [[(tétrazolyl-5)-2' biphényl-yl-4] méthyl]-8 1,2,4-triazolo [4,3-c] pyrimidine
   - n-Propyl-7 diméthyl-2,5 [[(tétrazolyl-5)-2' biphényl-yl-4] méthyl]-8 1,2,4-triazolo [1,5-c] pyrimidine
   - n-Propyl-7 méthyl-5 [[(tétrazolyl-5)-2' biphényl-yl-4] méthyl]-8 1,2,4-triazolo [1,5-c] pyrimidine
   - n-Propyl-7 méthyl-5 amino-2 [[(tétrazolyl-5)-2' biphényl-yl-4] méthyl]-8 1,2,4-triazolo [1,5-c] pyrimidine
   - n-Propyl-7 méthyl-5 méthylamino-2 [[(tétrazolyl-5)-2' biphényl-yl-4] méthyl]-8 1,2,4-triazolo [1,5-c] pyrimidine
   - [n-Propyl-7 méthyl-5 [[(tétrazolyl-5)-2' biphényl-yl-4] méthyl]-8 1,2,4-triazolo [1,5-c] pyrimidine-yl-2] carboxylate d'éthyle
   - [n-Propyl-7 méthyl-5 [[(tétrazolyl-5)-2' biphényl-yl-4] méthyl]-8 1,2,4-triazolo [1,5-c] pyrimidine-yl-2] acétate d'éthyle
   - Ethyl-7 diméthyl-2,5 [[(tétrazolyl-5)-2' biphényl-yl-4] méthyl]-8 1,2,4-triazolo [1,5-c] pyrimidine
   - n-Butyl-7 méthyl-5 [[(tétrazolyl-5)-2' biphényl-yl-4] méthyl]-8 1,2,4-triazolo [1,5-c] pyrimidine one-2 (3H)
   - n-Propyl-7 éthyl-5 [[(tétrazolyl-5)-2' biphényl-yl-4] méthyl]-8 1,2,4-triazolo [1,5-c] pyrimidine one-2 (3H)
   - n-Propyl-7 méthoxyméthyl-5 [[(tétrazolyl-5)-2' biphényl-yl-4] méthyl]-8 1,2,4-triazolo [1,5-c] pyrimidine one-2 (3H)

9. Procédé de préparation des composés de formule (I) selon l'une quelconque des revendications 1 à 8, caractérisé en ce qu'il comprend la préparation d'un composé intermédiaire de formule (X) ou (X") :

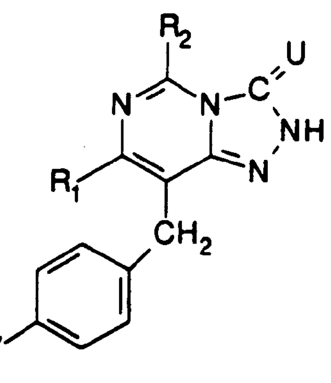

Formule (X)

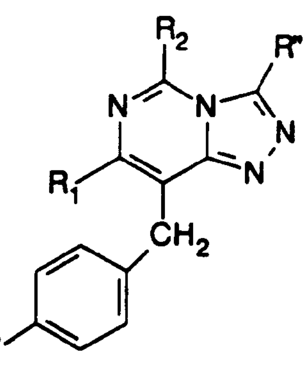

Formule (X")

dans lesquelles $R_1$, $R_2$ sont tels que définis la revendication 1 ; V représente un groupe choisi parmi les radicaux $NO_2$,

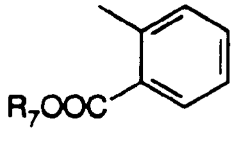 , 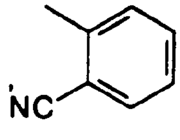 , 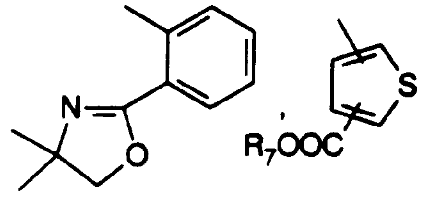 , 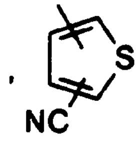

$R_7$ représentant un radical alkyle inférieur ou un benzyle ; U représente l'atome d'oxygène ou de soufre ou un groupement N-R", R" est tel que défini à la revendication 1 ; par cyclisation des composés de formule (IX) :

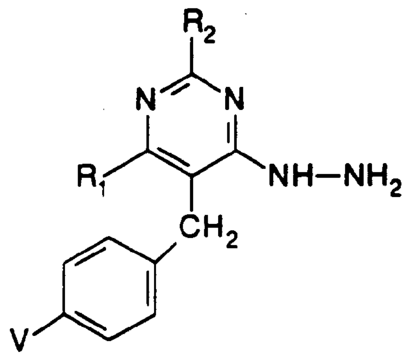

dans laquelle $R_1$, $R_2$ et V sont définis comme ci-dessus ; ladite cyclisation étant réalisée par l'action sur les composés de formule (IX) :
soit du carbonyl diimidazole au reflux du tétrahydrofurane ;
soit de l'urée par chauffage sans solvant ou dans un solvant comme la N-méthyl pyrrolidone ;
soit du xanthogénate de potassium au reflux d'un alcool comme le méthoxy éthanol ;
soit du sulfure de carbone dans un alcool en présence ou non d'une amine comme la triéthylamine ;
soit d'un isocyanate suivie d'un traitement au $POCl_3$ ;
soit d'un isothiocyanate suivie d'une méthylation en $SCH_3$ par l'iodure de méthyle puis cyclisation thermique ;
soit d'un ortho ester par chauffage;
soit encore d'un chlorure d'acide suivie d'une cyclisation à l'aide de $POCl_3$.

**10.** Procédé de préparation selon la revendication 9, caractérisé en ce qu'il comprend la préparation d'un composé intermédiaire de formule (XI) ou (XI") :

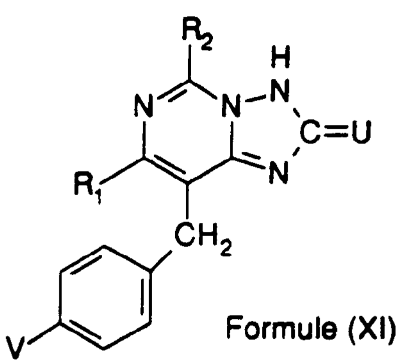

Formule (XI)

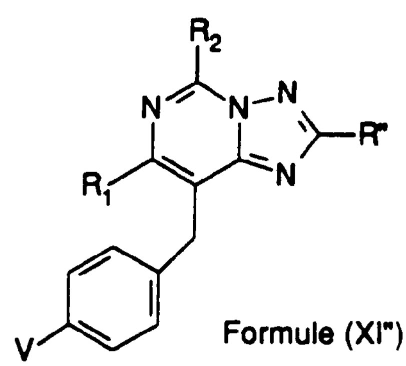

Formule (XI")

dans lesquelles $R_1$, $R_2$, V, U et R" sont définis comme ci-dessus, par isomérisation des composés précités de formule (X) ou (X") en milieu basique dans l'eau ou dans un mélange eau-alcool à une température comprise entre 20 et 100°C ; cette isomérisation pouvant être encore réalisée en milieu acide par chauffage dans l'acide acétique en présence ou non d'acétate de sodium ou de potassium ou encore dans le dichloro benzène en présence d'acide formique.

**11.** Procédés de préparation des composés de formule (I) dans laquelle $R_3$ représente un groupement

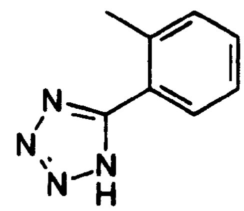

caractérisés en ce qu'on fait réagir un composé de formule :

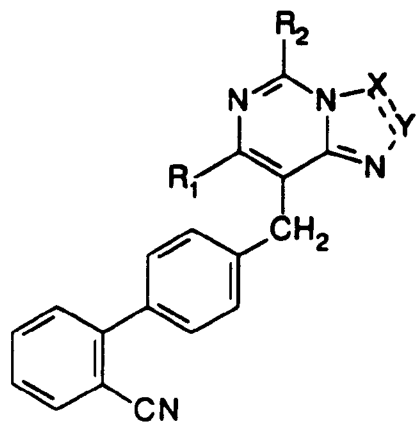

dans laquelle $R_1$, $R_2$, X et Y sont définis comme ci-dessus avec l'azoture de sodium dans le diméthyl formamide en présence d'un sel d'ammonium comme le chlorure d'ammonium à une température comprise entre 100 et 150°C ou par chauffage dans le toluène ou le xylène avec l'azoture de triméthyl étain suivi d'un traitement par le gaz chorhydrique dans le tétrahydrofurane par exemple.

**12.** Procédé de préparation d'une composition pharmaceutique, caractérisé en ce qu'on incorpore une quantité pharmaceutiquement efficace d'au moins un composé de formule (I) telle que définie dans l'une quelconque des revendications 1 à 8, ou un de ses sels d'addition pharmaceutiquement acceptables, dans un excipient, véhicule ou support pharmaceutiquement acceptable.

**13.** Procédé selon la revendication 12, caractérisé en ce que la composition pharmaceutique est formulée sous forme de gélules, de comprimés dosés de 1 à 400 mg ou sous forme de préparations injectables dosées de 0,01 à 50 mg.

**Patentansprüche**

**Patentansprüche für folgende Vertragsstaaten : AT, BE, CH, DE, DK, FR, GB, IT, LI, LU, MC, NL, PT, SE**

**1.** Triazolpyrimidinderivate, dadurch gekennzeichnet, daß sie der allgemeinen Formel (I) entsprechen:

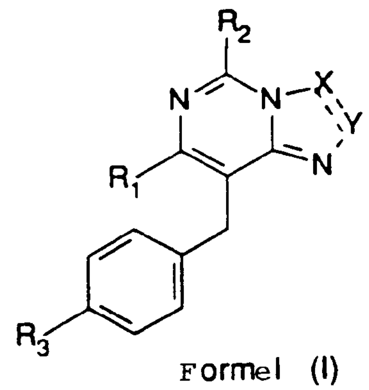

Formel (I)

worin:

$R_1$ eine nied.Alkylgruppe mit 1 bis 6 Kohlenstoffatomen oder eine $C_3$-$C_7$-Cycloalkylgruppe darstellt,

$R_2$ ein Wasserstoffatom, eine nied.Alkylgruppe mit 1 bis 6 Kohlenstoffatomen, eine nied.Halogenalkylgruppe mit 1 bis 6 Kohlenstoffatomen, eine Gruppe $NR_4R'_4$, eine Gruppe $NH$-$NH_2$ oder eine Gruppe $(CH_2)_mOR_4$, $(CH_2)_mSR_4$ darstellt,

$R_4$ und $R'_4$, die gleich oder verschieden sind, ein Wasserstoffatom oder eine nied.Alkylgruppe mit 1 bis 6 Kohlenstoffatomen darstellen und m eine ganze Zahl von 0 bis 5 ist,

die Gruppe —X----Y— oder —Y----X— eine der folgenden bivalenten Gruppen darstellt:

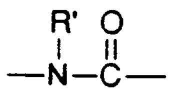 , 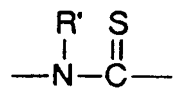 , 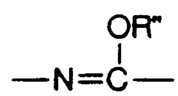 , 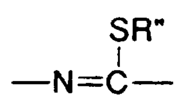 , 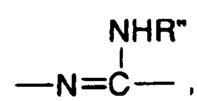 ,

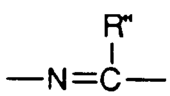 , 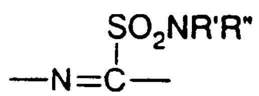

worin R' und R", die gleich oder verschieden sind, darstellen: ein Wassserstoffatom, eine nied.Alkylgruppe mit 1 bis 6 Kohlenstoffatomen, eine nied.Halogenalkylgruppe mit 1 bis 6 Kohlenstoffatomen, eine Gruppe $(CH_2)_nCOOR_5$, $(CH_2)_{n'}OR_5$, $(CH_2)_{n'}O$-$CO$-$R_5$, $(CH_2)_{n'}SR_5$, wobei n eine ganze Zahl von 0 bis 5 ist, n' eine ganze Zahl von 1 bis 5 ist und $R_5$ ein Wasserstoffatom oder eine nied.Alkylgruppe mit 1 bis 6 Kohlenstoffatomen ist;

eine Phenyl-, Pyridyl-, Thienyl- oder Furylgruppe;

$R_3$ eine Gruppe $NO_2$, $NH_2$ darstellt oder eine der folgenden Gruppen repräsentiert:

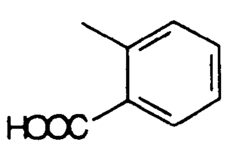 , 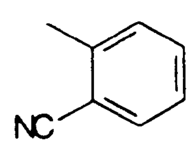 , 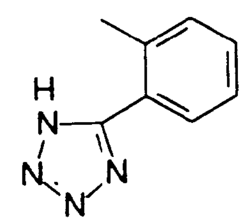 , 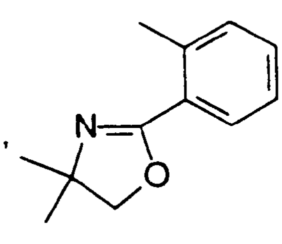 ,

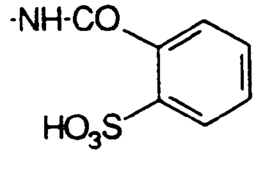 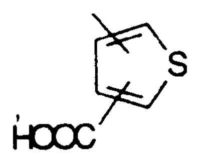 , 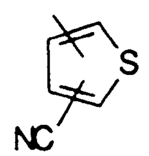 , 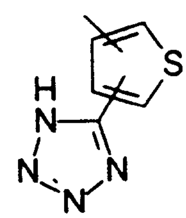

Die oben beschriebenen Derivate sind auch in ihrer Tautomerenform in Betracht zu ziehen und können

in Form von Additionssalzen, insbesondere pharmazeutisch akzeptablen Additionssalzen, vorliegen.

2. Triazolpyrimidinderivate nach Anspruch 1, dadurch gekennzeichnet, daß:

$R_1$ eine nied.Alkylgruppe mit 1 bis 6 Kohlenstoffatomen oder eine $C_3$-$C_7$-Cycloalkylgruppe darstellt,

$R_2$ ein Wasserstoffatom, eine nied.Alkylgruppe mit 1 bis 6 Kohlenstoffatomen, eine Gruppe NH-$NH_2$, eine Gruppe $(CH_2)_mOR_4$ oder $(CH_2)_mSR_4$ darstellt, wobei $R_4$ ein Wasserstoffatom oder eine nied.Alkylgruppe mit 1 bis 6 Kohlenstoffatomen darstellt und m eine ganze Zahl von 0 bis 2 ist,

die Gruppe —X---Y— eine Gruppe, ausgewählt aus den folgenden bivalenten Gruppen darstellt:

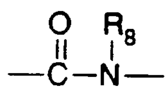 ; 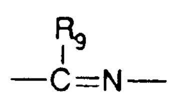 ; 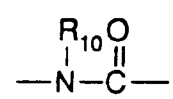 ; 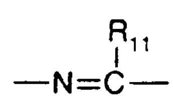

worin:

$R_8$ eine Gruppe, ausgewählt aus der Gruppe bestehend aus Wasserstoff, $(CH_2)_nOH$, $(CH_2)_nCOOH$, $-R_{12}$ oder $-(CH_2)_nCOOR_{12}$, ist, wobei $R_{12}$ eine nied.Alkylgruppe mit 1 bis 6 Kohlenstoffatomen darstellt und n eine ganze Zahl gleich 1 oder 2 ist;

$R_9$ Wasserstoff oder eine -SH-Gruppe darstellt;

$R_{10}$ Wasserstoff oder eine nied.Alkylgruppe mit 1 bis 6 Kohlenstoffatomen darstellt;

$R_{11}$ eine Gruppe, ausgewählt aus der Gruppe bestehend aus Wasserstoff, einer nied.Alkylgruppe mit 1 bis 6 Kohlenstoffatomen, einer nied.Halogenalkylgruppe mit 1 bis 6 Kohlenstoffatomen, Phenyl, Pyridinyl, $-O(CH_2)_nOH$, $-OR_{12}$, $-O(CH_2)_n$ $OCOR_{12}$, SH, $-SR_{12}$, $-S(CH_2)_nCOOR_{12}$, $-S(CH_2)_n$ $OCOR_{12}$, -NH $(CH_2)_n$ $COOR_{12}$, $-NR_{13}R_{14}$, $SO_2NR_{13}R_{14}$, $(CH_2)_nOH$, $(CH_2)_n$ $OR_{12}$, COOH, $COOR_{12}$, $(CH_2)_nCOOH$, $(CH_2)_nCOOR_{12}$ darstellt, wobei n und $R_{12}$ wie zuvor definiert sind, $R_{13}$ und $R_{14}$, die gleich oder verschieden sind, Wasserstoff oder eine nied.Alkylgruppe mit 1 bis 6 Kohlenstoffatomen darstellen;

$R_3$ eine der folgenden Gruppen darstellt:

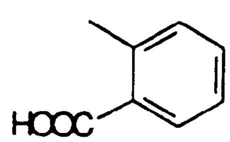 , 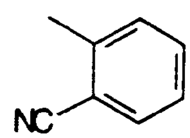 , 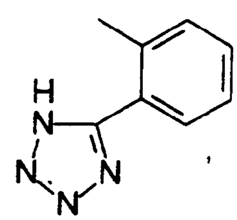 , 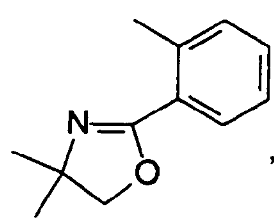 ,

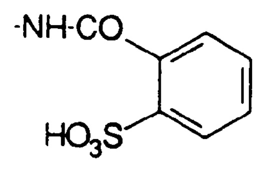 , 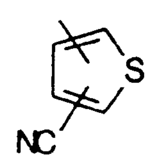 , 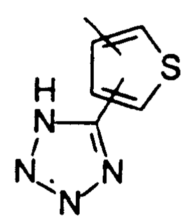

3. Derivate nach Anspruch 1 oder 2, dadurch gekennzeichnet, daß $R_1$ eine Gruppe, ausgewählt aus Ethyl, n-Propyl und n-Butyl, ist.

4. Derivate nach einem der Ansprüche 1 bis 3, dadurch gekennzeichnet, daß $R_2$ eine Gruppe, ausgewählt aus Methyl, Ethyl oder Methoxymethyl, ist.

5. Derivate nach einem der Ansprüche 1 bis 4, dadurch gekennzeichnet, daß die Gruppe —X----Y— eine der folgenden bivalenten Gruppen darstellt:

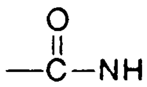 , 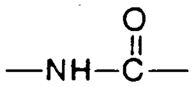 , 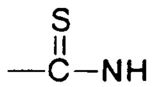 , 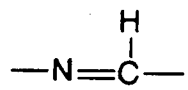 ,

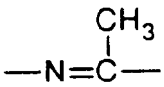 , 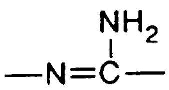 , 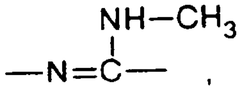 , 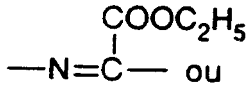 ou

oder

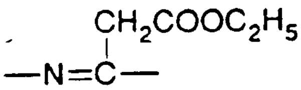

oder ihre Tautomerenform.

6. Derivate nach einem der Ansprüche 1 bis 5, dadurch gekennzeichnet, daß $R_3$ eine 2-(5-Tetrazolyl)-phenylgruppe ist.

7. Derivat nach Anspruch 1 oder 2, dadurch gekennzeichnet, daß es sich um das Derivat 7-n-Propyl-5-methyl-8-[[2'-(5-tetrazolyl)-biphenyl-4-yl]-methyl]-1,2,4-triazolo-[1,5-c]-pyrimidin-2-on-(3H) handelt.

8. Derivate nach Anspruch 1 oder 2, dadurch gekennzeichnet, daß sie aus den folgenden Derivaten ausgewählt sind:
   - 7-n-Propyl-5-methyl-8-[[2'-(5-tetrazolyl)-biphenyl-4-yl]-methyl]-1,2,4-triazolo-[4,3-c]-pyrimidin-3-on-(2H)
   - 7-n-Propyl-5-methyl-3-mercapto-8-[[2'-(5-tetrazolyl)-biphenyl-4-yl]-methyl]-1,2,4-triazolo-[4,3-c]-pyrimidin
   - 7-n-Propyl-2,5-dimethyl-8-[[2'-(5-tetrazolyl)-biphenyl-4-yl]-methyl]-1,2,4-triazolo-[1,5-c]-pyrimidin
   - 7-n-Propyl-5-methyl-8-[[2'-(5-tetrazolyl)-biphenyl-4-yl]-methyl]-1,2,4-triazolo-[1,5-c]-pyrimidin
   - 7-n-Propyl-5-methyl-2-amino-8-[[2'-(5-tetrazolyl)-biphenyl-4-yl]-methyl]-1, 2,4-triazolo-[1,5-c]-pyrimidin
   - 7-n-Propyl-5-methyl-2-methylamino-8-[[2'-(5-tetrazolyl)-biphenyl-4-yl]-methyl]-1,2,4-triazolo-[1,5-c]-pyrimidin
   - [7-n-Propyl-5-methyl-8-[[2'-(5-tetrazolyl)-biphenyl-4-yl]-methyl]-1,2,4-triazolo-[1,5-c]-pyrimidin-2-yl]-ethylcarboxylat
   - [7-n-Propyl-5-methyl-8-[[2'-(5-tetrazolyl)-biphenyl-4-yl]-methyl]-1,2,4-triazolo-[1,5-c]-pyrimidin-2-yl]-ethylacetat
   - 7-Ethyl-2,5-dimethyl-8-[[2'-(5-tetrazolyl)-biphenyl-4-yl]-methyl]-1,2,4-triazolo-[1,5-c]-pyrimidin
   - 7-n-Butyl-5-methyl-8-[[2'-(5-tetrazolyl)-biphenyl-4-yl]-methyl]-1,2,4-triazolo-[1,5-c]-pyrimidin-2-on-(3H)
   - 7-n-Propyl-5-ethyl-8-[[2'-(5-tetrazolyl)-biphenyl-4-yl]-methyl]-1,2,4-triazolo-[1,5-c]-pyrimidin-2-on-(3H)
   - 7-n-Propyl-5-methoxymethyl-8-[[2'-(5-tetrazolyl)-biphenyl-4-yl]-methyl]-1,2,4-triazolo-[1,5-c]-pyrimidin-2-on-(3H).

9. Verfahren zur Herstellung der Verbindungen der Formel (I) nach einem der Ansprüche 1 bis 8, dadurch gekennzeichnet, daß es die Herstellung einer Zwischenverbindung der Formel (X) oder (X"):

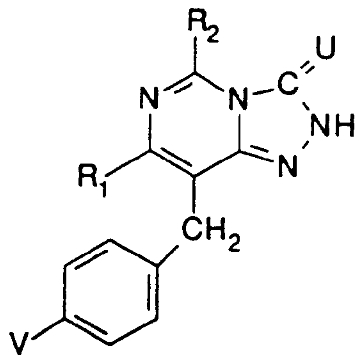

Formel (X)

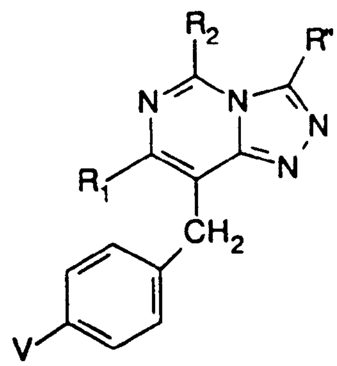

Formel (X")

worin $R_1$ und $R_2$ wie in Anspruch 1 definiert sind, V eine Gruppe, ausgewählt aus den Gruppen $NO_2$,

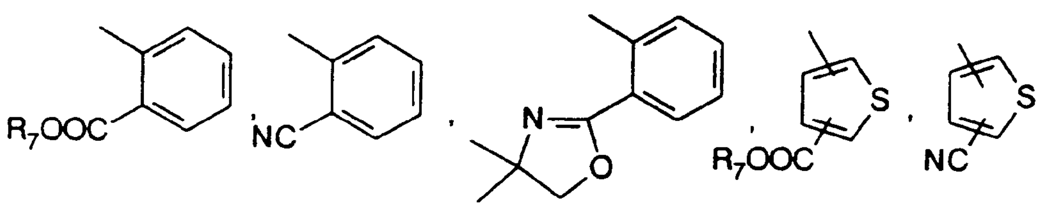

, , , ,

darstellt, $R_7$ eine nied.Alkylgruppe oder Benzyl darstellt, U ein Sauerstoff- oder Schwefelatom oder eine Gruppe N-R" darstellt, wobei R" wie in Anspruch 1 definiert ist,
durch Zyklisierung der Verbindungen der Formel (IX):

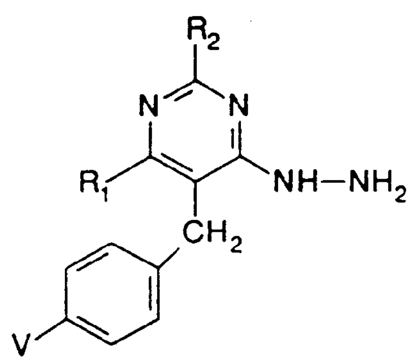

worin $R_1$, $R_2$ und V wie oben definiert sind, umfaßt, welche Zyklisierung durch Umsetzung der Verbindungen der Formel (IX):
entweder mit Carbonyldiimidazol am Rückfluß des Tetrahydrofurans;
oder mit Harnstoff durch Erhitzen ohne Lösungsmittel oder in einem Lösungsmittel, wie N-Methylpyrrolidon;
oder mit Kaliumxanthogenat am Rückfluß eines Alkohols, wie Methoxyethanol;
oder mit Schwefelkohlenstoff in einem Alkohol, gegebenenfalls in Anwesenheit eines Amins, wie Triethylamin;
oder mit einem Isocyanat und anschließender Behandlung mit $POCl_3$;
oder mit einem Isothiocyanat und anschließender Methylierung in $SCH_3$ mit Methyljodid und dann thermischer Zyklisierung;
oder mit einem Orthoester durch Erhitzen;
oder aber mit einem Säurechlorid und anschließender Zyklisierung mittels $POCl_3$
ausgeführt wird.

10. Herstellungsverfahren nach Anspruch 9, dadurch gekennzeichnet, daß es die Herstellung einer Zwischenverbindung der Formel (XI) oder (XI"):

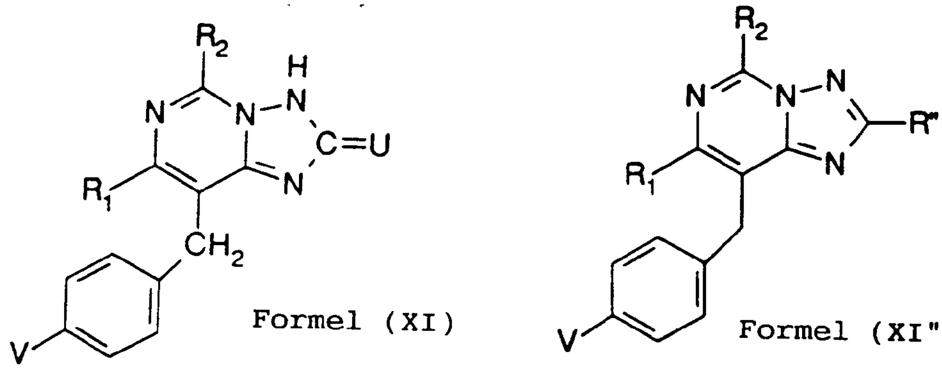

Formel (XI) Formel (XI")

worin $R_1$, $R_2$, V, U und R" wie oben definiert sind, durch Isomerisierung der vorgenannten Verbindungen der Formel (X) oder (X") in basischem Medium in Wasser oder in einer Wasser-Alkohol-Mischung bei einer Temperatur zwischen 20 und 100° umfaßt, welche Isomerisierung auch noch in saurem Medium durch Erhitzen in Essigsäure, gegebenenfalls in Gegenwart von Natrium- oder Kaliumacetat oder auch in Dichlorbenzol in Gegenwart von Ameisensäure durchgeführt werden kann.

**11.** Verfahren zur Herstellung der Verbindungen der Formel (I), worin $R_3$ eine Gruppe

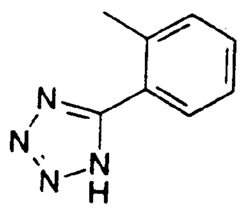

darstellt, dadurch gekennzeichnet, daß man eine Verbindung der Formel:

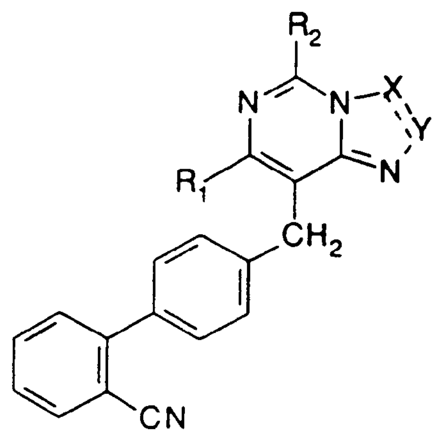

worin $R_1$, $R_2$, X und Y wie oben definiert sind, mit Natriumnitrid in Dimethylformamid in Gegenwart eines Ammoniumsalzes, wie Ammoniumchlorid, bei einer Temperatur zwischen 100 und 150°C oder durch Erhitzen in Toluol oder Xylol mit Trimethylzinnitrid und anschließender Behandlung mit Salzsäuregas in z.B. Tetrahydrofuran reagieren läßt.

**12.** Pharmazeutische Zusammensetzung, dadurch gekennzeichnet, daß sie eine pharmazeutisch wirksame Menge mindestens einer Verbindung der Formel (I), wie in einem der Ansprüche 1 bis 8 definiert, oder eines ihrer pharmazeutisch akzeptablen Additionssalze, gegebenenfalls eingearbeitet in einem pharmazeutisch akzeptablen Exzipienten, Vehikel oder Träger, enthält.

**13.** Pharmazeutische Zusammensetzung mit antagonistischer Aktivität gegen Angiotensin-II-Rezeptoren zur erfolgreichen Behandlung von kardiovaskulären Erkrankungen, insbesondere Bluthochdruck, Herzinsuffizienz, Erkrankungen der Arterienwand, dadurch gekennzeichnet, daß sie eine pharmazeutisch wirksame Menge mindestens einer Verbindung der Formel (I), wie in einem der Ansprüche 1 bis 8 definiert, oder eines ihrer pharmazeutisch akzeptablen Additionssalze, gegebenenfalls eingearbeitet in einem pharmazeutisch akzeptablen Exzipienten, Vehikel oder Träger, enthält.

**14.** Verfahren zur Herstellung einer pharmazeutischen Zusammensetzung, dadurch gekennzeichnet, daß eine pharmazeutisch wirksame Menge mindestens einer Verbindung der Formel (I), wie in einem der Ansprüche 1 bis 8 definiert, oder eines ihrer pharmazeutisch akzeptablen Additionssalze in einen pharmazeutisch akzeptablen Exzipienten, Vehikel oder Träger eingearbeitet wird.

**15.** Verfahren nach Anspruch 14, dadurch gekennzeichnet, daß die pharmazeutische Zusammensetzung in Form von Kapseln, Tabletten mit einer Dosierung von 1 bis 400 mg oder in Form von Injektionspräparaten mit einer Dosierung von 0,01 bis 50 mg formuliert wird.

**Patentansprüche für folgenden Vertragsstaat : ES**

**1.** Verfahren zur Herstellung von Verbindungen der Formel (I)

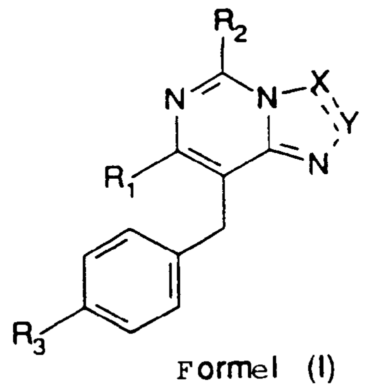

Formel (I)

worin:

$R_1$ eine nied.Alkylgruppe mit 1 bis 6 Kohlenstoffatomen oder eine $C_3$- $C_7$-Cycloalkylgruppe darstellt,

$R_2$ ein Wasserstoffatom, eine nied.Alkylgruppe mit 1 bis 6 Kohlenstoffatomen, eine nied.Halogenalkylgruppe mit 1 bis 6 Kohlenstoffatomen, eine Gruppe $NR_4R'_4$, eine Gruppe $NH\text{-}NH_2$ oder eine Gruppe $(CH_2)_mOR_4$, $(CH_2)_mSR_4$ darstellt,

$R_4$ und $R'_4$, die gleich oder verschieden sind, ein Wasserstoffatom oder eine nied.Alkylgruppe mit 1 bis 6 Kohlenstoffatomen darstellen und m eine ganze Zahl von 0 bis 5 ist,

die Gruppe —X---Y— oder —Y----X— eine der folgenden bivalenten Gruppen darstellt:

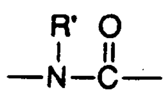 , 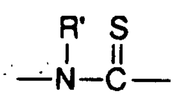 , 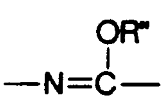 , 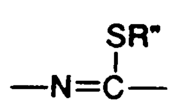 , 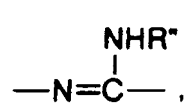 ,

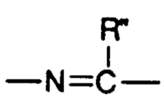 , 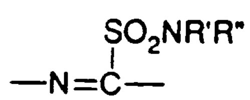

worin R' und R", die gleich oder verschieden sind, darstellen:

ein Wassserstoffatom, eine nied.Alkylgruppe mit 1 bis 6 Kohlenstoffatomen, eine nied.Halogenalkylgruppe mit 1 bis 6 Kohlenstoffatomen, eine Gruppe $(CH_2)_nCOOR_5$, $(CH_2)_{n'}OR_5$, $(CH_2)_{n'}O\text{-}CO\text{-}R_5$, $(CH_2)_{n'}SR_5$, wobei n eine ganze Zahl von 0 bis 5 ist, n' eine ganze Zahl von 1 bis 5 ist und $R_5$ ein Wasserstoffatom oder eine nied.Alkylgruppe mit 1 bis 6 Kohlenstoffatomen ist;

eine Phenyl-, Pyridyl-, Thienyl- oder Furylgruppe;

$R_3$ eine Gruppe $NO_2$, $NH_2$ darstellt oder eine der folgenden Gruppen repräsentiert:

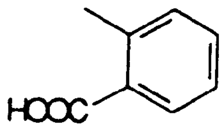 , 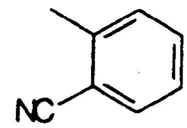 , 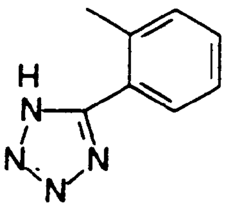 , 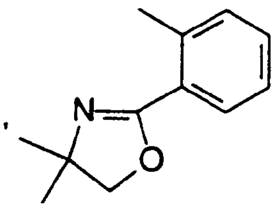 ,

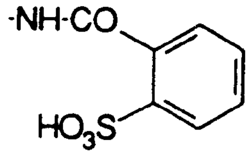 , 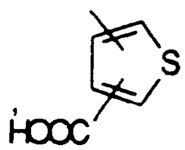 , 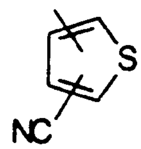 , 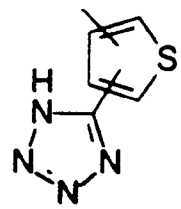 ,

wobei die oben beschriebenen Derivate auch in ihrer Tautomerenform in Betracht zu ziehen sind und in Form von Additionssalzen, insbesondere pharmazeutisch akzeptablen Additionssalzen, vorliegen können, dadurch gekennzeichnet, daß es die Herstellung einer Zwischenverbindung der Formel (X) oder (X"):

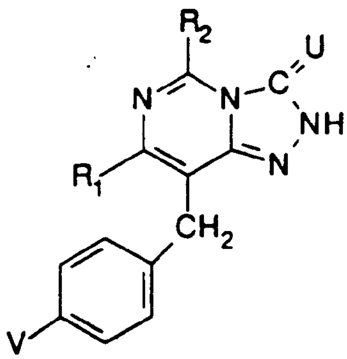

Formel (X)

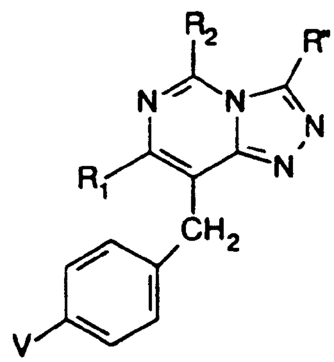

Formel (X")

worin $R_1$ und $R_2$ wie zuvor definiert sind, V eine Gruppe, ausgewählt aus den Gruppen $NO_2$,

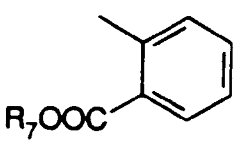 , 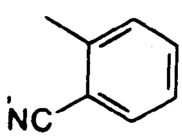 , 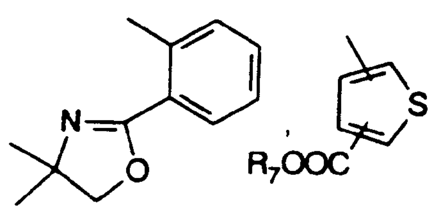 , 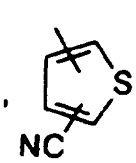

darstellt, $R_7$ eine nied.Alkylgruppe oder Benzyl darstellt, U ein Sauerstoff- oder Schwefelatom oder eine Gruppe N-R" darstellt, wobei R" wie zuvor definiert ist,
durch Zyklisierung der Verbindungen der Formel (IX):

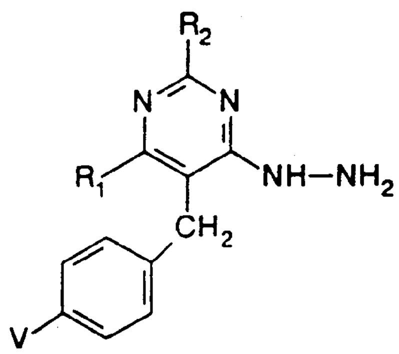

worin $R_1$, $R_2$ und V wie oben definiert sind, umfaßt, welche Zyklisierung durch Umsetzung der Verbindungen der Formel (IX):
entweder mit Carbonyldiimidazol am Rückfluß des Tetrahydrofurans;
oder mit Harnstoff durch Erhitzen ohne Lösungsmittel oder in einem Lösungsmittel, wie N-Methylpyrrolidon;
oder mit Kaliumxanthogenat am Rückfluß eines Alkohols, wie Methoxyethanol;
oder mit Schwefelkohlenstoff in einem Alkohol, gegebenenfalls in Anwesenheit eines Amins, wie Triethylamin;
oder mit einem Isocyanat und anschließender Behandlung mit $POCl_3$;
oder mit einem Isothiocyanat und anschließender Methylierung in $SCH_3$ mit Methyljodid und dann thermischer Zyklisierung;
oder mit einem Orthoester durch Erhitzen;
oder aber mit einem Säurechlorid und anschließender Zyklisierung mittels $POCl_3$
ausgeführt wird.

2. Herstellungsverfahren nach Anspruch 1, dadurch gekennzeichnet, daß es die Herstellung einer Zwischenverbindung der Formel (XI) oder (XI"):

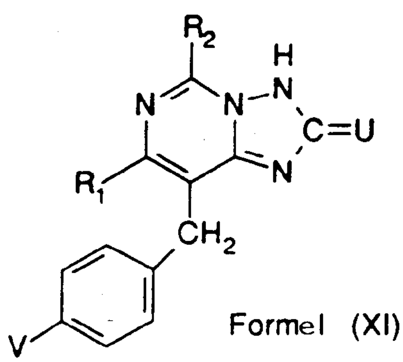

Formel (XI)

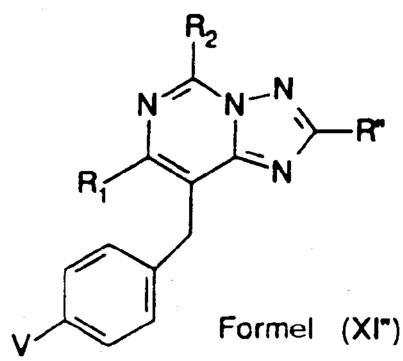

Formel (XI")

worin $R_1$, $R_2$, V, U und R" wie oben definiert sind, durch Isomerisierung der vorgenannten Verbindungen der Formel (X) oder (X") in basischem Medium in Wasser oder in einer Wasser-Alkohol-Mischung bei einer Temperatur zwischen 20 und 100° umfaßt, welche Isomerisierung auch noch in saurem Medium durch Erhitzen in Essigsäure, gegebenenfalls in Gegenwart von Natrium- oder Kaliumacetat oder auch in Dichlorbenzol in Gegenwart von Ameisensäure durchgeführt werden kann.

3. Herstellungsverfahren nach Anspruch 1 für Verbindungen der Formel (I), worin $R_3$ eine Gruppe

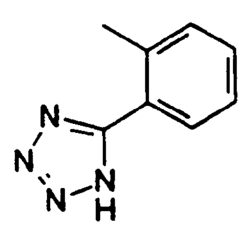

darstellt, dadurch gekennzeichnet, daß man eine Verbindung der Formel:

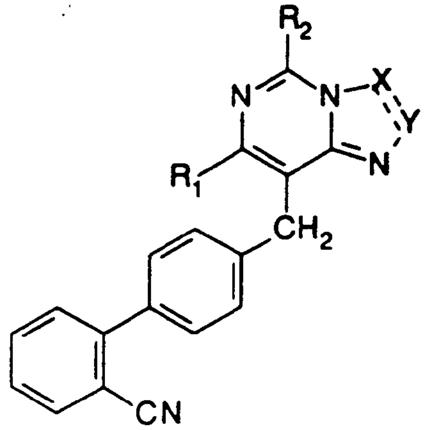

worin $R_1$, $R_2$, X und Y wie oben definiert sind, mit Natriumnitrid in Dimethylformamid in Gegenwart eines Ammoniumsalzes, wie Ammoniumchlorid, bei einer Temperatur zwischen 100 und 150°C oder durch Erhitzen in Toluol oder Xylol mit Trimethylzinnitrid und anschließender Behandlung mit Salzsäuregas in z.B. Tetrahydrofuran reagieren läßt.

**4.** Verfahren nach einem der Ansprüche 1 bis 3 zur Herstellung von Verbindungen der Formel (I), worin:

$R_1$ eine nied.Alkylgruppe mit 1 bis 6 Kohlenstoffatomen oder eine $C_3$-$C_7$-Cycloalkylgruppe darstellt,

$R_2$ ein Wasserstoffatom, eine nied.Alkylgruppe mit 1 bis 6 Kohlenstoffatomen, eine Gruppe NH-$NH_2$, eine Gruppe $(CH_2)_mOR_4$ oder $(CH_2)_mSR_4$ darstellt, wobei $R_4$ ein Wasserstoffatom oder eine nied.Alkylgruppe mit 1 bis 6 Kohlenstoffatomen darstellt und m eine ganze Zahl von 0 bis 2 ist,

die Gruppe —X---Y— eine Gruppe, ausgewählt aus den folgenden bivalenten Gruppen darstellt:

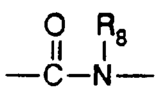 ; 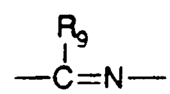 ; 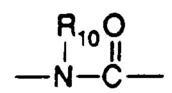 ; 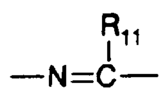

worin:

$R_8$ eine Gruppe, ausgewählt aus der Gruppe bestehend aus Wasserstoff, $(CH_2)_nOH$, $(CH_2)_nCOOH$, -$R_{12}$ oder -$(CH_2)_nCOOR_{12}$, ist, wobei $R_{12}$ eine nied.Alkylgruppe mit 1 bis 6 Kohlenstoffatomen darstellt und n eine ganze Zahl gleich 1 oder 2 ist;

$R_9$ Wasserstoff oder eine -SH-Gruppe darstellt;

$R_{10}$ Wasserstoff oder eine nied.Alkylgruppe mit 1 bis 6 Kohlenstoffatomen darstellt;

$R_{11}$ eine Gruppe, ausgewählt aus der Gruppe bestehend aus Wasserstoff, einer nied.Alkylgruppe mit 1 bis 6 Kohlenstoffatomen, einer nied.Halogenalkylgruppe mit 1 bis 6 Kohlenstoffatomen, Phenyl, Pyridinyl, -O $(CH_2)_nOH$, -$OR_{12}$, -O $(CH_2)_n$ $OCOR_{12}$, SH, -$SR_{12}$, -$S(CH_2)_nCOOR_{12}$, $S(CH_2)_n$ $OCOR_{12}$, -NH $(CH_2)_n$ $COOR_{12}$, -$NR_{13}R_{14}$, $SO_2NR_{13}R_{14}$, $(CH_2)_nOH$, $(CH_2)_n$ $OR_{12}$, COOH, $COOR_{12}$, $(CH_2)_nCOOH$, $(CH_2)_nCOOR_{12}$ darstellt, wobei n und $R_{12}$ wie zuvor definiert sind, $R_{13}$ und $R_{14}$, die gleich oder verschieden sind, Wasserstoff oder eine nied.Alkylgruppe mit 1 bis 6 Kohlenstoffatomen darstellen;

$R_3$ eine der folgenden Gruppen darstellt:

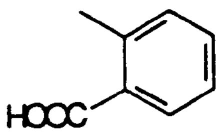, 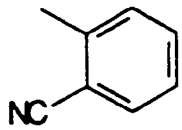, 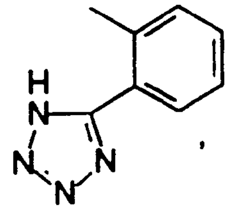, 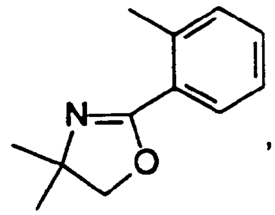,

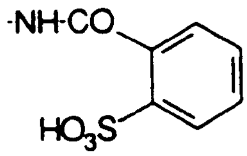, 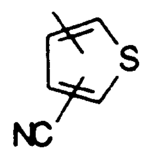, 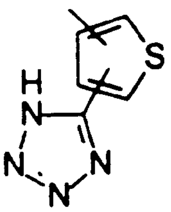

5. Verfahren nach einem der Ansprüche 1 bis 3, dadurch gekennzeichnet, daß $R_1$ eine Gruppe, ausgewählt aus Ethyl, n-Propyl und n-Butyl, ist.

6. Verfahren nach einem der Ansprüche 1 bis 3, dadurch gekennzeichnet, daß $R_2$ eine Gruppe, ausgewählt aus Methyl, Ethyl oder Methoxymethyl, ist.

7. Verfahren nach einem der Ansprüche 1 bis 3, dadurch gekennzeichnet, daß die Gruppe —X----Y— eine der folgenden bivalenten Gruppen darstellt:

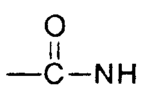, 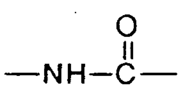, 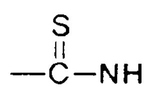, 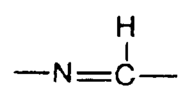,

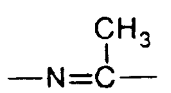, 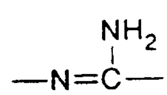, 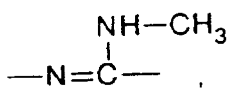, 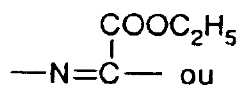 ou

oder

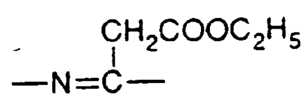

oder ihre Tautomerenform.

8. Verfahren nach einem der Ansprüche 1 bis 3, dadurch gekennzeichnet, daß $R_3$ eine 2-(5-Tetrazolyl)-phenylgruppe ist.

9. Verfahren nach einem der Ansprüche 1 bis 3 zur Herstellung des Derivats 7-n-Propyl-5-methyl-8-[[2'-(5-tetrazolyl)-biphenyl-4-yl]-methyl]-1,2,4-triazolo-[1,5-c]-pyrimidin-2-on-(3H).

10. Verfahren nach einem der Ansprüche 1 bis 3 zur Herstellung der folgenden Derivaten:
- 7-n-Propyl-5-methyl-8-[[2'-(5-tetrazolyl)-biphenyl-4-yl]-methyl]-1,2,4-triazolo-[4,3-c]-pyrimidin-3-on-(2H)
- 7-n-Propyl-5-methyl-3-mercapto-8-[[2'-(5-tetrazolyl)-biphenyl-4-yl]-methyl]-1,2,4-triazolo-[4,3-c]-pyrimidin
- 7-n-Propyl-2,5-dimethyl-8-[[2'-(5-tetrazolyl)-biphenyl-4-yl]-methyl]-1,2,4-triazolo-[1,5-c]-pyrimidin
- 7-n-Propyl-5-methyl-8-[[2'-(5-tetrazolyl)-biphenyl-4-yl]-methyl]-1,2,4-triazolo-[1,5-c]-pyrimidin
- 7-n-Propyl-5-methyl-2-amino-8-[[2'-(5-tetrazolyl)-biphenyl-4-yl]-methyl]-1,2,4-triazolo-[1,5-c]-pyrimidin

- 7-n-Propyl-5-methyl-2-methylamino-8-[[2'-(5-tetrazolyl)-biphenyl-4-yl]-methyl]-1,2,4-triazolo-[1,5-c]-pyrimidin
- [7-n-Propyl-5-methyl-8-[[2'-(5-tetrazolyl)-biphenyl-4-yl]-methyl]-1,2,4-triazolo-[1,5-c]-pyrimidin-2-yl]-ethylcarboxylat
- [7-n-Propyl-5-methyl-8-[[2'-(5-tetrazolyl)-biphenyl-4-yl]-methyl]-1,2,4-triazolo-[1,5-c]-pyrimidin-2-yl]-ethylacetat
- 7-Ethyl-2,5-dimethyl-8-[[2'-(5-tetrazolyl)-biphenyl-4-yl]-methyl]-1,2,4-triazolo-[1,5-c]-pyrimidin
- 7-n-Butyl-5-methyl-8-[[2'-(5-tetrazolyl)-biphenyl-4-yl]-methyl]-1,2,4-triazolo-[1,5-c]-pyrimidin-2-on-(3H)
- 7-n-Propyl-5-ethyl-8-[[2'-(5-tetrazolyl)-biphenyl-4-yl]-methyl]-1,2,4-triazolo-[1,5-c]-pyrimidin-2-on-(3H)
- 7-n-Propyl-5-methoxymethyl-8-[[2'-(5-tetrazolyl)-biphenyl-4- yl]-methyl]-1,2,4-triazolo-[1,5-c]-pyrimidin-2-on-(3H).

**11.** Verfahren zur Herstellung einer pharmazeutischen Zusammensetzung, dadurch gekennzeichnet, daß eine pharmazeutisch wirksame Menge mindestens einer Verbindung der Formel (I), die nach einem der Ansprüche 1 bis 9 erhalten wurde, in einen pharmazeutisch akzeptablen Exzipienten, Vehikel oder Träger eingearbeitet wird.

**12.** Verfahren nach Anspruch 10, dadurch gekennzeichnet, daß die pharmazeutische Zusammensetzung in Form von Kapseln, Tabletten mit einer Dosierung von 1 bis 400 mg oder in Form von Injektionspräparaten mit einer Dosierung von 0,01 bis 50 mg formuliert wird.

**Patentansprüche für folgenden Vertragsstaat : GR**

**1.** Triazolpyrimidinderivate, dadurch gekennzeichnet, daß sie der allgemeinen Formel (I) entsprechen:

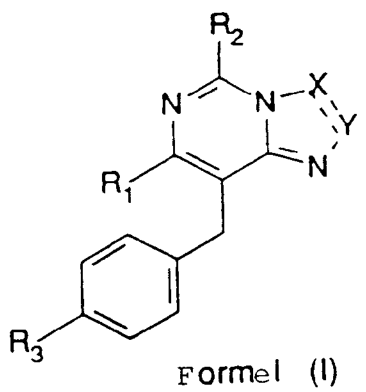

Formel (I)

worin:

$R_1$ eine nied.Alkylgruppe mit 1 bis 6 Kohlenstoffatomen oder eine $C_3$-$C_7$-Cycloalkylgruppe darstellt,

$R_2$ ein Wasserstoffatom, eine nied.Alkylgruppe mit 1 bis 6 Kohlenstoffatomen, eine nied.Halogenalkylgruppe mit 1 bis 6 Kohlenstoffatomen, eine Gruppe $NR_4R'_4$, eine Gruppe $NH$-$NH_2$ oder eine Gruppe $(CH_2)_mOR_4$, $(CH_2)_mSR_4$ darstellt,

$R_4$ und $R'_4$, die gleich oder verschieden sind, ein Wasserstoffatom oder eine nied.Alkylgruppe mit 1 bis 6 Kohlenstoffatomen darstellen und m eine ganze Zahl von 0 bis 5 ist,

die Gruppe —X----Y— oder —Y----X— eine der folgenden bivalenten Gruppen darstellt:

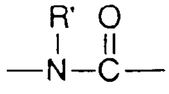 , 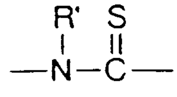 , 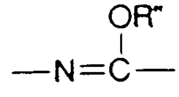 , 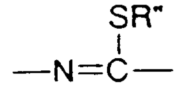 , 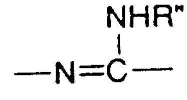 ,

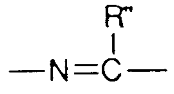 , 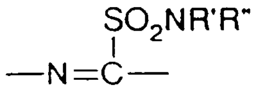

worin R' und R", die gleich oder verschieden sind, darstellen:

ein Wassserstoffatom, eine nied.Alkylgruppe mit 1 bis 6 Kohlenstoffatomen, eine nied.Halogenalkylgruppe mit 1 bis 6 Kohlenstoffatomen, eine Gruppe $(CH_2)_nCOOR_5$, $(CH_2)_{n'}OR_5$, $(CH_2)_{n'}O\text{-}CO\text{-}R_5$, $(CH_2)_{n'}SR_5$, wobei n eine ganze Zahl von 0 bis 5 ist, n' eine ganze Zahl von 1 bis 5 ist und $R_5$ ein Wasserstoffatom oder eine nied.Alkylgruppe mit 1 bis 6 Kohlenstoffatomen ist;

eine Phenyl-, Pyridyl-, Thienyl- oder Furylgruppe;

$R_3$ eine Gruppe $NO_2$, $NH_2$ darstellt oder eine der folgenden Gruppen repräsentiert:

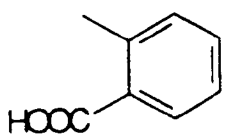 , 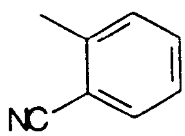 , 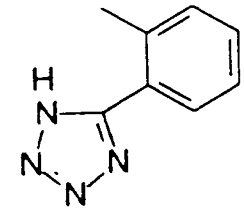 , 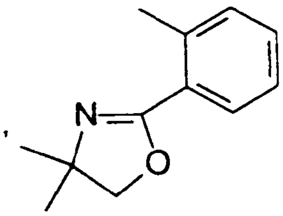 ,

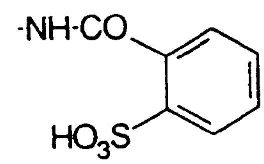 , 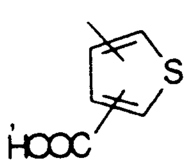 , 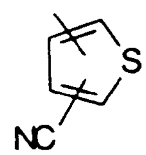 , 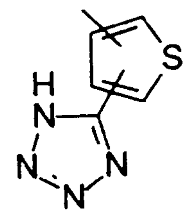

Die oben beschriebenen Derivate sind auch in ihrer Tautomerenform in Betracht zu ziehen und können in Form von Additionssalzen, insbesondere pharmazeutisch akzeptablen Additionssalzen, vorliegen.

**2.** Triazolpyrimidinderivate nach Anspruch 1, dadurch gekennzeichnet, daß:

$R_1$ eine nied.Alkylgruppe mit 1 bis 6 Kohlenstoffatomen oder eine $C_3$-$C_7$-Cycloalkylgruppe darstellt,

$R_2$ ein Wasserstoffatom, eine nied.Alkylgruppe mit 1 bis 6 Kohlenstoffatomen, eine Gruppe NH-$NH_2$, eine Gruppe $(CH_2)_mOR_4$ oder $(CH_2)_mSR_4$ darstellt, wobei $R_4$ ein Wasserstoffatom oder eine nied.Alkylgruppe mit 1 bis 6 Kohlenstoffatomen darstellt und m eine ganze Zahl von 0 bis 2 ist,

die Gruppe —X---Y— eine Gruppe, ausgewählt aus den folgenden bivalenten Gruppen darstellt:

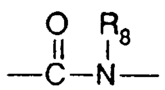 ; 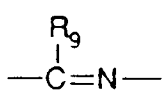 ; 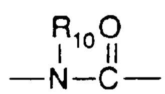 ; 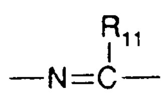

worin:

$R_8$ eine Gruppe, ausgewählt aus der Gruppe bestehend aus Wasserstoff, $(CH_2)_nOH$, $(CH_2)_nCOOH$, $-R_{12}$ oder $-(CH_2)_nCOO_{12}$, ist,

wobei $R_{12}$ eine nied.Alkylgruppe mit 1 bis 6 Kohlenstoffatomen darstellt und n eine ganze Zahl gleich 1 oder 2 ist;

$R_9$ Wasserstoff oder eine -SH-Gruppe darstellt;

$R_{10}$ Wasserstoff oder eine nied.Alkylgruppe mit 1 bis 6 Kohlenstoffatomen darstellt;

$R_{11}$ eine Gruppe, ausgewählt aus der Gruppe bestehend aus Wasserstoff, einer nied.Alkylgruppe mit 1 bis 6 Kohlenstoffatomen, einer nied.Halogenalkylgruppe mit 1 bis 6 Kohlenstoffatomen, Phenyl, Pyridinyl, $-O(CH_2)_nOH$, $-OR_{12}$, $-O(CH_2)_n\ OCOR_{12}$, SH, $-SR_{12}$, $-S(CH_2)_nCOOR_{12}$, $-S(CH_2)_n\ OCOR_{12}$, $-NH\ (CH_2)_n COOR_{12}$, $-NR_{13}R_{14}$, $SO_2NR_{13}R_{14}$, $(CH_2)_nOH$, $(CH_2)_n\ OR_{12}$, COOH, $COOR_{12}$, $(CH_2)_nCOOH$, $(CH_2)_nCOOR_{12}$ darstellt, wobei n und $R_{12}$ wie zuvor definiert sind, $R_{13}$ und $R_{14}$, die gleich oder verschieden sind, Wasserstoff oder eine nied.Alkylgruppe mit 1 bis 6 Kohlenstoffatomen darstellen;

$R_3$ eine der folgenden Gruppen darstellt:

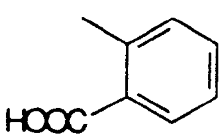, 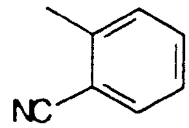, 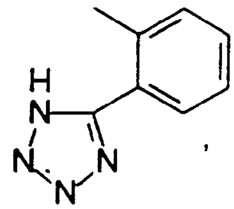, 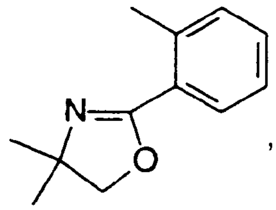,

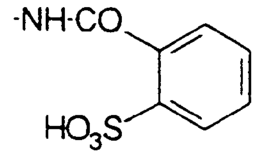, 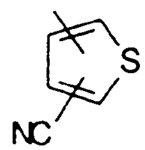, 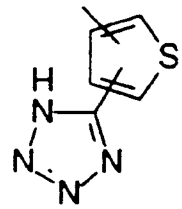

3. Derivate nach Anspruch 1 oder 2, dadurch gekennzeichnet, daß $R_1$ eine Gruppe, ausgewählt aus Ethyl, n-Propyl und n-Butyl, ist.

4. Derivate nach einem der Ansprüche 1 bis 3, dadurch gekennzeichnet, daß $R_2$ eine Gruppe, ausgewählt aus Methyl, Ethyl oder Methoxymethyl, ist.

5. Derivate nach einem der Ansprüche 1 bis 4, dadurch gekennzeichnet, daß die Gruppe —X----Y— eine der folgenden bivalenten Gruppen darstellt:

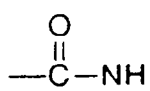, 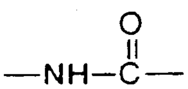, 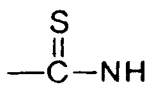, 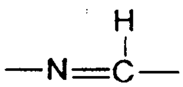,

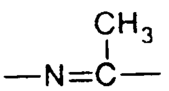, 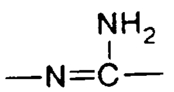, 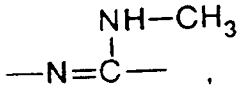, 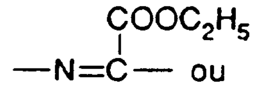 ou

oder

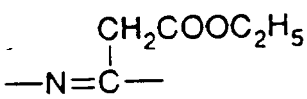

oder ihre Tautomerenform.

6. Derivate nach einem der Ansprüche 1 bis 5, dadurch gekennzeichnet, daß $R_3$ eine 2-(5-Tetrazolyl)-phenylgruppe ist.

7. Derivat nach Anspruch 1 oder 2, dadurch gekennzeichnet, daß es sich um das Derivat 7-n-Propyl-5-methyl-8-[[2'-(5-tetrazolyl)-biphenyl-4-yl]-methyl]-1,2,4-triazolo-[1,5-c]-pyrimidin-2-on-(3H) handelt.

8. Derivate nach Anspruch 1 oder 2, dadurch gekennzeichnet, daß sie aus den folgenden Derivaten ausgewählt sind:
   - 7-n-Propyl-5-methyl-8-[[2'-(5-tetrazolyl)-biphenyl-4-yl]-methyl]-1,2,4-triazolo-[4,3-c]-pyrimidin-3-on-(2H)
   - 7-n-Propyl-5-methyl-3-mercapto-8-[[2'-(5-tetrazolyl)-biphenyl-4-yl]-methyl]-1,2,4-triazolo-[4,3-c]-pyrimidin
   - 7-n-Propyl-2,5-dimethyl-8-[[2'-(5-tetrazolyl)-biphenyl-4-yl]-methyl]-1,2,4-triazolo-[1,5-c]-pyrimidin
   - 7-n-Propyl-5-methyl-8-[[2'-(5-tetrazolyl)-biphenyl-4-yl]-methyl]-1,2,4-triazolo-[1,5-c]-pyrimidin
   - 7-n-Propyl-5-methyl-2-amino-8-[[2'-(5-tetrazolyl)-biphenyl-4-yl]-methyl]-1,2,4-triazolo-[1,5-c]-pyrimidin

- 7-n-Propyl-5-methyl-2-methylamino-8-[[2'-(5-tetrazolyl)-biphenyl-4-yl]-methyl]-1,2,4-triazolo-[1,5-c]-pyrimidin
- [7-n-Propyl-5-methyl-8-[[2'-(5-tetrazolyl)-biphenyl-4-yl]-methyl]-1,2,4-triazolo-[1,5-c]-pyrimidin-2-yl]-ethylcarboxylat
- [7-n-Propyl-5-methyl-8-[[2'-(5-tetrazolyl)-biphenyl-4-yl]-methyl]-1,2,4-triazolo-[1,5-c]-pyrimidin-2-yl]-ethylacetat
- 7-Ethyl-2,5-dimethyl-8-[[2'-(5-tetrazolyl)-biphenyl-4-yl]-methyl]-1,2,4-triazolo-[1,5-c]-pyrimidin
- 7-n-Butyl-5-methyl-8-[[2'-(5-tetrazolyl)-biphenyl-4-yl]-methyl]-1,2,4-triazolo-[1,5-c]-pyrimidin-2-on-(3H)
- 7-n-Propyl-5-ethyl-8-[[2'-(5-tetrazolyl)-biphenyl-4-yl]-methyl]-1,2,4-triazolo-[1,5-c]-pyrimidin-2-on-(3H)
- 7-n-Propyl-5-methoxymethyl-8-[[2'-(5-tetrazolyl)-biphenyl-4-yl]-methyl]-1,2,4-triazolo-[1,5-c]-pyrimidin-2-on-(3H).

**9.** Verfahren zur Herstellung der Verbindungen der Formel (I) nach einem der Ansprüche 1 bis 8, dadurch gekennzeichnet, daß es die Herstellung einer Zwischenverbindung der Formel (X) oder (X"):

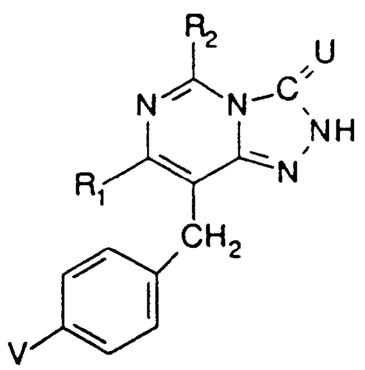

Formel (X)

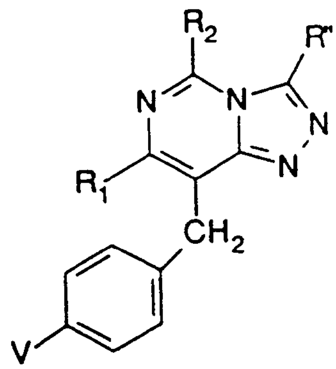

Formel (X")

worin $R_1$ und $R_2$ wie in Anspruch 1 definiert sind, V eine Gruppe, ausgewählt aus den Gruppen $NO_2$,

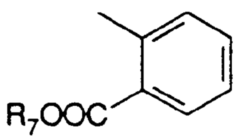, 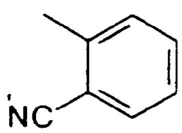, 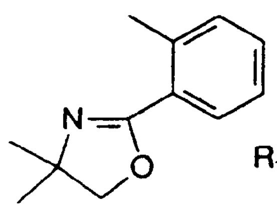, 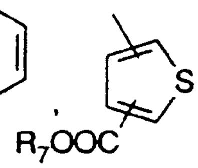, 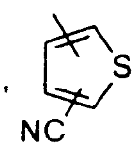

darstellt, $R_7$ eine nied.Alkylgruppe oder Benzyl darstellt, U ein Sauerstoff- oder Schwefelatom oder eine Gruppe N-R" darstellt, wobei R" wie in Anspruch 1 definiert ist,
durch Zyklisierung der Verbindungen der Formel (IX):

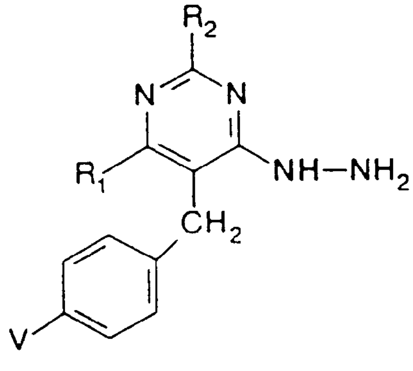

worin $R_1$, $R_2$ und V wie oben definiert sind, umfaßt, welche Zyklisierung durch Umsetzung der Verbindungen der Formel (IX):
entweder mit Carbonyldiimidazol am Rückfluß des Tetrahydrofurans;
oder mit Harnstoff durch Erhitzen ohne Lösungsmittel oder in einem Lösungsmittel, wie N-Methylpyrrolidon;
oder mit Kaliumxanthogenat am Rückfluß eines Alkohols, wie Methoxyethanol;
oder mit Schwefelkohlenstoff in einem Alkohol, gegebenenfalls in Anwesenheit eines Amins, wie Triethylamin;
oder mit einem Isocyanat und anschließender Behandlung mit $POCl_3$;
oder mit einem Isothiocyanat und anschließender Methylierung in $SCH_3$ mit Methyljodid und dann thermischer Zyklisierung;
oder mit einem Orthoester durch Erhitzen;
oder aber mit einem Säurechlorid und anschließender Zyklisierung mittels $POCl_3$
ausgeführt wird.

**10.** Herstellungsverfahren nach Anspruch 9, dadurch gekennzeichnet, daß es die Herstellung einer Zwischenverbindung der Formel (XI) oder (XI"):

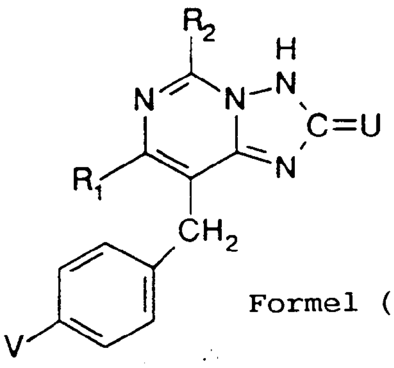
Formel (XI)

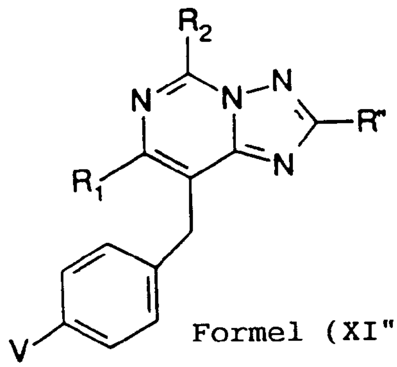
Formel (XI")

worin $R_1$, $R_2$, V, U und R" wie oben definiert sind, durch Isomerisierung der vorgenannten Verbindungen der Formel (X) oder (X") in basischem Medium in Wasser oder in einer Wasser-Alkohol-Mischung bei einer Temperatur zwischen 20 und 100° umfaßt, welche Isomerisierung auch noch in saurem Medium durch Erhitzen in Essigsäure, gegebenenfalls in Gegenwart von Natrium- oder Kaliumacetat oder auch in Dichlorbenzol in Gegenwart von Ameisensäure durchgeführt werden kann.

**11.** Verfahren zur Herstellung der Verbindungen der Formel (I), worin $R_3$ eine Gruppe

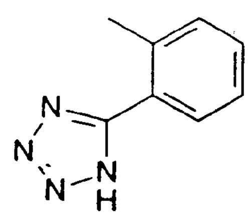

darstellt, dadurch gekennzeichnet, daß man eine Verbindung der Formel:

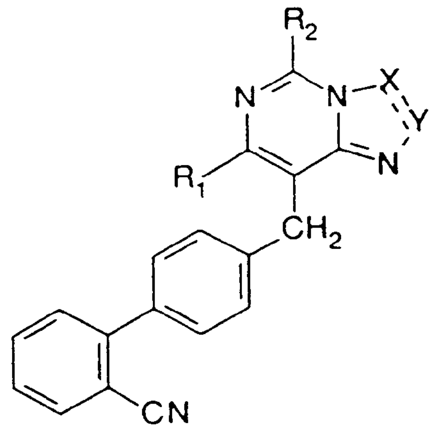

worin $R_1$, $R_2$, X und Y wie oben definiert sind, mit Natriumnitrid in Dimethylformamid in Gegenwart eines Ammoniumsalzes, wie Ammoniumchlorid, bei einer Temperatur zwischen 100 und 150°C oder durch Erhitzen in Toluol oder Xylol mit Trimethylzinnitrid und anschließender Behandlung mit Salzsäuregas in z.B. Tetrahydrofuran reagieren läßt.

**12.** Verfahren zur Herstellung einer pharmazeutischen Zusammensetzung, dadurch gekennzeichnet, daß eine pharmazeutisch wirksame Menge mindestens einer Verbindung der Formel (I), wie in einem der Ansprüche 1 bis 8 definiert, oder eines ihrer pharmazeutisch akzeptablen Additionssalze in einen pharmazeutisch akzeptablen Exzipienten, Vehikel oder Träger eingearbeitet wird.

**13.** Verfahren nach Anspruch 12, dadurch gekennzeichnet, daß die pharmazeutische Zusammensetzung in Form von Kapseln, Tabletten mit einer Dosierung von 1 bis 400 mg oder in Form von Injektionspräparaten mit einer Dosierung von 0,01 bis 50 mg formuliert wird.

## Claims

**Claims for the following Contracting States : AT, BE, CH, DE, DK, FR, GB, IT, LI, LU, MC, NL, PT, SE**

**1.** Triazolopyrimidine derivatives, characterized in that they correspond to general formula (I):

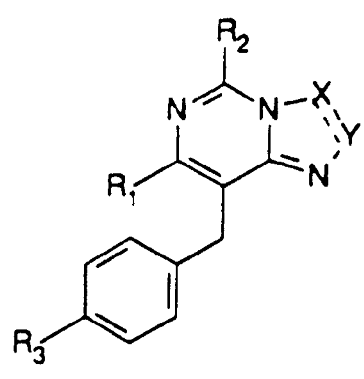

Formula (I)

in which:

$R_1$ represents a lower alkyl radical having 1 to 6 carbon atoms or a $C_3$-$C_7$ cycloalkyl radical,

$R_2$ represents a hydrogen atom, a lower alkyl radical having 1 to 6 carbon atoms, a lower haloalkyl radical having 1 to 6 carbon atoms, a group $NR_4R'_4$, an $NH$-$NH_2$ group or a group $(CH_2)_mOR_4$ or $(CH_2)_mSR_4$,

$R_4$ and $R'_4$, which may be identical or different, represent a hydrogen atom or a lower alkyl radical

having 1 to 6 carbon atoms and m represents an integer from 0 to 5,

the assembly -X---Y- or -Y---X- represents one of the following bivalent radicals:

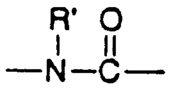 , 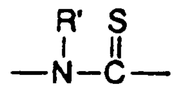 , 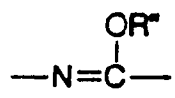 , 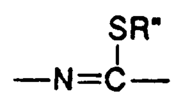 , 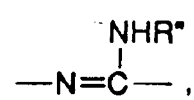 ,

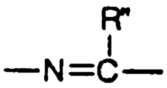 , 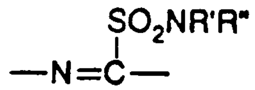

in which R' and R", which may be identical or different, represent:

a hydrogen atom, a lower alkyl radical having 1 to 6 carbon atoms, a lower haloalkyl radical having 1 to 6 carbon atoms or a group $(CH_2)_nCOOR_5$, $(CH_2)_{n'}-OR_5$, $(CH_2)_{n'}-O\ CO-R_5$ or $(CH_2)_{n'}SR_5$; n being an integer from 0 to 5, n' being an integer from 1 to 5 and $R_5$ being a hydrogen atom or a lower alkyl radical having 1 to 6 carbon atoms,

a phenyl, pyridyl, thienyl or furyl radical,

$R_3$ represents an $NO_2$ or $NH_2$ group or represents one of the following radicals:

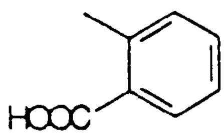 , 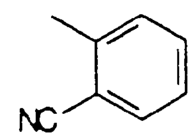 , 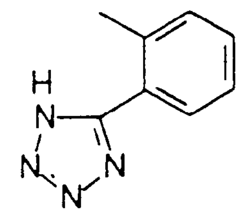 , 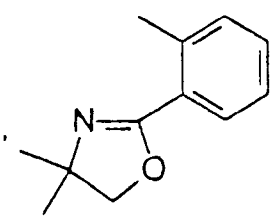 ,

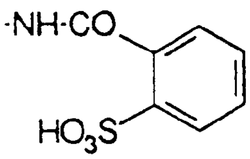 , 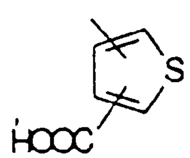 , 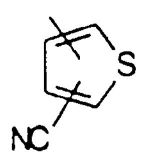 , 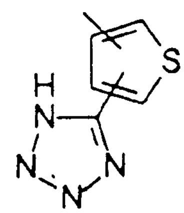

The abovementioned derivatives must also be considered in their tautomeric forms and may take the form of addition salts, especially of pharmaceutically acceptable addition salts.

**2.** Triazolopyrimidine derivatives according to claim 1, characterized in that:

$R_1$ represents a lower alkyl radical having 1 to 6 carbon atoms or a $C_3$-$C_7$ cycloalkyl radical:

$R_2$ represents a hydrogen atom, a lower alkyl radical having 1 to 6 carbon atoms, an $NH-NH_2$ group-for a group $(CH_2)_mOR_4$ or $(CH_2)_mSR_4$; $R_4$ representing a hydrogen atom or a lower alkyl radical having 1 to 6 carbon atoms and m representing an integer from 0 to 2;

the assembly -X---Y- representing a radical selected from the following bivalent radicals:

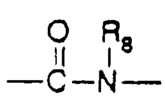 ; 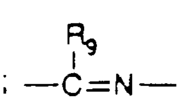 ; 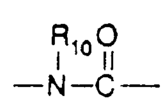 ; 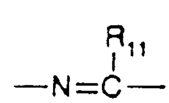

in which:

$R_8$ represents a radical selected from the group consisting of a hydrogen atom, $-(CH_2)_nOH$, $-(CH_2)_nCOOH$, $-R_{12}$, or $-(CH_2)_nCOOR_{12}$; $R_{12}$ representing a lower alkyl radical having 1 to 6 carbon atoms and n an integer equal to 1 or 2;

$R_9$ represents a hydrogen atom or an -SH radical;

$R_{10}$ represents a hydrogen atom or a lower alkyl radical having 1 to 6 carbon atoms;

$R_{11}$ represents a radical selected from the group consisting of a hydrogen atom, a lower alkyl radical having

1 to 6 carbon atoms, a lower haloalkyl radical having 1 to 6 carbon atoms, a phenyl, pyridinyl, $-O(CH_2)_nOH$, $-OR_{12}$, $-O(CH_2)_nOCOR_{12}$, SH, $-SR_{12}$, $-S(CH_2)_nCOOR_{12}$, $-S(CH_2)_nOCOR_{12}$, $-NH(CH_2)_nCOOR_{12}$, $-NR_{13}R_{14}$, $SO_2NR_{13}R_{14}$, $(CH_2)_nOH$, $(CH_2)_nOR_{12}$, COOH, $COOR_{12}$, $(CH_2)_nCOOH$, or $(CH_2)_nCOOR_{12}$; n and $R_{12}$ being defined as stated above, $R_{13}$ and $R_{14}$, which may be identical or different, representing the hydrogen atom or a lower alkyl radical having from 1 to 6 carbon atoms;

$R_3$ represents one of the following radicals:

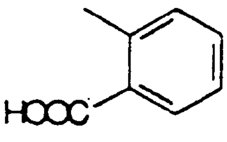 , 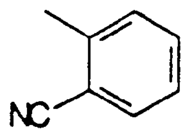 , 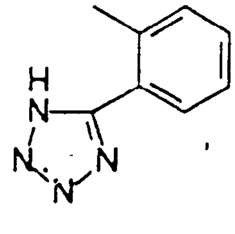 , 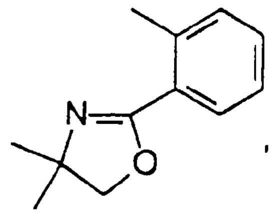 ,

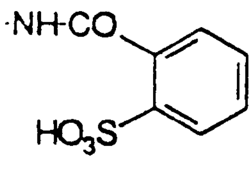 , 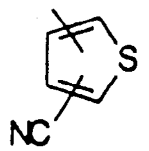 , 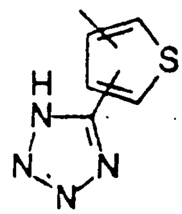

3. Derivatives according to claim 1 or 2, characterized in that $R_1$ is a group selected from ethyl, n-propyl and n-butyl.

4. Derivatives according to any one of claims 1 to 3, characterized in that $R_2$ is a group selected from methyl, ethyl or methoxymethyl.

5. Derivatives according to any one of claims 1 to 4, characterized in that the assembly -X---Y- represents one of the following bivalent radicals

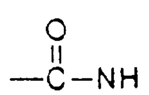 , 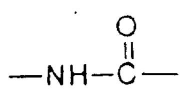 ,

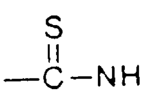 , 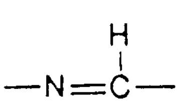 , 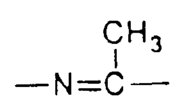 , 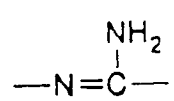 , 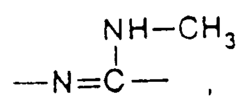 ,

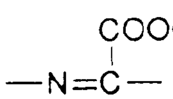 or 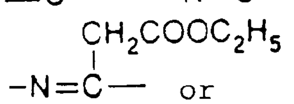 or

their tautomeric form.

6. Derivatives according to any one of claims 1 to 5, characterized in that $R_3$ is a 2-(5-tetrazolyl)phenyl group.

7. Derivative according to Claim 1 or 2, characterised in that it is the derivative:
- 7-n-Propyl-5-methyl-8-{[2'-(5-tetrazolyl)-4-biphenylyl]methyl}-1,2,4-triazolo[1,5-c]pyrimidin-2(3H)one.

8. Derivatives according to Claim 1 or 2, characterised in that they are selected from the derivatives:
- 7-n-Propyl-5-methyl-8-{[2'-(5-tetrazolyl)-4-biphenylyl]methyl}-1,2,4-triazolo[4,3-c]pyrimidin-3(2H)-one
- 7-n-Propyl-5-methyl-3-mercapto-8-{[2'-(5-tetrazolyl)-4-biphenylyl]methyl}-1,2,4-triazolo[4,3-c]pyrimidine
- 7-n-Propyl-2,5-dimethyl-8-{[2'-(5-tetrazolyl)4-biphenylyl]methyl}-1,2,4-triazolo[1,5-c]pyrimidine
- 7-n-Propyl-5-methyl-8-{[2'-(5-tetrazolyl)-4-biphenylyl]methyl}-1,2,4-triazolo[1,5-c]pyrimidine
- 7-n-Propyl-5-methyl-2-amino-8-{[2'-(5-tetrazolyl)-4-biphenylyl]methyl}-1,2,4-triazolo[1,5-c]pyrimidine

- 7-n-propyl-5-methyl-2-methylamino-8-{[2'-(5-tetrazolyl)-4-biphenylyl]methyl}-1,2,4-triazolo[1,5-c]-pyrimidine
- Ethyl [7-n-propyl-5-methyl-8-{[2'-(5-tetrazolyl)-4-biphenylyl]methyl}-1,2,4-triazolo[1,5-c]pyrimidine-2-yl] carboxylate
- Ethyl [7-n-propyl-5-methyl-8-{[2'-(5-tetrazolyl)-4-biphenylyl]methyl}-1,2,4-triazolo[1,5-c]pyrimidin-2-yl]acetate
- 7-Ethyl-2,5-dimethyl-8-{[2'-(5-tetrazolyl)-4-bi-phenylyl]methyl}-1,2,4-triazolo[1,5-c]pyrimidine
- 7-n-Butyl-5-methyl-8-{[2'-(5-tetrazolyl)-4-biphenylyl]methyl}-1,2,4-triazolo[1,5-c]pyrimidin-2(3H)-one
- 7-n-Propyl-5-ethyl-8-{[2'-(5-tetrazolyl)-4-biphenylyl]-methyl}-1,2,4-triazolo[1,5-c]pyrimidin-2(3H)-one
- 7-n-Propyl-5-methoxymethyl-8-{[2'-(5-tetrazolyl)-4-biphenylyl]methyl}-1,2,4-triazolo[1,5-c]pyrimidin-2(3H)-one.

**9.** Process for preparing the compounds of formula (I) according to any one of claims 1 to 8, characterized in that it comprises the preparation of an intermediate compound of formula (X) or (X"):

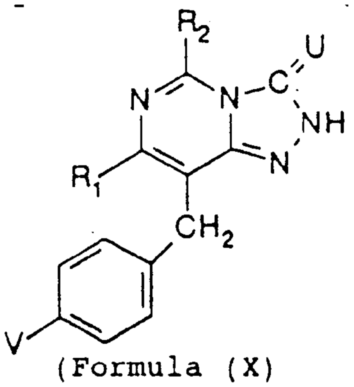

(Formula (X)

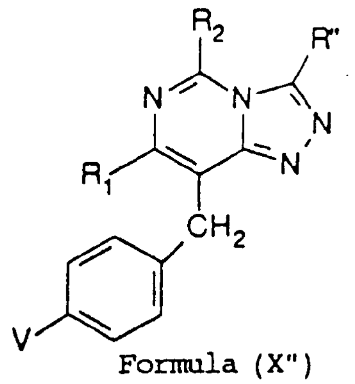

Formula (X")

in which $R_1$ and $R_2$ are as defined in Claim 1; V represents a group selected from the radicals $NO_2$,

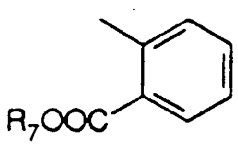

,

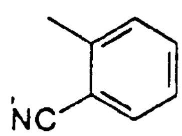

,

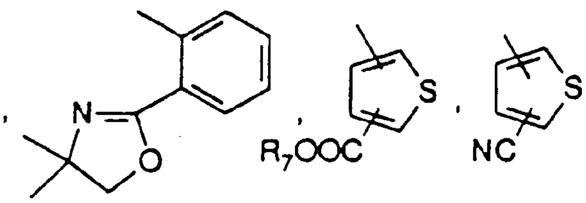

, ,

$R_7$ representing a lower alkyl radical or a benzyl;
U represents an oxygen or sulphur atom or a group N-R",
R" being as defined in Claim 1; by cyclisation of the compounds of formule (IX):

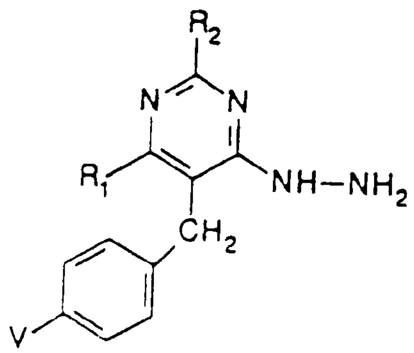

in which $R_1$, $R_2$ and V are defined as above; the said cyclisation being carried out by the action:
either of carbonyldiimidazole under reflux of tetrahydrofuran;
or by urea by heating without a solvent or in a solvent such as N-methylpyrrolidone;

or of potassium xanthogenate under reflux of an alcohol such as methoxyethanol;
or of carbon disulphide in an alcohol in the presence or absence of an amine such as triethylamine;
or of an isocyanate followed by a treatment with $POCl_3$;
or of an isothiocyanate followed by a methylation to $SCH_3$ with methyl iodide and then thermal cyclisation;
or of an ortho ester, by heating;
or alternatively of an acid chloride followed by a cyclisation using $POCl_3$;
on the compounds of formula (IX).

10. Preparation process according to Claim 9, characterised in that it comprises the preparation of an intermediate compound of formula (XI) or (XI"):

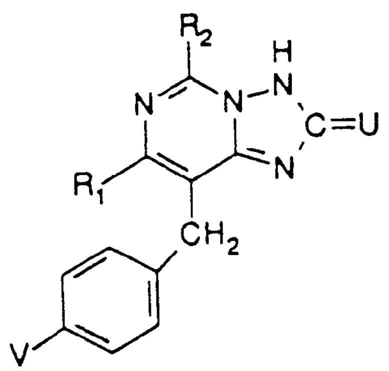

Formula (XI)

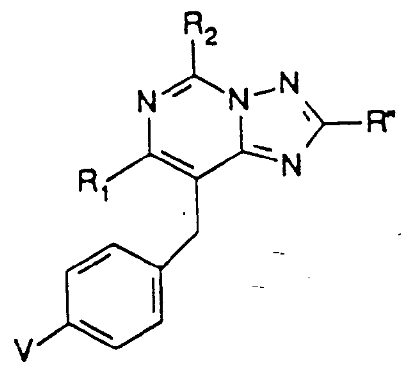

(Formula (XI")

in which $R_1$, $R_2$, V, U and R" are defined as above, by isomerisation of the abovementioned compounds of formula (X) or (X") in a basic medium in water or in a water/alcohol mixture at a temperature of between 20 and 100°C; it also being possible for this isomerisation to be carried out in an acid medium by heating in acetic acid in the presence or absence of sodium acetate or potassium acetate or alternatively in dichlorobenzene in the presence of formic acid.

11. Processes for preparing the compounds of formula (I) in which $R_3$ represents a group

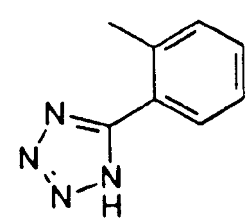

characterised in that a compound of formula:

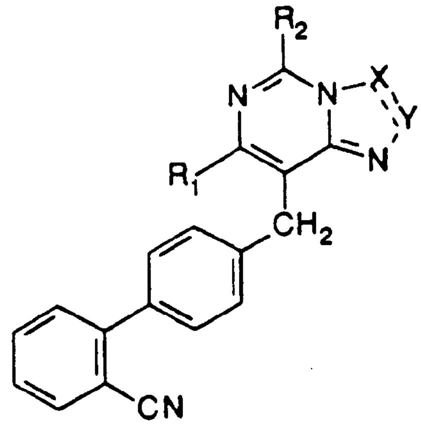

in which $R_1$, $R_2$, X and Y are defined as above, is reacted with sodium azide in dimethylformamide in the presence of an ammonium salt such as ammonium chloride at a temperature of between 100 and 150°C, or by heating in toluene or xylene with trimethyltin azide followed by a treatment with hydrogen chloride gas in tetrahydrofuran, for example.

**12.** Pharmaceutical composition, characterised in that it comprises a pharmaceutically effective amount of at least one compound of formula (I) as defined in any one of Claims 1 to 8, or one of its pharmaceutically acceptable addition salts, optionally incorporated in a pharmaceutically acceptable excipient, vehicle or carrier.

**13.** Pharmaceutical composition having angiotensin II receptor antagonist activity, permitting a favourable treatment of cardiovascular diseases, in particular hypertension, cardiac insufficiency and diseases of the arterial wall, characterised in that it contains a pharmaceutically effective amount of at least one compound of formula (I) as defined in any one of Claims 1 to 8, or one of its pharmaceutically acceptable addition salts, optionally incorporated in a pharmaceutically acceptable excipient, vehicle or carrier.

**14.** Process for preparing a pharmaceutical composition, characterised in that a pharmaceutically effective amount of at least one compound of formula (I) as defined in any one of Claims 1 to 8, or one of its pharmaceutically acceptable addition salts, is incorporated in a pharmaceutically acceptable excipient, vehicle or carrier.

**15.** Process according to Claim 14, characterised in that the pharmaceutical composition is formulated in the form of hard gelatin capsules or tablets containing 1 to 400 mg of active principle or in the form of injectable preparations containing 0.01 to 50 mg of active principle.

**Claims for the following Contracting State : ES**

**1.** Process for the preparation of compounds of formula (I):

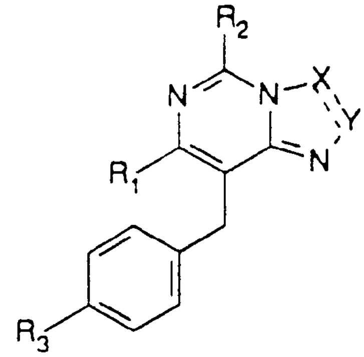

Formula (I)

in which:

$R_1$ represents a lower alkyl radical having 1 to 6 carbon atoms or a $C_3$-$C_7$ cycloalkyl radical,

$R_2$ represents a hydrogen atom, a lower alkyl radical having 1 to 6 carbon atoms, a lower haloalkyl radical having 1 to 6 carbon atoms, a group $NR_4R'_4$, an $NH$-$NH_2$ group or a group $(CH_2)_mOR_4$ or $(CH_2)_mSR_4$,

$R_4$ and $R'_4$, which may be identical or different, represent a hydrogen atom or a lower alkyl radical having 1 to 6 carbon atoms and m represents an integer from 0 to 5,

the assembly -X---Y- or -Y---X- represents one of the following bivalent radicals:

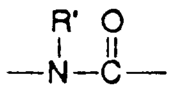 , 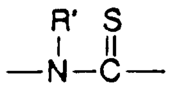 , 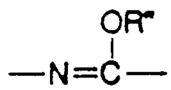 , 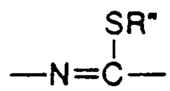 , 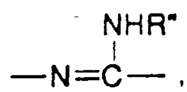 ,

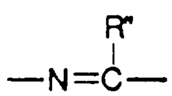 , 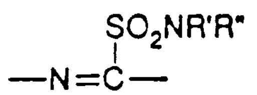

in which R' and R", which may be identical or different, represent:

a hydrogen atom, a lower alkyl radical having 1 to 6 carbon atoms, a lower haloalkyl radical having 1 to 6 carbon atoms or a group $(CH_2)_nCOOR_{5'}(CH_2)_{n'}$-$OR_5$, $(CH_2)_{n'}$-O-CO-$R_5$ or $(CH_2)_{n'}SR_5$; n being an integer from 0 to 5, n' being an integer from 1 to 5 and $R_5$ being a hydrogen atom or a lower alkyl radical having 1 to 6 carbon atoms,

a phenyl, pyridyl, thienyl or furyl radical,

$R_3$ represents an $NO_2$ or $NH_2$ group or represents one of the following radicals:

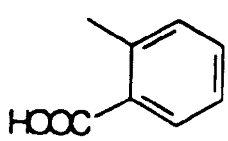 , 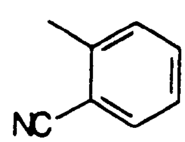 , 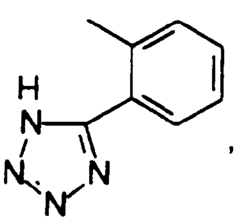 , 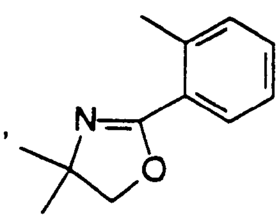

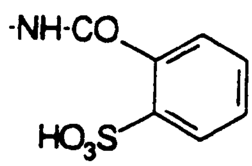 , 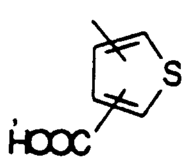 , 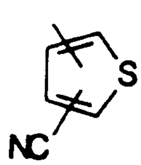 , 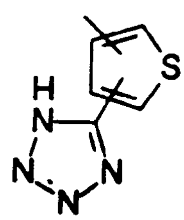 ,

the abovementioned derivatives having also to be considered in their tautomeric forms and being able to take the form of addition salts, especially of pharmaceutically acceptable addition salts, characterized in that it comprises the preparation of an intermediate compound of formula (X) or (X"):

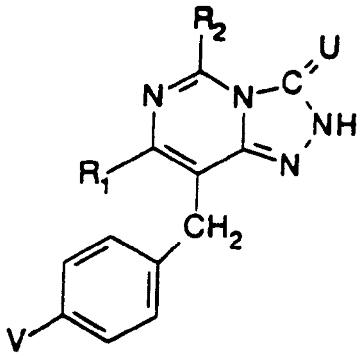

Formula (X)

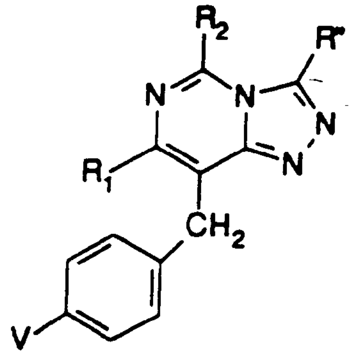

Formula (X")

in which $R_1$ and $R_2$ are as defined above; V represents a group selected from the radicals $NO_2$,

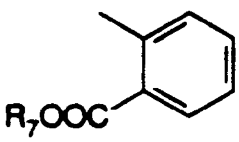
,
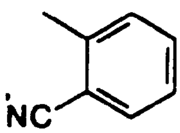
,
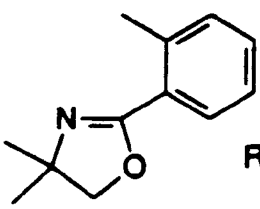
,
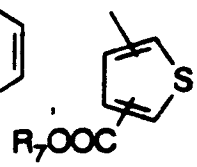
,
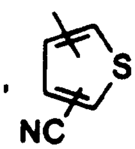

$R_7$ representing a lower alkyl or a benzyl radical; and U represents an oxygen or sulphur atom or a group N-R", R" being as defined above; by cyclisation of the compounds of formula (IX):

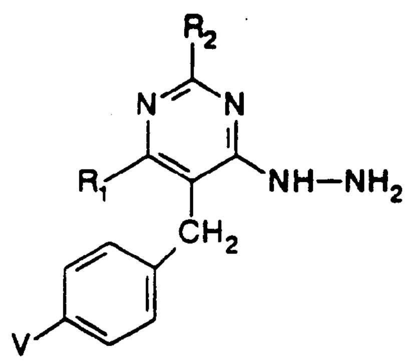

in which $R_1$, $R_2$ and V are defined as above; the said cyclisation being carried out by the action:
either of carbonyldiimidazole under reflux of tetrahydrofuran;
or by urea by heating without a solvent or in a solvent such as N-methylpyrrolidone;
or of potassium xanthogenate under reflux of an alcohol such as methoxyethanol;
or of carbon disulphide in an alcohol in the presence or absence of an amine such as triethylamine;
or of an isocyanate followed by a treatment with $POCl_3$;
or of an isothiocyanate followed by a methylation to $SCH_3$ with methyl iodide and then thermal cyclisation;
or of an ortho ester, by heating;
or alternatively of an acid chloride followed by a cyclisation using $POCl_3$;
on the compounds of formula (IX).

2. Preparation process according to Claim 1, characterised in that it comprises the preparation of an intermediate compound of formula (XI) or (XI"):

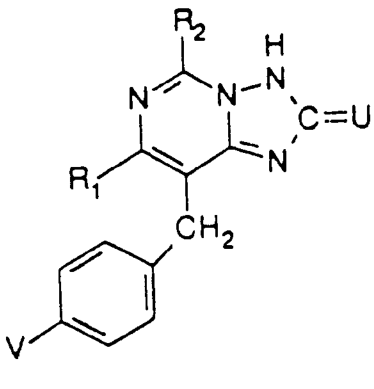

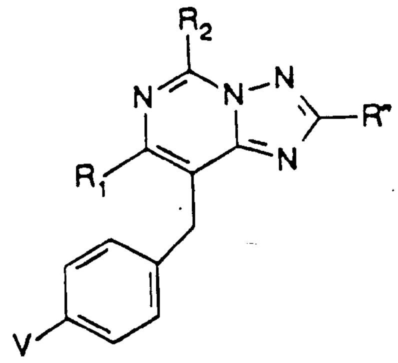

Formula (XI) (Formula (XI")

in which $R_1$, $R_2$, V, U and R" are defined as above, by isomerisation of the abovementioned compounds of formula (X) or (X") in a basic medium in water or in a water/alcohol mixture at a temperature of between 20 and 100°C; it also being possible for this isomerisation to be carried out in an acid medium by heating in acetic acid in the presence or absence of sodium acetate or potassium acetate or alternatively in dichlorobenzene in the presence of formic acid.

**3.** Preparation process according to claim 1 for preparing the compounds of formula (I) in which $R_3$ represents a group

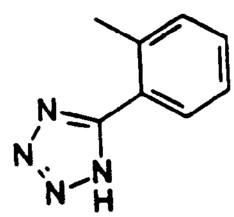

characterized in that a compound of formula:

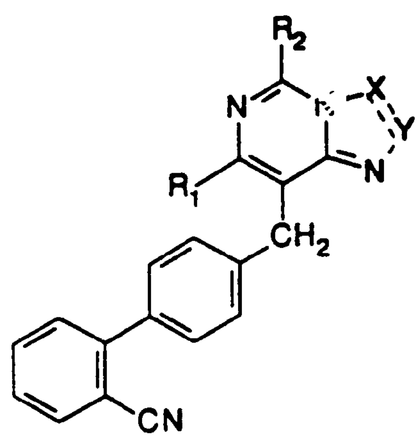

in which $R_1$, $R_2$, X and Y are defined as above, is reacted with sodium azide in dimethylformamide in the presence of an ammonium salt such as ammonium chloride at a temperature of between 100 and 150°C, or by heating in toluene or xylene with trimethyltin azide followed by a treatment with hydrogen chloride gas in tetrahydrofuran, for example.

**4.** Process according to one of claims 1 to 3,
for preparing the compounds of formula (I) in which:

$R_1$ represents a lower alkyl radical having 1 to 6 carbon atoms or a $C_3$-$C_7$ cycloalkyl radical:

$R_2$ represents a hydrogen atom, a lower alkyl radical having 1 to 6 carbon atoms, an NH-$NH_2$ group or a group $(CH_2)_mOR_4$ or $(CH_2)_mSR_4$; $R_4$ representing a hydrogen atom or a lower alkyl radical having 1 to 6 carbon atoms and m representing an integer from 0 to 2;

the assembly -X---Y- representing a radical selected from the following bivalent radicals:

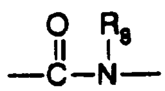 ; 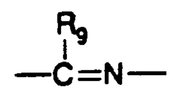 ; 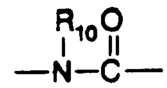 ; 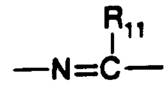

in which:

$R_8$ represents a radical selected from the group consisting of a hydrogen atom, $-(CH_2)_nOH$, $-(CH_2)_nCOOH$, $-R_{12}$, or $-(CH_2)_nCOOR_{12}$; $R_{12}$ representing a lower alkyl radical having 1 to 6 carbon atoms and n an integer equal to 1 or 2;

$R_9$ represents a hydrogen atom or an -SH radical;

$R_{10}$ represents a hydrogen atom or a lower alkyl radical having 1 to 6 carbon atoms;

$R_{11}$ represents a radical selected from the group consisting of a hydrogen atom, a lower alkyl radical having 1 to 6 carbon atoms, a lower haloalkyl radical having 1 to 6 carbon atoms, a phenyl, pyridinyl, $-O(CH_2)_nOH$, $-OR_{12}$, $-O(CH_2)_nOCOR_{12}$, SH, $-SR_{12}$, $-S(CH_2)_nCOOR_{12}$, $-S(CH_2)_nOCOR_{12}$, $-NH(CH_2)_nCOOR_{12}$, $-NR_{13}R_{14}$, $SO_2NR_{13}R_{14}$, $(CH_2)_nOH$, $(CH_2)_nOR_{12}$, COOH, $COOR_{12}$, $(CH_2)_nCOOH$, or $(CH_2)_nCOOR_{12}$; n and $R_{12}$ being defined as stated above, $R_{13}$ and $R_{14}$, which may be identical or different, representing a hydrogen atom or a lower alkyl radical having from 1 to 6 carbon atoms;

$R_3$ represents one of the following radicals:

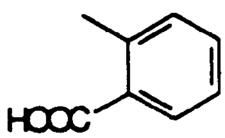 , 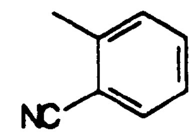 , 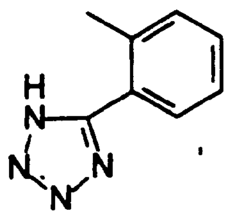 , 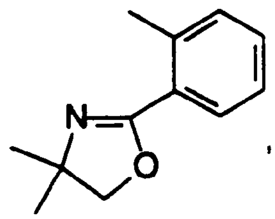 ,

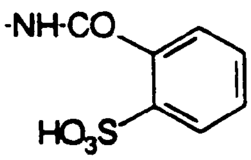 , 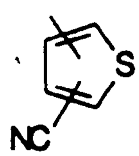 , 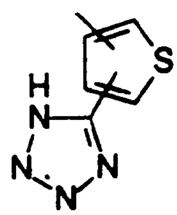

5. Process according to one of claims 1 to 3, characterized in that $R_1$ is a group selected from ethyl, n-propyl and 4-butyl.

6. Process according to one of claims 1 to 3, characterized in that $R_2$ is a group selected from methyl, ethyl or methoxymethyl.

7. Process according to one of claims 1 to 3, characterized in that the assembly -X---Y- represents one of the following bivalent radicals

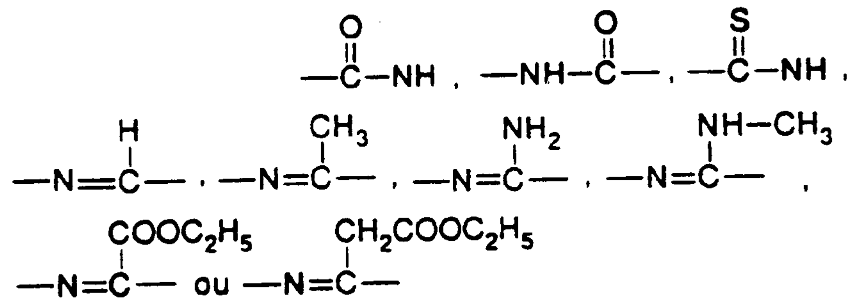

ou

or their tautomeric form.

8. Process according to one of claims 1 to 3, characterized in that $R_3$ is a 2-(5-tetrazolyl)phenyl group.

9. Process according to one of claims 1 to 3, for preparing the derivative:
- 7-n-Propyl-5-methyl-8-([2'-(5-tetrazolyl)-4-biphenylyl/methyl)-1,2,4-triazolo[1,5-c]pyrimidin-2(3H)one.

10. Process according to one of claims 1 to 3, for preparing the derivatives:
- 7-n-Propyl-5-methyl-8-{[2'-(5-tetrazolyl)-4-biphenylyl]methyl}-1,2,4-triazolo[4,3-c]pyrimidin-3(2H)-one
- 7-n-Propyl-5-methyl-3-mercapto-8-{[2'-(5-tetrazolyl)-4-biphenylyl]methyl}-1,2,4-triazolo[4,3-c]pyrimidine
- 7-n-Propyl-2,5-dimethyl-8-{[2'-(5-tetrazolyl)4-biphenylyl]methyl}-1,2,4-triazolo[1,5-c]pyrimidine
- 7-n-Propyl-5-methyl-8-{[2'-(5-tetrazolyl)-4-biphenylyl]methyl}-1,2,4-triazolo[1,5-c]pyrimidine
- 7-n-Propyl-5-methyl-2-amino-8-{[2'-(5-tetrazolyl)-4-biphenylyl]methyl}-1,2,4-triazolo[1,5-c]pyrimidine
- 7-n-Propyl-5-methyl-2-methylamino-8-{[2'-(5-tetrazolyl)-4-biphenylyl]methyl}-1,2,4-triazolo[1,5-c]-pyrimidine
- Ethyl [7-n-propyl-5-methyl-8-{[2'-(5-tetrazolyl)-4-biphenylyl]methyl}-1,2,4-triazolo[1,5-c]pyrimidine-2-yl] carboxylate
- Ethyl [7-n-propyl-5-methyl-8-{(2'-(5-tetrazolyl)-4-biphenylyl]methyl}-1,2,4-triazolo[1,5-c]pyrimidin-2-yl]acetate
- 7-Ethyl-2,5-dimethyl-8-{[2'-(5-tetrazolyl)-4-biphenylyl]methyl}-1,2,4-triazolo[1,5-c]pyrimidine
- 7-n-Butyl-5-methyl-8-{[2'-(5-tetrazolyl)-4-biphenylyl]methyl}-1,2,4-triazolo[1,5-c]pyrimidin-2(3H)-one
- 7-n-Propyl-5-ethyl-8-{[2'-(5-tetrazolyl)-4-biphenylyl]-methyl}-1,2,4-triazolo[1,5-c] pyrimidin-2(3H)-one
- 7-n-Propyl-5-methoxymethyl-8-{[2'-(5-tetrazolyl)-4-biphenylyl]methyl}-1,2,4-triazolo[1,5-c]pyrimidin-2(3H)-one.

11. Process for preparing a pharmaceutical composition, characterized in that a pharmaceutically effective amount of at least one compound of formula (I) obtained according to any one of claims 1 to 9, is incorporated in a pharmaceutically acceptable excipient, vehicle or carrier.

12. Process according to claim 10, characterized in that the pharmaceutical composition is formulated in the form of hard gelatin capsules or tablets containing 1 to 400 mg of active principle or in the form of injectable preparations containing 0.01 to 50 mg of active principle.

**Claims for the following Contracting State : GR**

1. Triazolopyrimidine derivatives, characterized in that they correspond to general formula (I):

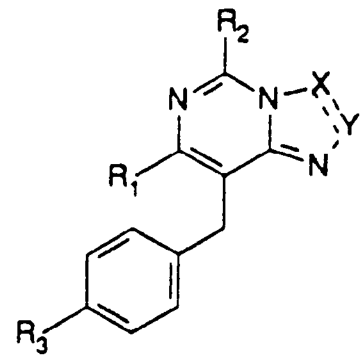

Formula (I)

in which:

$R_1$ represents a lower alkyl radical having 1 to 6 carbon-atoms or a $C_3$-$C_7$ cycloalkyl radical,

$R_2$ represents a hydrogen atom, a lower alkyl radical having 1 to 6 carbon atoms, a lower haloalkyl radical having 1 to 6 carbon atoms, a group $NR_4R'_4$, an $NH$-$NH_2$ group or a group $(CH_2)_mOR_4$ or $(CH_2)_mSR_4$,

$R_4$ and $R'_4$, which may be identical or different, represent a hydrogen atom or a lower alkyl radical having 1 to 6 carbon atoms and m represents an integer from 0 to 5,

the assembly -X---Y- or -Y---X- represents one of the following bivalent radicals:

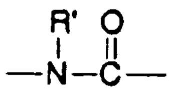 , 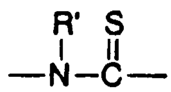 , 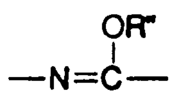 , 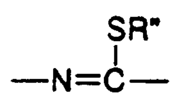 , 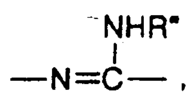 ,

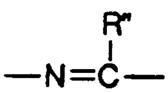 , 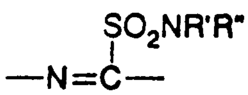

in which R' and R", which may be identical or different, represent:

a hydrogen atom, a lower alkyl radical having 1 to 6 carbon atoms, a lower haloalkyl radical having 1 to 6 carbon atoms or a group $(CH_2)_nCOOR_5$, $(CH_2)_{n'}$-$OR_5$, $(CH_2)_{n'}$-O-CO-$R_5$ or $(CH_2)_{n'}SR_5$; n being an integer from 0 to 5, n' being an integer from 1 to 5 and $R_5$ being a hydrogen atom or a lower alkyl radical having 1 to 6 carbon atoms,

a phenyl, pyridyl, thienyl or furyl radical,

$R_3$ represents an $NO_2$ or $NH_2$ group or represents one of the following radicals:

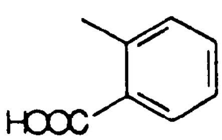 , 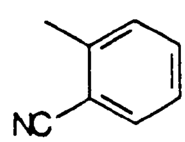 , 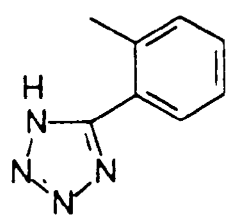 , 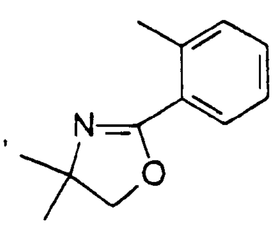 ,

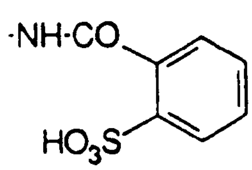 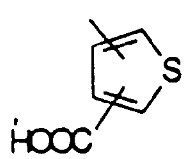 , 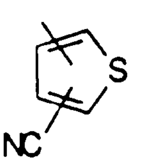 , 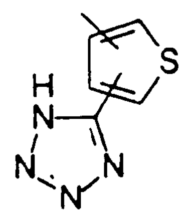

The abovementioned derivatives must also be considered in their tautomeric forms and may take the form of addition salts, especially of pharmaceutically acceptable addition salts.

2. Triazolopyrimidine derivatives according to claim 1, characterized in that:

$R_1$ represents a lower alkyl radical having 1 to 6 carbon atoms or a $C_3$-$C_7$ cycloalkyl radical:

$R_2$ represents a hydrogen atom, a lower alkyl radical having 1 to 6 carbon atoms, an NH-$NH_2$ group or a group $(CH_2)_mOR_4$ or $(CH_2)_mSR_4$; $R_4$ representing a hydrogen atom or a lower alkyl radical having 1 to 6 carbon atoms and m representing an integer from 0 to 2;

the assembly -X---Y- representing a radical selected from the following bivalent radicals:

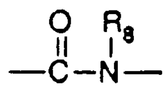 ; 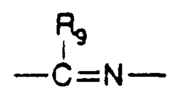 ; 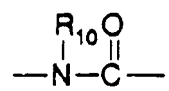 ; 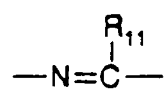

in which:

$R_8$ represents a radical selected from the group consisting of a hydrogen atom, $-(CH_2)_nOH$, $-(CH_2)_nCOOH$, $-R_{12}$, or $-(CH_2)_nCOOR_{12}$; $R_{12}$ representing a lower alkyl radical having 1 to 6 carbon atoms and n an integer equal to 1 or 2;

$R_9$ represents a hydrogen atom or an -SH radical;

$R_{10}$ represents a hydrogen atom or a lower alkyl radical having 1 to 6 carbon atoms;

$R_{11}$ represents a radical selected from the group consisting of a hydrogen atom, a lower alkyl radical having 1 to 6 carbon atoms, a lower haloalkyl radical having 1 to 6 carbon atoms, a phenyl, pyridinyl, $-O(CH_2)_nOH$, $-OR_{12}$, $-O(CH_2)_nOCOR_{12}$, SH, $-SR_{12}$, $-S(CH_2)_nCOOR_{12}$, $-S(CH_2)_nOCOR_{12}$, $-NH(CH_2)_nCOOR_{12}$, $-NR_{13}R_{14}$, $SO_2NR_{13}R_{14}$, $(CH_2)_nOH$, $(CH_2)_nOR_{12}$, COOH, $COOR_{12}$, $(CH_2)_nCOOH$, or $(CH_2)_nCOOR_{12}$; n and $R_{12}$ being defined as stated above, $R_{13}$ and $R_{14}$, which may be identical or different, representing the hydrogen atom or a lower alkyl radical having from 1 to 6 carbon atoms;

$R_3$ represents one of the following radicals:

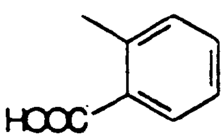 , 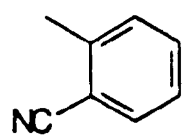 , 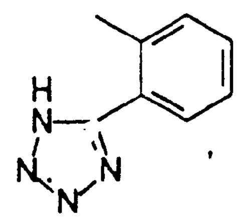 , 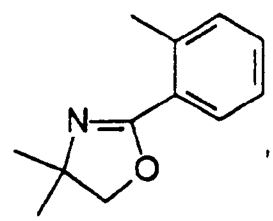 ,

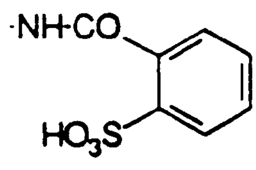 , 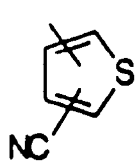 , 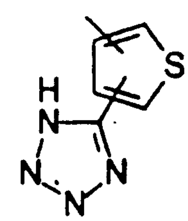

3. Derivatives according to claim 1 or 2, characterized in that $R_1$ is a group selected from ethyl, n-propyl and n-butyl.

4. Derivatives according to any one of claims 1 to 3, characterized in that $R_2$ is a group selected from methyl, ethyl or methoxymethyl.

5. Derivatives according to any one of claims 1 to 4, characterized in that the assembly -X---Y- represents one of the following bivalent radicals

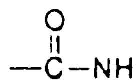 , 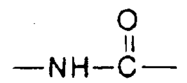 ,

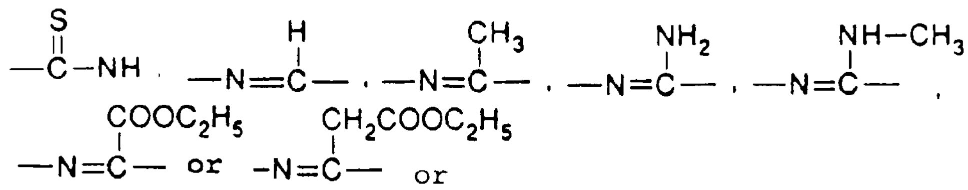

, or … or

their tautomeric form.

6. Derivatives according to any one of claims 1 to 5, characterized in that $R_3$ is a 2-(5-tetrazolyl)phenyl group.

7. Derivative according to Claim 1 or 2, characterised in that it is the derivative:
- 7-n-Propyl-5-methyl-8-{[2'-(5-tetrazolyl)-4-biphenylyl]methyl}-1,2,4-triazolo[1,5-c]pyrimidin-2(3H)one.

8. Derivatives according to Claim 1 or 2, characterised in that they are selected from the derivatives:
   - 7-n-Propyl-5-methyl-8-{[2'-(5-tetrazolyl)-4-biphenylyl]methyl}-1,2,4-triazolo[4,3-c]pyrimidin-3(2H)-one
   - 7-n-Propyl-5-methyl-3-mercapto-8-{[2'-(5-tetrazolyl)-4-biphenylyl]methyl}-1,2,4-triazolo[4,3-c]pyrimidine
   - 7-n-Propyl-2,5-dimethyl-8-{[2'-(5-tetrazolyl)4-biphenylyl]methyl}-1,2,4-triazolo[1,5-c]pyrimidine
   - 7-n-Propyl-5-methyl-8-{[2'-(5-tetrazolyl)-4-biphenylyl]methyl}-1,2,4-triazolo[1,5-c]pyrimidine
   - 7-n-Propyl-5-methyl-2-amino-8-{[2'-(5-tetrazolyl)-4-biphenylyl]methyl}-1,2,4-triazolo[1,5-c]pyrimidine
   - 7-n-Propyl-5-methyl-2-methylamino-8-{[2'-(5-tetrazolyl)-4-biphenylyl]methyl}-1,2,4-triazolo[1,5-c]-pyrimidine
   - Ethyl [7-n-propyl-5-methyl-8-{[2'-(5-tetrazolyl)-4-biphenylyl]methyl}-1,2,4-triazolo[1,5-c]pyrimidine-2-yl] carboxylate
   - Ethyl [7-n-propyl-5-methyl-8-{[2'-(5-tetrazolyl)-4-biphenylyl]methyl}-1,2,4-triazolo[1,5-c]pyrimidin-2-yl]acetate
   - 7-Ethyl-2,5-dimethyl-8-{[2'-(5-tetrazolyl)-4-biphenylyl]methyl}-1,2,4-triazolo[1,5-c]pyrimidine
   - 7-n-Butyl-5-methyl-8-{[2'-(5-tetrazolyl)-4-biphenylyl]methyl}-1,2,4-triazolo[1,5-c]pyrimidin-2(3H)-one
   - 7-n-Propyl-5-ethyl-8-{[2'-(5-tetrazolyl)-4-biphenylyl]-methyl}-1,2,4-triazolo[1,5-c]pyrimidin-2(3H)-one
   - 7-n-Propyl-S-methoxymethyl-8-{[2'-(5-tetrazolyl)-4-biphenylyl]methyl}-1,2,4-triazolo[1,5-c]pyrimidin-2(3H)-one.

9. Process for preparing the compounds of formula (I) according to any one of claims 1 to 8, characterized in that it comprises the preparation of an intermediate compound of formula (X) or (X"):

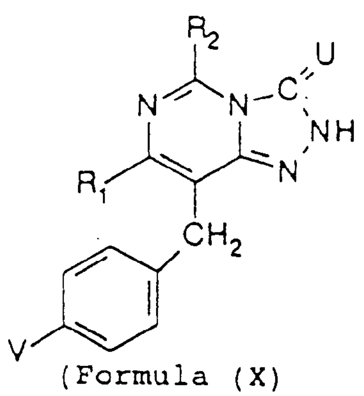

(Formula (X)

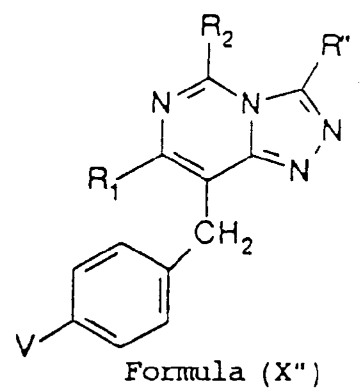

Formula (X")

in which $R_1$ and $R_2$ are as defined in Claim 1; V represents a group selected from the radicals $NO_2$,

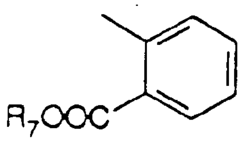 , 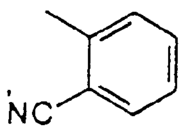 , 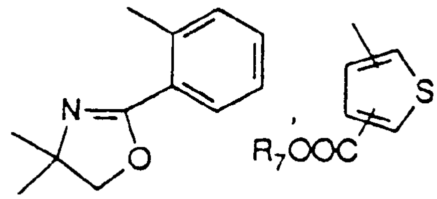 , 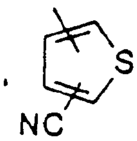

$R_7$ representing a lower alkyl radical or a benzyl;
U represents an oxygen or sulphur atom or a group N-R",
R" being as defined in Claim 1; by cyclisation of the compounds of formula (IX):

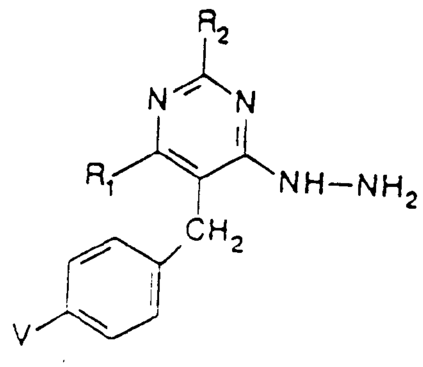

in which $R_1$, $R_2$ and V are defined as above; the said cyclisation being carried out by the action:
either of carbonyldiimidazole under reflux of tetrahydrofuran;
or by urea by heating without a solvent or in a solvent such as N-methylpyrrolidone;
or of potassium xanthogenate under reflux of an alcohol such as methoxyethanol;
or of carbon disulphide in an alcohol in the presence or absence of an amine such as triethylamine;
or of an isocyanate followed by a treatment with $POCl_3$;
or of an isothiocyanate followed by a methylation to $SCH_3$ with methyl iodide and then thermal cyclisation;
or of an ortho ester, by heating;
or alternatively of an acid chloride followed by a cyclisation using $POCl_3$;
on the compounds of formula (IX).

**10.** Preparation process according to Claim 9, characterised in that it comprises the preparation of an intermediate compound of formula (XI) or (XI"):

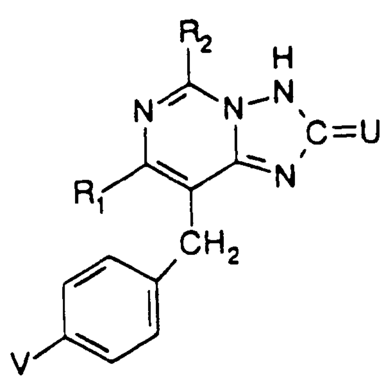 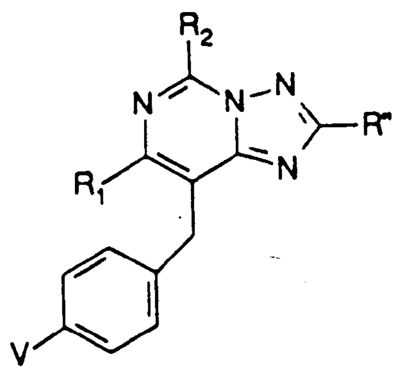

Formula (XI) (Formula (XI")

in which $R_1$, $R_2$, V, U and R" are defined as above, by isomerisation of the abovementioned compounds of formula (X) or (X") in a basic medium in water or in a water/alcohol mixture at a temperature of between

20 and 100°C; it also being possible for this isomerisation to be carried out in an acid medium by heating in acetic acid in the presence or absence of sodium acetate or potassium acetate or alternatively in dichlorobenzene in the presence of formic acid.

11. Processes for preparing the compounds of formula (I) in which $R_3$ represents a group

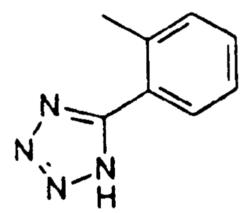

characterised in that a compound of formula:

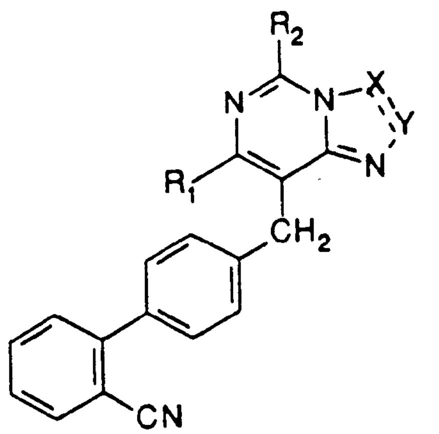

in which $R_1$, $R_2$, X and Y are defined as above, is reacted with sodium azide in dimethylformamide in the presence of an ammonium salt such as ammonium chloride at a temperature of between 100 and 150°C, or by heating in toluene or xylene with trimethyltin azide followed by a treatment with hydrogen chloride gas in tetrahydrofuran, for example.

12. Process for preparing a pharmaceutical composition, characterized in that a pharmaceutically effective amount of at least one compound of formula (I) as defined in any one of claims 1 to 8, or one of its pharmaceutically acceptable addition salts, is incorporated in a pharmaceutically acceptable excipient, vehicle or carrier.

13. Process according to claim 12, characterized in that the pharmaceutical composition is formulated in the form of hard gelatin capsules or tablets containing 1 to 400 mg of active principle or in the form of injectable preparations containing 0.01 to 50 mg of active principle.